# EUROPEAN PATENT SPECIFICATION

(11) **EP 4 717 713 B1**
(45) Date of publication and mention of the grant of the patent: **04.11.2026**
(21) Application number: 26154560.2
(22) Date of filing: 11.02.2021
(51) Int. Cl.: C07K 14/745, C12N 5/0783

(54) **CHROMATOGRAPHY RESIN AND USES THEREOF**
CHROMATOGRAPHIEHARZ UND VERWENDUNGEN DAVON
RÉSINE DE CHROMATOGRAPHIE ET SES UTILISATIONS

(30) Priority: 11.02.2020 US 202062975141 P; 26.02.2020 US 202062982002 P
(43) Date of publication of application: 01.04.2026
(62) Document number(s) of the earlier application(s) in accordance with Art. 76 EPC: 21710703.6 / 4 103 600
(73) Proprietor: ImmunityBio, Inc., San Diego, CA 92121 (US)
(72) Inventor: WONG, Hing C., Miramar, FL 33025 (US); JIAO, Jin-AN, Miramar, FL 33025 (US); PRENDES, Caitlin, Miramar, FL 33025 (US)
(74) Representative: AWA Sweden AB

(56) References cited:
- WO-A2-03/037911
- RAO L M ET AL: "Purification and characterization of rabbit tissue factor", THROMBOSIS RESEARCH, ELSEVIER, AMSTERDAM, NL, vol. 56, no. 1, 1 October 1989 (1989-10-01), pages 109 - 118, XP026443422, ISSN: 0049-3848, [retrieved on 19891001]

## Description

### CROSS-REFERENCE TO RELATED APPLICATION

This application claims priority to U.S. Provisional Patent Application Serial No. 62/982,002, filed on February 26, 2020, and U.S. Provisional Patent Application Serial No. 62/975,141, filed on February 11, 2020, both of which are incorporated herein by reference in their entireties.

### TECHNICAL FIELD

The present disclosure relates to the field of biotechnology, and more specifically, to antigen-binding molecules. The invention pertains to the use of an affinity chromatography resin for purifying a single-chain or a multi-chain chimeric polypeptide which comprises a soluble tissue factor, the affinity chromatography resin comprising an anti-tissue factor antibody or antigen-binding fragment thereof comprising a heavy chain variable domain comprising CDRs of SEQ ID NOs: 1, 2 or 9, and 3, and a light chain variable domain comprising CDRs of SEQ ID NOs: 4, 5, and 6, attached to a base resin.

Embodiments of the invention are depicted in the dependent claims.

Hereinafter, the terminology "embodiment" relates to the disclosure and only forms part of the invention if it falls within the scope of the claims.

### BACKGROUND

Tissue factor (TF), a 263 amino acid integral membrane glycoprotein with a molecular weight of ~46 kDa and the trigger protein of the extrinsic blood coagulation pathway, is the primary initiator of coagulation *in vivo.* Tissue factor, normally not in contact with circulating blood, initiates the coagulation cascade upon exposure to the circulating coagulation serine protease factors. Vascular damage exposes sub-endothelial cells expressing tissue factor, resulting in the formation of a calcium-dependent, high-affinity complex with pre-existing plasma factor VIIa (FVIIa). Binding of the serine protease FVIIa to tissue factor promotes rapid cleavage of FX to FXa and FIX to FIXa. The proteolytic activity of the resulting FXa and an active membrane surface then inefficiently converts a small amount of prothrombin to thrombin. The thrombin generated by FXa initiates platelet activation and activates minute amounts of the pro-cofactors factor V (FV) and factor VIII (FVIII) to become active cofactors, factor Va (FVa) and factor VIIIa (FVIIIa). FIXa complexes with FVIIIa on the platelet surface forming the intrinsic tenase complex, which results in rapid generation of FXa. FXa complexes with FVa to form the pro-thrombinase complex on the activated platelet surface which results in rapid cleavage of prothrombin to thrombin.

In addition to the tissue factor-FVIIa complex, a recent study showed that the tissue factor-FVIIa-FXa complex can activate FVIII, which would provide additional levels of FVIIIa during the initiation phase. The extrinsic pathway is paramount in initiating coagulation via the activation of limited amounts of thrombin, whereas the intrinsic pathway maintains coagulation by dramatic amplification of the initial signal.

Much of the tissue factor expressed on a cell surface is "encrypted," which must be "decrypted" for full participation in coagulation. The mechanism of "decryption" of cell-surface tissue factor is still unclear at this time, however, exposure of anionic phospholipids plays a major role in this process. Healthy cells actively sequester anionic phospholipids such as phosphatidyl serine (PS) to the inner leaflet of the plasma membrane. Following cellular damage, activation, or increased levels of cytosolic Ca²⁺, this bilayer asymmetry is lost, resulting in increased PS exposure on the outer leaflet, which increases the specific activity of cell-surface tissue factor-FVIIa complexes. PS exposure is known to decrease the apparent Km for activation of FIX and FX by tissue factor-FVIIa complexes, but additional mechanisms could include conformational rearrangement of tissue factor or tissue factor-FVIIa and subsequent exposure of substrate binding sites. WO 02/037911 A2 discloses the cH36 antibody.

### SUMMARY

Provided herein are affinity chromatography resins that include an anti-tissue factor antibody or antigen-binding fragment thereof attached to a base resin, and methods of using the same.

Provided herein are affinity chromatography resins that include an anti-tissue factor antibody or antigen-binding fragment thereof comprising a heavy chain variable domain comprising CDRs of SEQ ID NOs: 1, 2 or 9, and 3, and a light chain variable domain comprising CDRs of SEQ ID NOs: 4, 5, and 6, attached to a base resin.

In some embodiments of any of the affinity chromatography resins described herein, the heavy chain variable domain comprises a sequence that is at least 90% identical to SEQ ID NO: 7, and the light chain variable domain comprises a sequence that is at least 90% identical to SEQ ID NO: 8. In some embodiments of any of the affinity chromatography resins described herein, the heavy chain variable domain comprises SEQ ID NO: 7, and the light chain variable domain comprises SEQ ID NO: 8.

In some embodiments of any of the affinity chromatography resins described herein, the base resin is sepharose. In some embodiments of any of the affinity chromatography resins described herein, the base resin is any support material. In some embodiments of any of the affinity chromatography resins described herein, the support material is agarose or a capto resin. In some embodiments of any of the affinity chromatography resins described herein, the anti-tissue factor antibody or antigen-binding fragment thereof is non-covalently attached to the base resin. In some embodiments of any of the affinity chromatography resins described herein, the anti-tissue factor antibody or antigen-binding fragment thereof is covalently attached to the base resin.

In some embodiments of any of the affinity chromatography resins described herein, the anti-tissue factor antibody or antigen-binding fragment thereof is covalently attached to the base resin through the formation of a disulfide bond between a cysteine in the anti-tissue factor antibody or antigen-binding fragment thereof and a chemical group on the base resin. In some embodiments of any of the affinity chromatography resins described herein, the anti-tissue factor antibody or antigen-binding fragment thereof is covalently attached to the base resin through the formation of a covalent bond between a free amine of the anti-tissue factor antibody or antigen-binding domain and a chemical group on the base resin.

In some embodiments of any of the affinity chromatography resins described herein, the base resin is CNBr-activated or N-hydroxysuccinimide (NHS)-activated solid support material. In some embodiments of any of the affinity chromatography resins described herein, the covalent bond is represented by Formula I below: 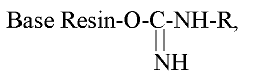 where R represents the anti-tissue factor antibody or antigen-binding fragment thereof.

Also provided herein are kits that include any of the affinity chromatography resins described herein.

Also provided herein are methods of purifying a tissue factor-containing protein that include the use of any of the affinity chromatography resins described herein. Some embodiments of any of the methods described herein further include: loading the affinity chromatography resin with a liquid comprising the tissue factor-containing protein; washing the affinity chromatography resin using one or more wash buffer(s); and eluting the tissue factor-containing protein using an elution buffer. In some embodiments of any of the methods described herein, the liquid comprising the tissue factor-containing protein is a clarified liquid culture medium. In some embodiments of any of the methods described herein, the liquid comprising the tissue factor-containing protein comprises a cell lysate.

In some embodiments of any of the methods described herein, the one or more wash buffer(s) are: (i) a first wash buffer comprising phosphate buffered saline; and (ii) a second wash buffer comprising about 0.01 M to about 0.2 M citrate and having a pH of about 4.5 to about 5.5. In some embodiments of any of the methods described herein, (i) the first wash buffer is phosphate buffered saline; and (ii) the second wash buffer is 0.1 M citrate, pH 5.0.

In some embodiments of any of the methods described herein, the elution buffer comprises 0.01 M to about 0.2 M acetate and has a pH of about 2.5 to about 3.5. In some embodiments of any of the methods described herein, the elution buffer comprises 0.1 M acetate and has a pH of about 2.9.

Also provided herein are methods of manufacturing a tissue factor-containing protein that include: (i) purifying a tissue factor-containing protein using any of the methods described herein that include the use of any of the affinity chromatography resins described herein; and (ii) performing one or more additional unit operations on an eluate obtained from step (i). In some embodiments of any of the methods described herein, the one or more additional unit operations comprises, in sequential order: performing low pH viral inactivation; performing depth filtration; performing polishing chromatography; performing nanofiltration; and performing ultrafiltration and diafiltration (UF/DF).

In some embodiments of any of the methods described herein, the tissue factor-containing protein is a single-chain chimeric polypeptide. In some embodiments of any of the methods described herein, the single-chain chimeric polypeptide comprises: (i) a first target-binding domain; (ii) a soluble tissue factor domain; and (iii) a second target-binding domain. In some embodiments of any of the methods described herein, the first target-binding domain and the soluble tissue factor domain directly abut each other. In some embodiments of any of the methods described herein, the single-chain chimeric polypeptide further comprises a linker sequence between the first target-binding domain and the soluble tissue factor domain. In some embodiments of any of the methods described herein, the soluble tissue factor domain and the second target-binding domain directly abut each other. In some embodiments of any of the methods described herein, the single-chain chimeric polypeptide further comprises a linker sequence between the soluble tissue factor domain and the second target-binding domain.

In some embodiments of any of the methods described herein, the first target-binding domain and the second target-binding domain bind specifically to the same antigen. In some embodiments of any of the methods described herein, the first target-binding domain and the second target-binding domain bind specifically to the same epitope. In some embodiments of any of the methods described herein, the first target-binding domain and the second target-binding domain comprise the same amino acid sequence. In some embodiments of any of the methods described herein, the first target-binding domain and the second target-binding domain bind specifically to different antigens.

In some embodiments of any of the methods described herein, one or both of the first target-binding domain and the second target-binding domain is an antigen-binding domain. In some embodiments of any of the methods described herein, one or both of the first target-binding domain and the second target-binding domain bind to a target selected from the group of: CD16a, CD28, CD3, CD33, CD20, CD19, CD22, CD123, IL-1R, IL-1, VEGF, IL-6R, IL-4, IL-10, PDL-1, TIGIT, PD-1, TIM3, CTLA4, MICA, MICB, IL-6, IL-8, TNFα, CD26, CD36, ULBP2, CD30, CD200, IGF-1R, MUC4AC, MUC5AC, Trop-2, CMET, EGFR, HER1, HER2, HER3, PSMA, CEA, B7H3, EPCAM, BCMA, P-cadherin, CEACAM5, a UL16-binding protein, HLA-DR, DLL4, TYRO3, AXL, MER, CD122, CD155, PDGF-DD, a ligand of TGF-β receptor II (TGF-βRII), a ligand of TGF-βRIII, a ligand of DNAM-1, a ligand of NKp46, a ligand of NKp44, a ligand of NKG2D, a ligand of NKp30, a ligand for a scMHCI, a ligand for a scMHCII, a ligand for a scTCR, a receptor for IL-1, a receptor for IL-2, a receptor for IL-3, a receptor for IL-7, a receptor for IL-8, a receptor for IL-10, a receptor for IL-12, a receptor for IL-15, a receptor for IL-17, a receptor for IL-18, a receptor for IL-21, a receptor for PDGF-DD, a receptor for stem cell factor (SCF), a receptor for stem cell-like tyrosine kinase 3 ligand (FLT3L), a receptor for MICA, a receptor for MICB, a receptor for a ULP16-binding protein, a receptor for CD155, a receptor for CD122, and a receptor for CD28.

In some embodiments of any of the methods described herein, one or both of the first target-binding domain and the second target-binding domain is a soluble interleukin, a soluble cytokine protein, or a soluble cell surface protein. In some embodiments of any of the methods described herein, the soluble interleukin, soluble cytokine protein, or soluble cell surface protein is selected from the group of: IL-1, IL-2, IL-3, IL-7, IL-8, IL-10, IL-12, IL-15, IL-17, IL-18, IL-21, PDGF-DD, SCF, FLT3L, MICA, MICB, and a ULP16-binding protein.

In some embodiments of any of the methods described herein, one or both of the first target-binding domain and the second target-binding domain is a soluble interleukin receptor, a soluble cytokine receptor, or a soluble cell surface receptor. In some embodiments of any of the methods described herein, the soluble interleukin receptor, the soluble cytokine receptor, or the soluble cell surface receptor is a soluble TGF-β receptor II (TGF-βRII), a soluble TGF-βRIII, a soluble NKG2D, a soluble NKp30, a soluble NKp44, a soluble NKp46, a soluble DNAM-1, a scMHCI, a scMHCII, a scTCR, a soluble CD155, or a soluble CD28.

In some embodiments of any of the methods described herein, the soluble tissue factor domain is a soluble human tissue factor domain. In some embodiments of any of the methods described herein, the soluble human tissue factor domain comprises a sequence that is at least 80% identical to SEQ ID NO: 10. In some embodiments of any of the methods described herein, the soluble tissue factor domain does not comprise any of: a lysine at an amino acid position that corresponds to amino acid position 20 of mature wildtype human tissue factor protein; an isoleucine at an amino acid position that corresponds to amino acid position 22 of mature wildtype human tissue factor protein; a tryptophan at an amino acid position that corresponds to amino acid position 45 of mature wildtype human tissue factor protein; an aspartic acid at an amino acid position that corresponds to amino acid position 58 of mature wildtype human tissue factor protein; a tyrosine at an amino acid position that corresponds to amino acid position 94 of mature wildtype human tissue factor protein; an arginine at an amino acid position that corresponds to amino acid position 135 of mature wildtype human tissue factor protein; and a phenylalanine at an amino acid position that corresponds to amino acid position 140 of mature wildtype human tissue factor protein.

In some embodiments of any of the methods described herein, the soluble tissue factor domain comprises one or more of: a lysine at an amino acid position that corresponds to amino acid position 20 of mature wildtype human tissue factor protein; an isoleucine at an amino acid position that corresponds to amino acid position 22 of mature wildtype human tissue factor protein; a tryptophan at an amino acid position that corresponds to amino acid position 45 of mature wildtype human tissue factor protein; an aspartic acid at an amino acid position that corresponds to amino acid position 58 of mature wildtype human tissue factor protein; a tyrosine at an amino acid position that corresponds to amino acid position 94 of mature wildtype human tissue factor protein; an arginine at an amino acid position that corresponds to amino acid position 135 of mature wildtype human tissue factor protein; and a phenylalanine at an amino acid position that corresponds to amino acid position 140 of mature wildtype human tissue factor protein.

In some embodiments of any of the methods described herein, the single-chain chimeric polypeptide further comprises one or more additional target-binding domains at its N- and/or C-terminus.

In some embodiments of any of the methods described herein, the tissue factor-containing protein is a multi-chain chimeric polypeptide. In some embodiments of any of the methods described herein, the multi-chain chimeric polypeptide comprises: (a) a first chimeric polypeptide comprising: (i) a first target-binding domain; (ii) a soluble tissue factor domain; and (iii) a first domain of a pair of affinity domains; (b) a second chimeric polypeptide comprising: (i) a second domain of a pair of affinity domains; and (ii) a second target-binding domain, where the first chimeric polypeptide and the second chimeric polypeptide associate through the binding of the first domain and the second domain of the pair of affinity domains. In some embodiments of any of the methods described herein, the first target-binding domain and the soluble tissue factor domain directly abut each other in the first chimeric polypeptide. In some embodiments of any of the methods described herein, the first chimeric polypeptide further comprises a linker sequence between the first target-binding domain and the soluble tissue factor domain in the first chimeric polypeptide. In some embodiments of any of the methods described herein, the soluble tissue factor domain and the first domain of the pair of affinity domains directly abut each other in the first chimeric polypeptide. In some embodiments of any of the methods described herein, the first chimeric polypeptide further comprises a linker sequence between the soluble tissue factor domain and the first domain of the pair of affinity domains in the first chimeric polypeptide. In some embodiments of any of the methods described herein, the second domain of the pair of affinity domains and the second target-binding domain directly abut each other in the second chimeric polypeptide. In some embodiments of any of the methods described herein, the second chimeric polypeptide further comprises a linker sequence between the second domain of the pair of affinity domains and the second target-binding domain in the second chimeric polypeptide.

In some embodiments of any of the methods described herein, the first target-binding domain and the second target-binding domain bind specifically to the same antigen. In some embodiments of any of the methods described herein, the first target-binding domain and the second target-binding domain bind specifically to different antigens.

In some embodiments of any of the methods described herein, one or both of the first target-binding domain and the second target-binding domain is an antigen-binding domain. In some embodiments of any of the methods described herein, one or both of the first target-binding domain and the second target-binding domain bind specifically to a target selected from the group of: CD16a, CD28, CD3, CD33, CD20, CD19, CD22, CD123, IL-1R, IL-1, VEGF, IL-6R, IL-4, IL-10, PDL-1, TIGIT, PD-1, TIM3, CTLA4, MICA, MICB, IL-6, IL-8, TNFα, CD26, CD36, ULBP2, CD30, CD200, IGF-1R, MUC4AC, MUC5AC, Trop-2, CMET, EGFR, HER1, HER2, HER3, PSMA, CEA, B7H3, EPCAM, BCMA, P-cadherin, CEACAM5, a UL16-binding protein, HLA-DR, DLL4, TYRO3, AXL, MER, CD122, CD155, PDGF-DD, a ligand of TGF-β receptor II (TGF-βRII), a ligand of TGF-βRIII, a ligand of DNAM-1, a ligand of NKp46, a ligand of NKp44, a ligand of NKG2D, a ligand of NKp30, a ligand for a scMHCI, a ligand for a scMHCII, a ligand for a scTCR, a receptor for IL-1, a receptor for IL-2, a receptor for IL-3, a receptor for IL-7, a receptor for IL-8, a receptor for IL-10, a receptor for IL-12, a receptor for IL-15, a receptor for IL-17, a receptor for IL-18, a receptor for IL-21, a receptor for PDGF-DD, a receptor for stem cell factor (SCF), a receptor for stem cell-like tyrosine kinase 3 ligand (FLT3L), a receptor for MICA, a receptor for MICB, a receptor for a ULP16-binding protein, a receptor for CD155, a receptor for CD122, and a receptor for CD28.

In some embodiments of any of the methods described herein, one or both of the first target-binding domain and the second target-binding domain is a soluble interleukin or cytokine protein. In some embodiments of any of the methods described herein, the soluble interleukin, cytokine, or ligand protein is selected from the group of: IL-1, IL-2, IL-3, IL-7, IL-8, IL-10, IL-12, IL-15, IL-17, IL-18, IL-21, PDGF-DD, SCF, and FLT3L.

In some embodiments of any of the methods described herein, one or both of the first target-binding domain and the second target-binding domain is a soluble interleukin or cytokine receptor. In some embodiments of any of the methods described herein, the soluble receptor is a soluble TGF-β receptor II (TGF-βRII), a soluble TGF-βRIII, a soluble NKG2D, a soluble NKp30, a soluble NKp44, a soluble NKp46, a soluble DNAM-1, a scMHCI, a scMHCII, a scTCR, a soluble CD155, or a soluble CD28.

In some embodiments of any of the methods described herein, the first chimeric polypeptide further comprises one or more additional target-binding domain(s). In some embodiments of any of the methods described herein, the second chimeric polypeptide further comprises one or more additional target-binding domains.

In some embodiments of any of the methods described herein, the soluble tissue factor domain is a soluble human tissue factor domain. In some embodiments of any of the methods described herein, the soluble human tissue factor domain comprises a sequence that is at least 80% identical to SEQ ID NO: 10.

In some embodiments of any of the methods described herein, the soluble tissue factor domain does not comprise any of: a lysine at an amino acid position that corresponds to amino acid position 20 of mature wildtype human tissue factor protein; an isoleucine at an amino acid position that corresponds to amino acid position 22 of mature wildtype human tissue factor protein; a tryptophan at an amino acid position that corresponds to amino acid position 45 of mature wildtype human tissue factor protein; an aspartic acid at an amino acid position that corresponds to amino acid position 58 of mature wildtype human tissue factor protein; a tyrosine at an amino acid position that corresponds to amino acid position 94 of mature wildtype human tissue factor protein; an arginine at an amino acid position that corresponds to amino acid position 135 of mature wildtype human tissue factor protein; and a phenylalanine at an amino acid position that corresponds to amino acid position 140 of mature wildtype human tissue factor protein.

In some embodiments of any of the methods described herein, the soluble tissue factor domain comprises one or more of: a lysine at an amino acid position that corresponds to amino acid position 20 of mature wildtype human tissue factor protein; an isoleucine at an amino acid position that corresponds to amino acid position 22 of mature wildtype human tissue factor protein; a tryptophan at an amino acid position that corresponds to amino acid position 45 of mature wildtype human tissue factor protein; an aspartic acid at an amino acid position that corresponds to amino acid position 58 of mature wildtype human tissue factor protein; a tyrosine at an amino acid position that corresponds to amino acid position 94 of mature wildtype human tissue factor protein; an arginine at an amino acid position that corresponds to amino acid position 135 of mature wildtype human tissue factor protein; and a phenylalanine at an amino acid position that corresponds to amino acid position 140 of mature wildtype human tissue factor protein.

In some embodiments of any of the methods described herein, the pair of affinity domains is a sushi domain from an alpha chain of human IL-15 receptor (IL15Rα) and a soluble IL-15. In some embodiments of any of the methods described herein, the pair of affinity domains is selected from the group consisting of: barnase and barnstar, a PKA and an AKAP, adapter/docking tag modules based on mutated RNase I fragments, and SNARE modules based on interactions of the proteins syntaxin, synaptotagmin, synaptobrevin, and SNAP25.

Also provided are tissue factor-containing proteins manufactured by any of the methods described herein. Also provided herein are pharmaceutical compositions that include any of the manufactured tissue factor-containing proteins described herein.

As used herein, the term "chimeric" refers to a polypeptide that includes amino acid sequences (e.g., domains) originally derived from two different sources (e.g., two different naturally-occurring proteins, e.g., from the same or different species). For example, a chimeric polypeptide can include domains from at least two different naturally occurring human proteins. In some examples, a chimeric polypeptide can include a domain that is a synthetic sequence (e.g., an scFv) and a domain that is derived from a naturally-occurring protein (e.g., a naturally-occurring human protein). In some embodiments, a chimeric polypeptide can include at least two different domains that are synthetic sequences (e.g., two different scFvs).

An "antigen-binding domain" is one or more protein domain(s) (e.g., formed from amino acids from a single polypeptide or formed from amino acids from two or more polypeptides (e.g., the same or different polypeptides) that is capable of specifically binding to one or more different antigen(s). In some examples, an antigen-binding domain can bind to an antigen or epitope with specificity and affinity similar to that of naturally-occurring antibodies. In some embodiments, the antigen-binding domain can be an antibody or a fragment thereof. In some embodiments, an antigen-binding domain can include an alternative scaffold. Non-limiting examples of antigen-binding domains are described herein. Additional examples of antigen-binding domains are known in the art.

A "soluble tissue factor domain" refers to a polypeptide having at least 70% identity (e.g., at least 75% identity, at least 80% identity, at least 85% identity, at least 90% identity, at least 95% identity, at least 99% identity, or 100% identical) to a segment of a wildtype mammalian tissue factor protein (e.g., a wildtype human tissue factor protein) that lacks the transmembrane domain and the intracellular domain. Non-limiting examples of soluble tissue factor domains are described herein.

The term "soluble interleukin protein" is used herein to refer to a mature and secreted interleukin protein or a biologically active fragment thereof. In some examples, a soluble interleukin protein can include a sequence that is at least 70% identical, at least 75% identical, at least 80% identical, at least 85% identical, at least 90% identical, at least 95% identical, at least 99% identical, or 100% identical to a wildtype mature and secreted mammalian interleukin protein (e.g., a wildtype human interleukin protein) and retains its biological activity. Non-limiting examples of soluble interleukin proteins are described herein.

The term "soluble cytokine protein" is used herein to refer to a mature and secreted cytokine protein or a biologically active fragment thereof. In some examples, a soluble cytokine protein can include a sequence that is at least 70% identical, at least 75% identical, at least 80% identical, at least 85% identical, at least 90% identical, at least 95% identical, at least 99% identical, or 100% identical to a wildtype mature and secreted mammalian interleukin protein (e.g., a wildtype human interleukin protein) and retains its biological activity. Non-limiting examples of soluble cytokine proteins are described herein.

The term "soluble interleukin receptor" is used herein in the broadest sense to refer to a polypeptide that lacks a transmembrane domain (and optionally an intracellular domain) that is capable of binding one or more of its natural ligands (e.g., under physiological conditions, e.g., in phosphate buffered saline at room temperature). For example, a soluble interleukin receptor can include a sequence that is at least 70% identical (e.g., at least 75% identical, at least 80% identical, at least 85% identical, at least 90% identical, at least 95% identical, at least 99% identical, or 100% identical) to an extracellular domain of wildtype interleukin receptor and retains its ability to specifically bind to one or more of its natural ligands, but lacks its transmembrane domain (and optionally, further lacks its intracellular domain). Non-limiting examples of soluble interleukin receptors are described herein.

The term "soluble cytokine receptor" is used herein in the broadest sense to refer to a polypeptide that lacks a transmembrane domain (and optionally an intracellular domain) that is capable of binding one or more of its natural ligands (e.g., under physiological conditions, e.g., in phosphate buffered saline at room temperature). For example, a soluble cytokine receptor can include a sequence that is at least 70% identical (e.g., at least 75% identical, at least 80% identical, at least 85% identical, at least 90% identical, at least 95% identical, at least 99% identical, or 100% identical) to an extracellular domain of wildtype cytokine receptor and retains its ability to specifically bind to one or more of its natural ligands, but lacks its transmembrane domain (and optionally, further lacks its intracellular domain). Non-limiting examples of soluble cytokine receptors are described herein.

The term "antibody" is used herein in its broadest sense and includes certain types of immunoglobulin molecules that include one or more antigen-binding domains that specifically bind to an antigen or epitope. An antibody specifically includes, e.g., intact antibodies (e.g., intact immunoglobulins), antibody fragments, and multi-specific antibodies. One example of an antigen-binding domain is an antigen-binding domain formed by a VH -VL dimer. Additional examples of an antibody are described herein. Additional examples of an antibody are known in the art.

"Affinity" refers to the strength of the sum total of non-covalent interactions between an antigen-binding site and its binding partner (e.g., an antigen or epitope). Unless indicated otherwise, as used herein, "affinity" refers to intrinsic binding affinity, which reflects a 1:1 interaction between members of an antigen-binding domain and an antigen or epitope. The affinity of a molecule X for its partner Y can be represented by the dissociation equilibrium constant (K_{D}). The kinetic components that contribute to the dissociation equilibrium constant are described in more detail below. Affinity can be measured by common methods known in the art, including those described herein. Affinity can be determined, for example, using surface plasmon resonance (SPR) technology (e.g., BIACORE^{®}) or biolayer interferometry (e.g., FORTEBIO^{®}). Additional methods for determining the affinity for an antigen-binding domain and its corresponding antigen or epitope are known in the art.

A "multi-chain polypeptide" as used herein to refers to a polypeptide comprising two or more (e.g., three, four, five, six, seven, eight, nine, or ten) protein chains (e.g., at least a first chimeric polypeptide and a second polypeptide), where the two or more proteins chains associate through non-covalent bonds to form a quaternary structure.

A "single-chain polypeptide" as used herein to refers to a single protein chain.

The term "pair of affinity domains" is two different protein domain(s) that bind specifically to each other with a K_{D} of less than of less than 1 x 10⁻⁷ M (e.g., less than 1 x 10⁻⁸ M, less than 1 x 10⁻⁹ M, less than 1 x 10⁻¹⁰ M, or less than 1 x 10⁻¹¹ M). **In** some examples, a pair of affinity domains can be a pair of naturally-occurring proteins. **In** some embodiments, a pair of affinity domains can be a pair of synthetic proteins. Non-limiting examples of pairs of affinity domains are described herein.

The term "epitope" means a portion of an antigen that specifically binds to an antigen-binding domain. Epitopes can, e.g., consist of surface-accessible amino acid residues and/or sugar side chains and may have specific three-dimensional structural characteristics, as well as specific charge characteristics. Conformational and non-conformational epitopes are distinguished in that the binding to the former but not the latter may be lost in the presence of denaturing solvents. An epitope may comprise amino acid residues that are directly involved in the binding, and other amino acid residues, which are not directly involved in the binding. Methods for identifying an epitope to which an antigen-binding domain binds are known in the art.

The term "treatment" means to ameliorate at least one symptom of a disorder. **In** some examples, the disorder being treated is cancer and to ameliorate at least one symptom of cancer includes reducing aberrant proliferation, gene expression, signaling, translation, and/or secretion of factors. Generally, the methods of treatment include administering a therapeutically effective amount of composition that reduces at least one symptom of a disorder to a subject who is in need of, or who has been determined to be in need of such treatment.

The term "purifying" means a step performed to isolate a protein (e.g., any of the exemplary single-chain or multi-chain chimeric polypeptides described herein) from one or more other impurities (e.g., bulk impurities) or components present in a fluid containing a protein (e.g., liquid culture medium proteins or one or more other components (e.g., DNA, RNA, other proteins, endotoxins, viruses, etc.) present in or secreted from a mammalian cell). Purification can be performed using a resin, membrane, or any other solid support that binds either a protein or contaminants (e.g., through the use of affinity chromatography, hydrophobic interaction chromatography, anion or cation exchange chromatography, or molecular sieve chromatography). A protein can be purified from a fluid containing the protein using at least one chromatography column (e.g., any of the chromatography columns described herein that include any of the affinity chromatography resins described herein).

The term "polishing" is a term of art and means a step performed to remove remaining trace or small amounts of contaminants or impurities from a fluid containing a protein (e.g., any of the single-chain or multi-chain chimeric polypeptides described herein) that is close to a final desired purity. For example, polishing can be performed by passing a fluid containing the protein through a chromatographic column(s) or membrane absorber(s) that selectively binds to either the protein or small amounts of contaminants or impurities present in a fluid containing the protein. In such an example, the eluate/filtrate of the chromatographic column(s) or membrane absorber(s) contains the protein.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting.

Other features and advantages of the invention will be apparent from the following detailed description and figures, and from the claims.

### BRIEF DESCRIPTION OF DRAWINGS

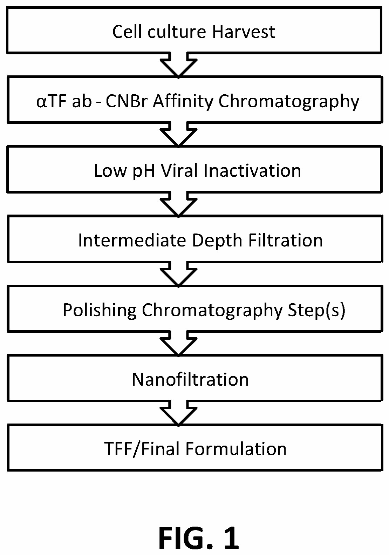

**FIG. 1** is a workflow schematic showing an exemplary workflow for manufacturing a tissue-factor containing protein that include providing a harvested cell culture (e.g., a substantially cell-free (e.g., at least 99% free of cells) liquid culture medium), purifying the tissue-factor containing protein using any of the affinity chromatography resins described herein, performing low pH viral inactivation, performing depth filtration, performing polishing chromatography, performing nanofiltration, and performing ultrafiltration/diafiltration (shown as "TFF/final formulation" in the schematic).

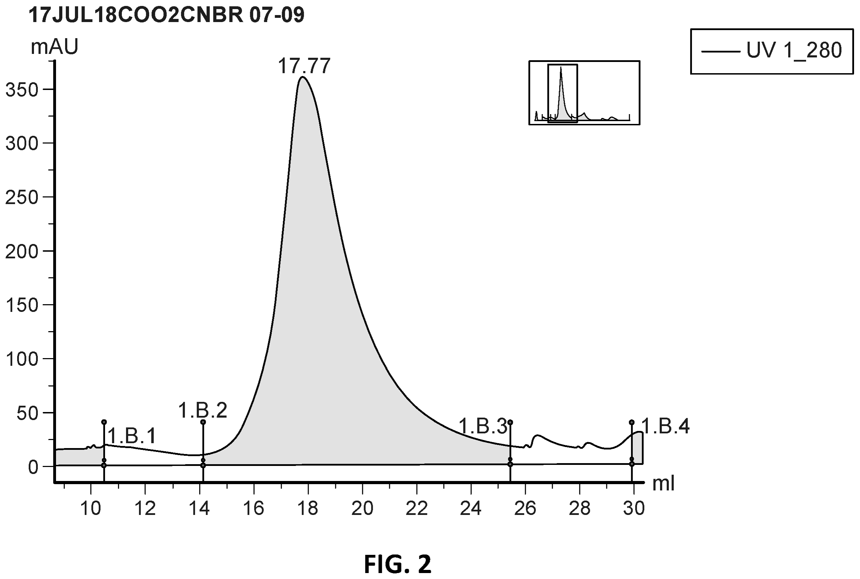

**FIG. 2** is an elution profile of 18t15-12s protein from an exemplary affinity chromatography resin provided herein (αTF antibody CNBr-Sepharose affinity column) that was equilibrated with phosphate buffered saline, loaded, washed with phosphate buffered saline, and eluted using 0.1M acetic acid, pH 2.9.

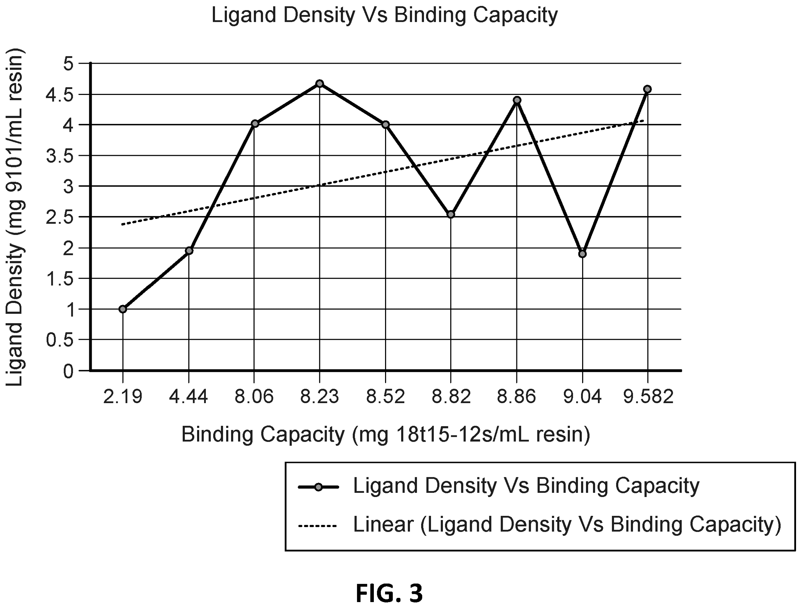

**FIG. 3** is a graph of ligand density versus binding capacity for different affinity chromatography resin preparations, showing the relationship between anti-tissue factor antibody coupling density and the binding capacity of the resin for a tissue-factor containing protein, such as 18t15-12s protein.

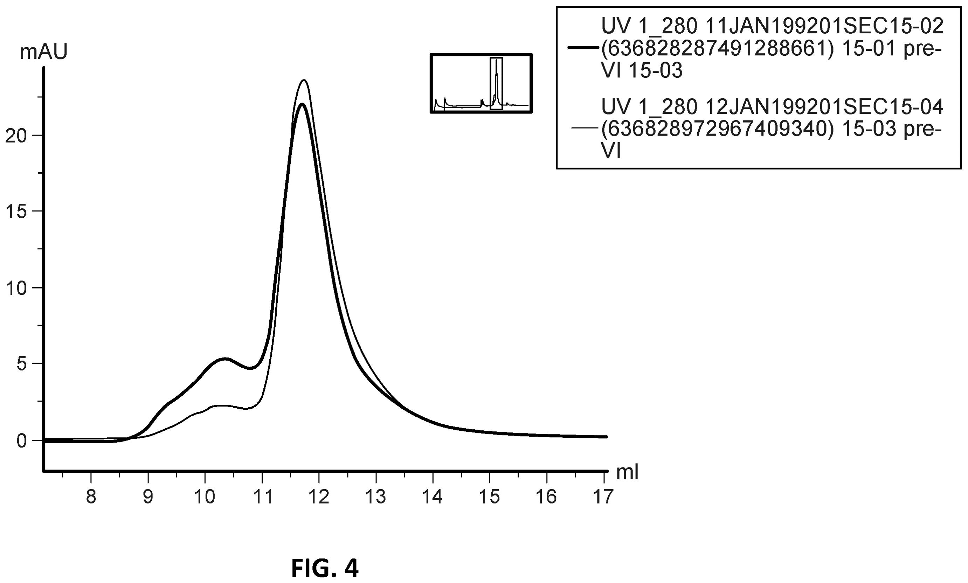

**FIG. 4** is a graph showing optimization of wash conditions for 18t15-12s purification using an exemplary affinity chromatography resin provided herein (anti-tissue factor CNBr-Sepharose affinity column). Each experiment was performed using a column equilibrated with phosphate buffered saline, loaded, washed with phosphate buffered saline, and eluted using 0.1M acetic acid, pH 2.9. The data shown in blue did not include a further 5 CV wash using 0.1M sodium citrate, pH 5.0, while the data in orange included a further 5 CV wash using 0.1 M sodium citrate, pH 5.0.

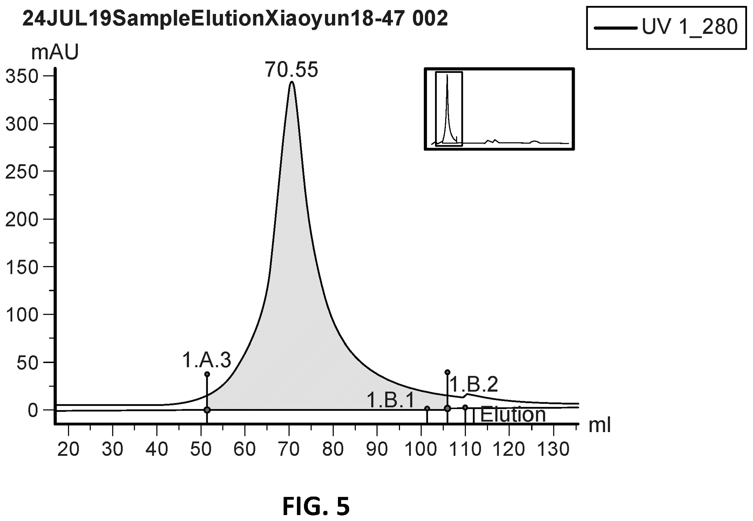

**FIG. 5** shows an elution profile of the first run of a newly generated an exemplary affinity chromatography resin provided herein (anti-tissue factor CNBr column) purified by equilibrating with the resin with phosphate buffered saline, loading the resin, washing the resin using phosphate buffered saline, and eluting the resin using 0.1M acetic acid, pH 2.9.

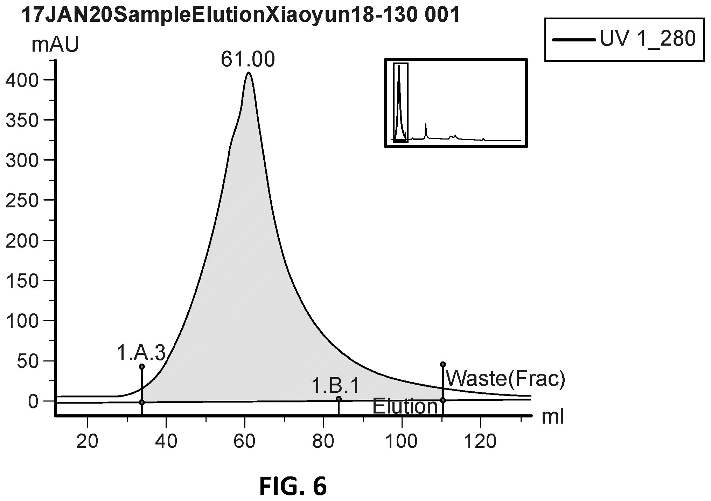

**FIG. 6** shows an elution profile of the 51^{st} purification run using the same column used in FIG. 5. The 51^{st} purification run was performed by equilibrating the column with phosphate buffered saline, loading the column, washing the column with phosphate buffered saline, and eluting the column using 0.1M acetic acid, pH 2.9.

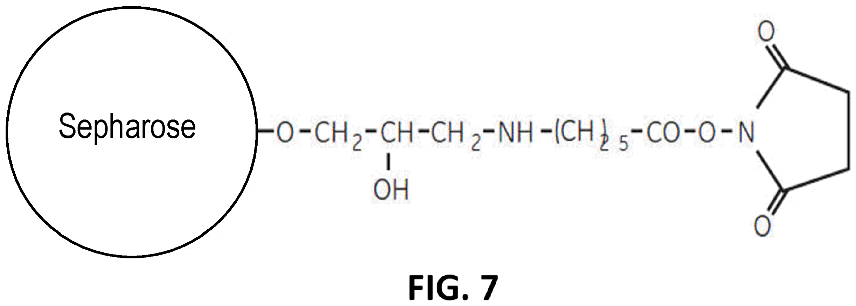

**FIG. 7** shows a partial structure of NHS-activated Sepharose High Performance resin bearing activated spacer arms.

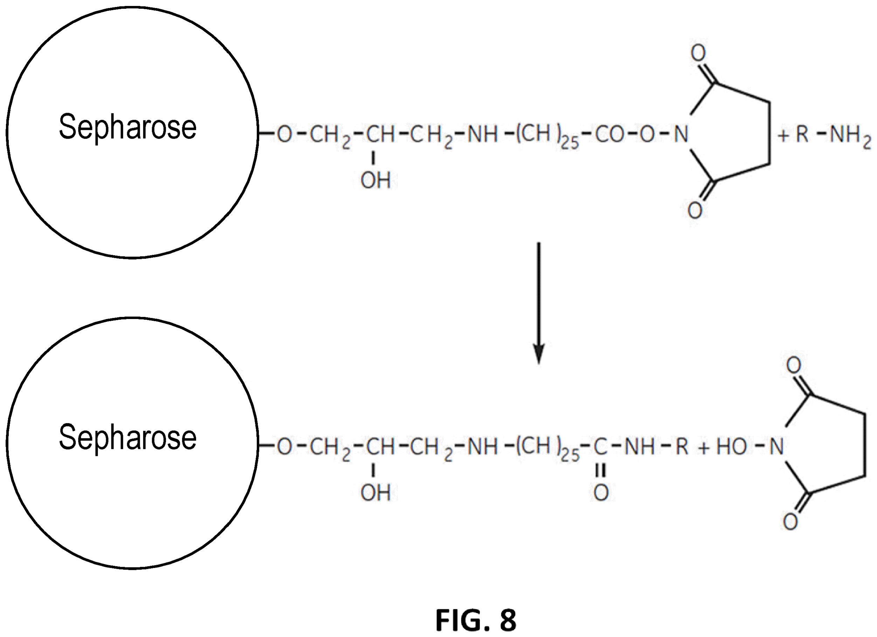

**FIG. 8** shows coupling of a protein ligand containing primary amino group (R-NH₂) to NHS-activated Sepharose resin.

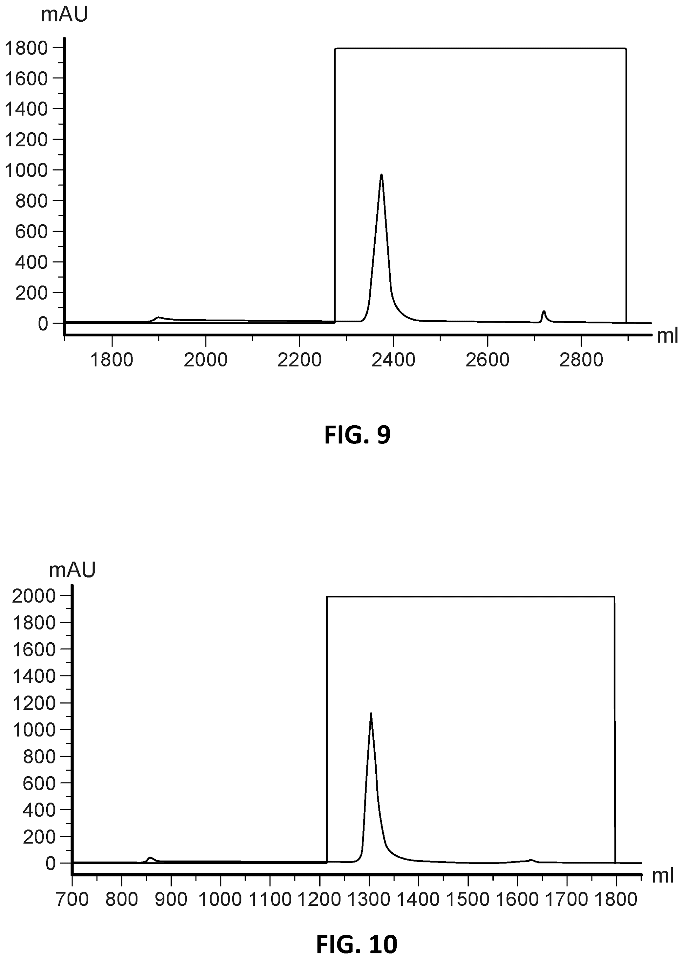

**FIG. 9** shows a chromatogram of TGFRt15-TGFRs elution profile from an exemplary affinity chromatography resin provided herein (anti-tissue factor CNBr column). The dark gray line is absorbance at 280 nm, and the light gray line represents the use of the elution buffer (0.1 M acetic acid, pH 2.9).
**FIG. 10** shows a chromatogram of 2t2 elution profile from an exemplary affinity chromatography resin provided herein (anti-tissue factor CNBr column). The dark gray line is absorbance at 280 nm, and the light gray line represents the use of the elution buffer (0.1 M acetic acid, pH 2.9).

### DETAILED DESCRIPTION

Provided herein is an affinity chromatography resin comprising an anti-tissue factor antibody or antigen-binding fragment thereof comprising a heavy chain variable domain comprising CDRs of SEQ ID NOs: 1, 2 or 9, and 3, and a light chain variable domain comprising CDRs of SEQ ID NOs: 4, 5, and 6, attached to a base resin.

Also provided herein is a kit including any of the affinity chromatography resins described herein. Also provided herein are methods of purifying a tissue factor-containing protein comprising the use of any of the affinity chromatography resins described herein. Also provided herein are methods of manufacturing a tissue factor-containing protein comprising: (i) purifying a tissue factor-containing protein using any of the methods of purifying a tissue factor-containing protein described herein; and (ii) performing one or more additional unit operations on an eluate obtained from step (i). Also provided herein is a tissue factor-containing protein manufactured by any of the methods described herein. Also provided herein is a pharmaceutical composition comprising any of the manufactured tissue factor-containing proteins described herein.

In some embodiments, an affinity chromatography resin as described herein can have a binding capacity for a tissue factor-containing protein of about 0.5 mg/mL to about 6.0 mg/mL (e.g., about 0.5 mg/mL to about 5.5 mg/mL, about 0.5 mg/mL to about 5.0 mg/mL, about 0.5 mg/mL to about 4.5 mg/mL, about 0.5 mg/mL to about 4.0 mg/mL, about 0.5 mg/mL to about 3.5 mg/mL, about 0.5 mg/mL to about 3.0 mg/mL, about 0.5 mg/mL to about 2.5 mg/mL, about 0.5 mg/mL to about 2.0 mg/mL, about 0.5 mg/mL to about 1.5 mg/mL, about 0.5 mg/mL to about 1.0 mg/mL, about 1.0 mg/mL to about 6.0 mg/mL, about 1.0 mg/mL to about 5.5 mg/mL, about 1.0 mg/mL to about 5.0 mg/mL, about 1.0 mg/mL to about 4.5 mg/mL, about 1.0 mg/mL to about 4.0 mg/mL, about 1.0 mg/mL to about 3.5 mg/mL, about 1.0 mg/mL to about 3.0 mg/mL, about 1.0 mg/mL to about 2.5 mg/mL, about 1.0 mg/mL to about 2.0 mg/mL, about 1.0 mg/mL to about 1.5 mg/mL, about 1.5 mg/mL to about 6.0 mg/mL, about 1.5 mg/mL to about 5.5 mg/mL, about 1.5 mg/mL to about 5.0 mg/mL, about 1.5 mg/mL to about 4.5 mg/mL, about 1.5 mg/mL to about 4.0 mg/mL, about 1.5 mg/mL to about 3.5 mg/mL, about 1.5 mg/mL to about 3.0 mg/mL, about 1.5 mg/mL to about 2.5 mg/mL, about 1.5 mg/mL to about 2.0 mg/mL, about 2.0 mg/mL to about 6.0 mg/mL, about 2.0 mg/mL to about 5.5 mg/mL, about 2.0 mg/mL to about 5.0 mg/mL, about 2.0 mg/mL to about 4.5 mg/mL, about 2.0 mg/mL to about 4.0 mg/mL, about 2.0 mg/mL to about 3.5 mg/mL, about 2.0 mg/mL to about 3.0 mg/mL, about 2.0 mg/mL to about 2.5 mg/mL, about 2.5 mg/mL to about 6.0 mg/mL, about 2.5 mg/mL to about 5.5 mg/mL, about 2.5 mg/mL to about 5.0 mg/mL, about 2.5 mg/mL to about 4.5 mg/mL, about 2.5 mg/mL to about 4.0 mg/mL, about 2.5 mg/mL to about 3.5 mg/mL, about 2.5 mg/mL to about 3.0 mg/mL, about 3.0 mg/mL to about 6.0 mg/mL, about 3.0 mg/mL to about 5.5 mg/mL, about 3.0 mg/mL to about 5.0 mg/mL, about 3.0 mg/mL to about 4.5 mg/mL, about 3.0 mg/mL to about 4.0 mg/mL, about 3.0 mg/mL to about 3.5 mg/mL, about 3.5 mg/mL to about 6.0 mg/mL, about 3.5 mg/mL to about 5.5 mg/mL, about 3.5 mg/mL to about 5.0 mg/mL, about 3.5 mg/mL to about 4.5 mg/mL, about 3.5 mg/mL to about 4.0 mg/mL, about 4.0 mg/mL to about 6.0 mg/mL, about 4.0 mg/mL to about 5.5 mg/mL, about 4.0 mg/mL to about 5.0 mg/mL, about 4.0 mg/mL to about 4.5 mg/mL, about 4.5 mg/mL to about 6.0 mg/mL, about 4.5 mg/mL to about 5.5 mg/mL, about 4.5 mg/mL to about 5.0 mg/mL, about 5.0 mg/mL to about 6.0 mg/mL, about 5.0 mg/mL to about 5.5 mg/mL, or about 5.5 mg/mL to about 6.0 mg/mL).

As used herein, "affinity chromatography" refers to a method of separating a solution or mixture based on a highly specific interaction between antigen and antibody, enzyme and substrate, receptor and ligand, or protein and nucleic acid. Affinity chromatography (e.g., Protein A, Protein G, Protein L, or metal chelate affinity chromatography) is an effective method to specifically purify target proteins with high yield recovery and low levels of contaminating proteins and other components in the starting mixture such as cell culture supernatants, serum, and plasma. Once a clarified liquid or mixture comprising the target protein is obtained, affinity chromatography can be used with a combination of one or more different purification techniques (e.g., ion exchange separation, hydrophobic interaction separation) for the purification of the target protein.

Antibody (e.g., monoclonal or polyclonal) based immunoaffinity chromatography has been widely used as a method/process to purify specific antigens or antigen-containing proteins (e.g., human tissue factor-containing proteins) in the fields of research, development, and manufacturing. For the immunoaffinity method, an antibody (e.g., monoclonal) or a mixture of antibodies (e.g., polyclonal) is immobilized (e.g., coupled) covalently onto solid support materials such as CNBr-activated Sepharose, NHS-Sepharose, and other related beads/resins suitable for protein coupling or immobilization. A solid support material is any material to which a biospecific ligand is covalently attached wherein examples of the materials used in affinity chromatography include agarose, cellulose, dextran, polyacrylamide, latex, and controlled pore glass. Useful support materials can have properties such as high surface-area to volume ratio, chemical groups that are easily modified for covalent attachment of ligands, minimal nonspecific binding properties, good flow characteristics and mechanical and chemical stability.

### Tissue Factor

Human tissue factor is a 263 amino-acid transmembrane protein containing three domains: (1) a 219-amino acid N-terminal extracellular domain (residues 1-219); (2) a 22-amino acid transmembrane domain (residues 220-242); and (3) a 21-amino acid cytoplasmic C-terminal tail (residues 242-263) ((UniProtKB Identifier Number: P13726). The cytoplasmic tail contains two phosphorylation sites at Ser253 and Ser258, and one S-palmitoylation site at Cys245. Deletion or mutation of the cytoplasmic domain was not found to affect tissue factor coagulation activity. Tissue factor has one S-palmitoylation site in the intracellular domain of the protein at Cys245. The Cys245 is located at the amino acid terminus of the intracellular domain and close to the membrane surface. The tissue factor transmembrane domain is composed of a single-spanning α-helix.

The extracellular domain of tissue factor, composed of two fibronectin type III domains, is connected to the transmembrane domain through a six-amino acid linker. This linker provides conformational flexibility to decouple the tissue factor extracellular domain from its transmembrane and cytoplasmic domains. Each tissue factor fibronectin type III module is composed of two overlapping β sheets with the top sheet domain containing three antiparallel β-strands and the bottom sheet containing four β-strands. The β-strands are connected by β-loops between strand βA and βB, βC and βD, and βE and βF, all of which are conserved in conformation in the two modules. There are three short α-helix segments connecting the β-strands. A unique feature of tissue factor is a 17-amino acid β-hairpin between strand β10 and strand β11, which is not a common element of the fibronectin superfamily. The N-terminal domain also contains a 12 amino acid loop between β6F and β7G that is not present in the C-terminal domain and is unique to tissue factor. Such a fibronectin type III domain structure is a feature of the immunoglobulin-like family of protein folds and is conserved among a wide variety of extracellular proteins.

The zymogen FVII is rapidly converted to FVIIa by limited proteolysis once it binds to tissue to form the active tissue factor-FVIIa complex. The FVIIa, which circulates as an enzyme at a concentration of approximately 0.1 nM (1% of plasma FVII), can also bind directly to tissue factor. The allosteric interaction between tissue factor and FVIIa on the tissue factor-FVIIa complex greatly increases the enzymatic activity of FVIIa: an approximate 20- to 100-fold increase in the rate of hydrolysis of small, chromogenic peptidyl substrates, and nearly a million-fold increase in the rate of activation of the natural macromolecular substrates FIX and FX. In concert with allosteric activation of the active site of FVIIa upon binding to tissue factor, the formation of tissue factor-FVIIa complex on phospholipid bilayer (i.e., upon exposure of phosphatidyl-L-serine on membrane surfaces) increases the rate of FIX or FX activation, in a Ca²⁺-dependent manner, an additional 1,000-fold. The roughly million-fold overall increase in FX activation by tissue factor-FVIIa-phospholipid complex relative to free FVIIa is a critical regulatory point for the coagulation cascade.

FVII is a ~50 kDa, single-chain polypeptide consisting of 406 amino acid residues, with an N-terminal γ-carboxyglutamate-rich (GLA) domain, two epidermal growth factor-like domains (EGF1 and EFG2), and a C-terminal serine protease domain. FVII is activated to FVIIa by a specific proteolytic cleavage of the Ile-¹⁵⁴-Arg¹⁵² bond in the short linker region between the EGF2 and the protease domain. This cleavage results in the light and heavy chains being held together by a single disulfide bond of Cys¹³⁵ and Cys²⁶². FVIIa binds phospholipid membrane in a Ca²⁺-dependent manner through its N-terminal GLA-domain. Immediately C-terminal to the GLA domain is an aromatic stack and two EGF domains. The aromatic stack connects the GLA to EGF1 domain which binds a single Ca²⁺ ion. Occupancy of this Ca²⁺-binding site increases FVIIa amidolytic activity and tissue factor association. The catalytic triad consist of His¹⁹³, Asp²⁴², and Ser³⁴⁴, and binding of a single Ca²⁺ ion within the FVIIa protease domain is critical for its catalytic activity. Proteolytic activation of FVII to FVIIa frees the newly formed amino terminus at Ile¹⁵³ to fold back and be inserted into the activation pocket forming a salt bridge with the carboxylate of Asp³⁴³ to generate the oxyanion hole. Formation of this salt bridge is critical for FVIIa activity. However, oxyanion hole formation does not occur in free FVIIa upon proteolytic activation. As a result, FVIIa circulates in a zymogen-like state that is poorly recognized by plasma protease inhibitors, allowing it to circulate with a half-life of approximately 90 minutes.

Tissue factor-mediated positioning of the FVIIa active site above the membrane surface is important for FVIIa towards cognate substrates. Free FVIIa adopts a stable, extended structure when bound to the membrane with its active site positioned ~80Å above the membrane surface. Upon FVIIa binding to tissue factor, the FVa active site is repositioned ~6Å closer to the membrane. This modulation may aid in a proper alignment of the FVIIa catalytic triad with the target substrate cleavage site. Using GLA-domainless FVIIa, it has been shown that the active site was still positioned a similar distance above the membrane, demonstrating that tissue factor is able to fully support FVIIa active site positioning even in the absence of FVIIa-membrane interaction. Additional data showed that tissue factor supported full FVIIa proteolytic activity as long as the tissue factor extracellular domain was tethered in some way to the membrane surface. However, raising the active site of FVIIa greater than 80Å above the membrane surface greatly reduced the ability of the tissue factor-FVIIa complex to activate FX but did not diminish tissue factor-FVIIa amidolytic activity.

Alanine scanning mutagenesis has been used to assess the role of specific amino acid side chains in the tissue factor extracellular domain for interaction with FVIIa (Gibbs et al., Biochemistry 33(47): 14003-14010, 1994; Schullek et al., J Biol Chem 269(30): 19399-19403, 1994). Alanine substitution identified a limited number of residue positions at which alanine replacements cause 5- to 10-fold lower affinity for FVIIa binding. Most of these residue side chains were found to be well-exposed to solvent in the crystal structure, concordant with macromolecular ligand interaction. The FVIIa ligand-binding site is located over an extensive region at the boundary between the two modules. In the C-module, residues Arg¹³⁵ and Phe¹⁴⁰ located on the protruding B-C loop provide an independent contact with FVIIa. Leu¹³³ is located at the base of the fingerlike structure and packed into the cleft between the two modules. This provides continuity to a major cluster of important binding residues consisting of Lys²⁰, Thr⁶⁰, Asp⁵⁸, and Ile²². Thr⁶⁰ is only partially solvent-exposed and may play a local structural role rather than making a significant contact with ligand. The binding site extends onto the concave side of the intermodule angle involving Glu²⁴ and Gln¹¹⁰, and potentially the more distant residue Val²⁰⁷. The binding region extends from Asp58 onto a convex surface area formed by Lys⁴⁸, Lys⁴⁶, Gln³⁷, Asp⁴⁴, and Trp⁴⁵. Trp⁴⁵ and Asp⁴⁴ do not interact independently with FVIIa, indicating that the mutational effect at the Trp⁴⁵ position may reflect a structural importance of this side chain for the local packing of the adjacent Asp⁴⁴ and Gln³⁷ side chain. The interactive area further includes two surface-exposed aromatic residues, Phe⁷⁶ and Tyr⁷⁸, which form part of the hydrophobic cluster in the N-module.

The known physiologic substrates of tissue factor-FVIIa are FVII, FIX, and FX and certain proteinase-activated receptors. Mutational analysis has identified a number of residues that, when mutated, support full FVIIa amidolytic activity towards small peptidyl substrates but are deficient in their ability to support macromolecular substrate (i.e., FVII, FIX, and FX) activation (Ruf et al., J Biol Chem 267(31): 22206-22210, 1992; Ruf et al., J Biol Chem 267(9): 6375-6381, 1992; Huang et al., J Biol Chem 271(36): 21752-21757, 1996; Kirchhofer et al., Biochemistry 39(25): 7380-7387, 2000). The tissue factor loop region at residues 159-165, and residues in or adjacent to this flexible loop have been shown to be critical for the proteolytic activity of the tissue factor-FVIIa complex. This defines the proposed substrate-binding exosite region of tissue factor that is quite distant from the FVIIa active site. A substitution of the glycine residue by a marginally bulkier residue alanine, significantly impairs tissue factor-FVIIa proteolytic activity. This suggests that the flexibility afforded by glycine is critical for the loop of residues 159-165 for tissue factor macromolecular substrate recognition.

The residues Lys¹⁶⁵ and Lys¹⁶⁶ have also been demonstrated to be important for substrate recognition and binding. Mutation of either of these residues to alanine results in a significant decrease in the tissue factor co-factor function. Lys¹⁶⁵ and Lys¹⁶⁶ face away from each other, with Lys¹⁶⁵ pointing towards FVIIa in most tissue factor-FVIIa structures, and Lys¹⁶⁶ pointing into the substrate binding exosite region in the crystal structure. Putative salt bridge formation between Lys¹⁶⁵ of and Gla³⁵ of FVIIa would support the notion that tissue factor interaction with the GLA domain of FVIIa modulates substrate recognition. These results suggest that the C-terminal portion of the tissue factor ectodomain directly interacts with the GLA-domain, the possible adjacent EGF1 domains, of FIX and FX, and that the presence of the FVIIa GLA-domain may modulate these interactions either directly or indirectly.

### Anti-Tissue Factor Antibodies and Antigen-Binding Antibody Fragments

In some examples, an anti-tissue factor antibody is a humanized or fully human anti-tissue factor antibody (see, e.g., the anti-tissue factor antibodies described in U.S. Patent No. 7,968,094 and U.S. Patent No. 8,007,795).

In some embodiments, an anti-tissue factor antibody can include a heavy chain variable domain that includes the following set of CDR sequences: a CDR1 including DYNVY (SEQ ID NO: 1); a CDR2 including YIDPYNGITIYDQNFKG (SEQ ID NO: 2); and a CDR3 including DVTTALDF (SEQ ID NO: 3).

In some embodiments, an anti-tissue factor antibody can include a heavy chain variable domain that includes the following set of CDR sequences: a CDR1 including DYNVY (SEQ ID NO: 1); a CDR2 including YIDPYNGITIYDQNLKG (SEQ ID NO: 9); and a CDR3 including DVTTALDF (SEQ ID NO: 3).
**Exemplary heavy chain variable domain (SEQ ID NO: 7)** 
QIQLVQSGGEVKKPGASVRVSCKASGYSFTDYNVYWVRQSPGKGLEWIGYIDPY
NGITIYDQNFKGKATLTVDKSTSTAYMELSSLRSEDTAVYFCARDVTTALDFWG
QGTTVTVSS
**Exemplary heavy chain (SEQ ID NO: 127)** 
QIQLVQSGGEVKKPGASVRVSCKASGYSFTDYNVYWVRQSPGKGLEWIGYIDPY
NGITIYDQNFKGKATLTVDKSTSTAYMELSSLRSEDTAVYFCARDVTTALDFWG
QGTTVTVSSEFASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVE
 
WESNGQPENNYKTTPPVLDSDGSFFLYSKLTVDKSRWQQGNVFSCSVMHEALH
NHYTQKSLSLSPGK
**cDNA encoding exemplary heavy chain (SEQ ID NO: 128)**

CAGATCCAGCTGGTGCAGAGCGGCGGCGAGGTGAAGAAGCCCGGCGCCAGCGT
GAGGGTGAGCTGCAAGGCCAGCGGCTACAGCTTCACCGACTACAACGTGTACT
GGGTGAGGCAGAGCCCCGGCAAGGGCCTGGAGTGGATCGGCTACATCGACCCC
TACAACGGCATCACCATCTACGACCAGAACTTCAAGGGCAAGGCCACCCTGAC
CGTGGACAAGAGCACCAGCACCGCCTACATGGAGCTGAGCAGCCTGAGGAGCG
AGGACACCGCCGTGTACTTCTGCGCCAGGGACGTGACCACCGCCCTGGACTTC
TGGGGCCAGGGCACCACCGTGACCGTGAGCAGCGAGTTCGCCAGCACCAAGGG
CCCCAGCGTGTTCCCCCTGGCCCCCAGCAGCAAGAGCACCAGCGGCGGCACCG
CCGCCCTGGGCTGCCTGGTGAAGGACTACTTCCCCGAGCCCGTGACCGTGAGC
TGGAACAGCGGCGCCCTGACCAGCGGCGTGCACACCTTCCCCGCCGTGCTGCA
GAGCAGCGGCCTGTACAGCCTGAGCAGCGTGGTGACCGTGCCCAGCAGCAGCC
TGGGCACCCAGACCTACATCTGCAACGTGAACCACAAGCCCAGCAACACCAAG
GTGGACAAGAAGGTGGAGCCCAAGAGCTGCGACAAGACCCACACCTGCCCCCC
CTGCCCCGCCCCCGAGCTGCTGGGCGGCCCCAGCGTGTTCCTGTTCCCCCCCA
AGCCCAAGGACACCCTGATGATCAGCAGGACCCCCGAGGTGACCTGCGTGGTG
GTGGACGTGAGCCACGAGGACCCCGAGGTGAAGTTCAACTGGTACGTGGACGG
CGTGGAGGTGCACAACGCCAAGACCAAGCCCAGGGAGGAGCAGTACAACAGCA
CCTACAGGGTGGTGAGCGTGCTGACCGTGCTGCACCAGGACTGGCTGAACGGC
AAGGAGTACAAGTGCAAGGTGAGCAACAAGGCCCTGCCCGCCCCCATCGAGAA
GACCATCAGCAAGGCCAAGGGCCAGCCCAGGGAGCCCCAGGTGTACACCCTGC
CCCCCAGCAGGGACGAGCTGACCAAGAACCAGGTGAGCCTGACCTGCCTGGTG
AAGGGCTTCTACCCCAGCGACATCGCCGTGGAGTGGGAGAGCAACGGCCAGCC
CGAGAACAACTACAAGACCACCCCCCCCGTGCTGGACAGCGACGGCAGCTTCT

TCCTGTACAGCAAGCTGACCGTGGACAAGAGCAGGTGGCAGCAGGGCAACGTG
TTCAGCTGCAGCGTGATGCACGAGGCCCTGCACAACCACTACACCCAGAAGAG
CCTGAGCCTGAGCCCCGGCAAGTGA

In some embodiments, an anti-tissue factor antibody can include a heavy chain variable domain that includes a sequence that is at least 70% identical (e.g., at least 72%, at least 74%, at least 75%, at least 76%, at least 78%, at least 80%, at least 82%, at least 84%, at least 85%, at least 86%, at least 88%, at least 90%, at least 92%, at least 94%, at least 95%, at least 96%, at least 97%, at least 98%, at least 99%, or 100% identical) to SEQ ID NO: 7.

In some embodiments, an anti-tissue factor antibody can include a heavy chain variable domain that includes a sequence that is at least 70% identical (e.g., at least 72%, at least 74%, at least 75%, at least 76%, at least 78%, at least 80%, at least 82%, at least 84%, at least 85%, at least 86%, at least 88%, at least 90%, at least 92%, at least 94%, at least 95%, at least 96%, at least 97%, at least 98%, at least 99%, or 100% identical) to SEQ ID NO: 127.

In some embodiments, an anti-tissue factor antibody can include a light chain variable domain that includes the following set of CDR sequences: a CDR1 including LASQTIDTWLA (SEQ ID NO: 4); a CDR2 including AATNLAD (SEQ ID NO: 5); and a CDR3 including QQVYSSPFT (SEQ ID NO: 6).
**Exemplary light chain variable domain (SEQ ID NO: 8)** 
DIQMTQSPASLSASVGDRVTITCLASQTIDTWLAWYLQKPGKSPQLLIYAATNLA
DGVPSRFSGSGSGTDFSFTISSLQPEDFATYYCQQVYSSPFTFGQGTKLEIK
**Exemplary light chain (SEQ ID NO: 129)** 
DIQMTQSPASLSASVGRVTITCLASQTIDTWLAWLQKPGKSPQLLIYAATNLDGV
PSRFSGSGSGTDFSFISSLQPEDFATYYCQQVYSSPFTFGQGTKLEIKRTVAAPSVF
IFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC
**cDNA encoding an exemplary light chain (SEQ ID NO: 130)**

GACATCCAGATGACCCAGAGCCCCGCCAGCCTGAGCGCCAGCGTGGGCGACAG
GGTGACCATCACCTGCCTGGCCAGCCAGACCATCGACACCTGGCTGGCCTGG
TACCTGCAGAAGCCCGGCAAGAGCCCCCAGCTGCTGATCTACGCCGCCACCAA
CCTGGCCGACGGCGTGCCCAGCAGGTTCAGCGGCAGCGGCAGCGGCACCGACT
TCAGCTTCAACCATCAGCAGCCTGCAGCCCGAGGACTTCGCCACCTACTACTG
CCAGCAGGTGTACTAGCAGCCCCTTCACCTTCGGCCAGGGCACCAAGCTGGAG
ATCAAGAGGACCGTGGCCGCCCCCAGCGTGTTCATCTTCCCCCCCAGCGACGA
GCAGCTGAAGAGCGGCACCGCCAGCGTGGTGTGCCTGCTGAACAACTTCTACC
CCAGGGAGGCCAAGGTGCAGTGGAAGGTGGACAACGCCCTGCAGAGCGGCAAC
AGCCAGGAGAGCGTGACCGAGCAGGACAGCAAGGACAGCACCTACAGCCTGAG
CAGCACCCTGACCCTGAGCAAGGCCGACTACGAGAAGCACAAGGTGTACGCCT
GCGAGGTGACCCACCAGGGCCTGAGCAGCCCCGTGACCAAGAGCTTCAACAGG
GGCGAGTGCTGA

In some embodiments, an anti-tissue factor antibody can include a light chain variable domain that includes a sequence that is at least 70% identical (e.g., at least 72%, at least 74%, at least 75%, at least 76%, at least 78%, at least 80%, at least 82%, at least 84%, at least 85%, at least 86%, at least 88%, at least 90%, at least 92%, at least 94%, at least 95%, at least 96%, at least 97%, at least 98%, at least 99%, or 100% identical) to SEQ ID NO: 8.

In some embodiments, an anti-tissue factor antibody can include a light chain variable domain that includes a sequence that is at least 70% identical (e.g., at least 72%, at least 74%, at least 75%, at least 76%, at least 78%, at least 80%, at least 82%, at least 84%, at least 85%, at least 86%, at least 88%, at least 90%, at least 92%, at least 94%, at least 95%, at least 96%, at least 97%, at least 98%, at least 99%, or 100% identical) to SEQ ID NO: 129.

Non-limiting examples of anti-tissue factor antibodies can include: a heavy chain variable domain including a sequence that is at least 70% identical (e.g., at least 72%, at least 74%, at least 75%, at least 76%, at least 78%, at least 80%, at least 82%, at least 84%, at least 85%, at least 86%, at least 88%, at least 90%, at least 92%, at least 94%, at least 95%, at least 96%, at least 97%, at least 98%, at least 99%, or 100% identical) to SEQ ID NO: 7, and a light chain variable domain including a sequence that is at least 70% identical (e.g., at least 72%, at least 74%, at least 75%, at least 76%, at least 78%, at least 80%, at least 82%, at least 84%, at least 85%, at least 86%, at least 88%, at least 90%, at least 92%, at least 94%, at least 95%, at least 96%, at least 97%, at least 98%, at least 99%, or 100% identical) to SEQ ID NO: 8.

Non-limiting examples of anti-tissue factor antibodies can include: a heavy chain variable domain including a sequence that is at least 70% identical (e.g., at least 72%, at least 74%, at least 75%, at least 76%, at least 78%, at least 80%, at least 82%, at least 84%, at least 85%, at least 86%, at least 88%, at least 90%, at least 92%, at least 94%, at least 95%, at least 96%, at least 97%, at least 98%, at least 99%, or 100% identical) to SEQ ID NO: 127, and a light chain variable domain including a sequence that is at least 70% identical (e.g., at least 72%, at least 74%, at least 75%, at least 76%, at least 78%, at least 80%, at least 82%, at least 84%, at least 85%, at least 86%, at least 88%, at least 90%, at least 92%, at least 94%, at least 95%, at least 96%, at least 97%, at least 98%, at least 99%, or 100% identical) to SEQ ID NO: 129.

In some embodiments of any of the anti-tissue factor antibodies described herein, the anti-tissue factor antibody binds to tissue factor with a binding constant (K_{D}) of about 0.1 nM to about 1 µM (e.g., about 0.1 nM to about 800 nM, about 0.1 nM to about 750 nM, about 0.1 nM to about 700 nM, about 0.1 nM to about 650 nM, about 0.1 nM to about 600 nM, about 0.1 nM to about 550 nM, about 0.1 nM to about 500 nM, about 0.1 nM to about 450 nM, about 0.1 nM to about 400 nM, about 0.1 nM to about 350 nM, about 0.1 nM to about 300, about 0.1 nM to about 250 nM, about 0.1 nM to about 200 nM, about 0.1 nM to about 150 nM, about 0.1 nM to about 100 nM, about 0.1 nM to about 80 nM, about 0.1 nM to about 60 nM, about 0.1 nM to about 50 nM, about 0.1 nM to about 40 nM, about 0.1 nM to about 25 nm, about 0.1 nM to about 20 nM, about 0.1 nM to about 15 nM, about 0.1 nM to about 10 nM, about 0.1 nM to about 8 nM, about 0.1 nM to about 6 nM, about 0.1 nM to about 5 nM, about 0.1 nM to about 4 nM, about 0.1 nM to about 3 nM, about 0.1 nM to about 2 nM, about 0.1 nM to about 1 nM, about 0.1 nM to about 0.8 nM, about 0.1 nM to about 0.6 nM, about 0.1 nM to about 0.4 nM, about 0.1 nM to about 0.2 nM; about 0.2 nM to about 1 µM, about 0.2 nM to about 800 nM, about 0.2 nM to about 750 nM, about 0.2 nM to about 700 nM, about 0.2 nM to about 650 nM, about 0.2 nM to about 600 nM, about 0.2 nM to about 550 nM, about 0.2 nM to about 500 nM, about 0.2 nM to about 450 nM, about 0.2 nM to about 400 nM, about 0.2 nM to about 350 nM, about 0.2 nM to about 300, about 0.2 nM to about 250 nM, about 0.2 nM to about 200 nM, about 0.2 nM to about 150 nM, about 0.2 nM to about 100 nM, about 0.2 nM to about 80 nM, about 0.2 nM to about 60 nM, about 0.2 nM to about 50 nM, about 0.2 nM to about 40 nM, about 0.2 nM to about 25 nm, about 0.2 nM to about 20 nM, about 0.2 nM to about 15 nM, about 0.2 nM to about 10 nM, about 0.2 nM to about 8 nM, about 0.2 nM to about 6 nM, about 0.2 nM to about 5 nM, about 0.2 nM to about 4 nM, about 0.2 nM to about 3 nM, about 0.2 nM to about 2 nM, about 0.2 nM to about 1 nM, about 0.2 nM to about 0.8 nM, about 0.2 nM to about 0.6 nM, about 0.2 nM to about 0.4 nM, about 0.2 nM to about 0.2 nM, about 0.4 nM to about 1 µM, about 0.4 nM to about 800 nM, about 0.4 nM to about 750 nM, about 0.4 nM to about 700 nM, about 0.4 nM to about 650 nM, about 0.4 nM to about 600 nM, about 0.4 nM to about 550 nM, about 0.4 nM to about 500 nM, about 0.4 nM to about 450 nM, about 0.4 nM to about 400 nM, about 0.4 nM to about 350 nM, about 0.4 nM to about 300, about 0.4 nM to about 250 nM, about 0.4 nM to about 200 nM, about 0.4 nM to about 150 nM, about 0.4 nM to about 100 nM, about 0.4 nM to about 80 nM, about 0.4 nM to about 60 nM, about 0.4 nM to about 50 nM, about 0.4 nM to about 40 nM, about 0.4 nM to about 25 nm, about 0.4 nM to about 20 nM, about 0.4 nM to about 15 nM, about 0.4 nM to about 10 nM, about 0.4 nM to about 8 nM, about 0.4 nM to about 6 nM, about 0.4 nM to about 5 nM, about 0.4 nM to about 4 nM, about 0.4 nM to about 3 nM, about 0.4 nM to about 2 nM, about 0.4 nM to about 1 nM, about 0.4 nM to about 0.8 nM, about 0.4 nM to about 0.6 nM, about 0.5 nM to about 1 µM, about 0.5 nM to about 800 nM, about 0.5 nM to about 750 nM, about 0.5 nM to about 700 nM, about 0.5 nM to about 650 nM, about 0.5 nM to about 600 nM, about 0.5 nM to about 550 nM, about 0.5 nM to about 500 nM, about 0.5 nM to about 450 nM, about 0.5 nM to about 400 nM, about 0.5 nM to about 350 nM, about 0.5 nM to about 300, about 0.5 nM to about 250 nM, about 0.5 nM to about 200 nM, about 0.5 nM to about 150 nM, about 0.5 nM to about 100 nM, about 0.5 nM to about 80 nM, about 0.5 nM to about 60 nM, about 0.5 nM to about 50 nM, about 0.5 nM to about 40 nM, about 0.5 nM to about 25 nm, about 0.5 nM to about 20 nM, about 0.5 nM to about 15 nM, about 0.5 nM to about 10 nM, about 0.5 nM to about 8 nM, about 0.5 nM to about 6 nM, about 0.5 nM to about 5 nM, about 0.5 nM to about 4 nM, about 0.5 nM to about 3 nM, about 0.5 nM to about 2 nM, about 0.5 nM to about 1 nM, about 0.5 nM to about 0.8 nM, about 0.5 nM to about 0.6 nM, about 0.6 nM to about 1 µM, about 0.6 nM to about 800 nM, about 0.6 nM to about 750 nM, about 0.6 nM to about 700 nM, about 0.6 nM to about 650 nM, about 0.6 nM to about 600 nM, about 0.6 nM to about 550 nM, about 0.6 nM to about 500 nM, about 0.6 nM to about 450 nM, about 0.6 nM to about 400 nM, about 0.6 nM to about 350 nM, about 0.6 nM to about 300, about 0.6 nM to about 250 nM, about 0.6 nM to about 200 nM, about 0.6 nM to about 150 nM, about 0.6 nM to about 100 nM, about 0.6 nM to about 80 nM, about 0.6 nM to about 60 nM, about 0.6 nM to about 50 nM, about 0.6 nM to about 40 nM, about 0.6 nM to about 25 nm, about 0.6 nM to about 20 nM, about 0.6 nM to about 15 nM, about 0.6 nM to about 10 nM, about 0.6 nM to about 8 nM, about 0.6 nM to about 6 nM, about 0.6 nM to about 5 nM, about 0.6 nM to about 4 nM, about 0.6 nM to about 3 nM, about 0.6 nM to about 2 nM, about 0.6 nM to about 1 nM, about 0.6 nM to about 0.8 nM, about 1 nM to about 1 µM, about 1 nM to about 800 nM, about 1 nM to about 750 nM, about 1 nM to about 700 nM, about 1 nM to about 650 nM, about 1 nM to about 600 nM, about 1 nM to about 550 nM, about 1 nM to about 500 nM, about 1 nM to about 450 nM, about 1 nM to about 400 nM, about 1 nM to about 350 nM, about 1 nM to about 300, about 1 nM to about 250 nM, about 1 nM to about 200 nM, about 1 nM to about 150 nM, about 1 nM to about 100 nM, about 1 nM to about 80 nM, about 1 nM to about 60 nM, about 1 nM to about 50 nM, about 1 nM to about 40 nM, about 1 nM to about 25 nm, about 1 nM to about 20 nM, about 1 nM to about 15 nM, about 1 nM to about 10 nM, about 1 nM to about 8 nM, about 1 nM to about 6 nM, about 1 nM to about 5 nM, about 1 nM to about 4 nM, about 1 nM to about 3 nM, about 1 nM to about 2 nM, about 2 nM to about 1 µM, about 2 nM to about 800 nM, about 2 nM to about 750 nM, about 2 nM to about 700 nM, about 2 nM to about 650 nM, about 2 nM to about 600 nM, about 2 nM to about 550 nM, about 2 nM to about 500 nM, about 2 nM to about 450 nM, about 2 nM to about 400 nM, about 2 nM to about 350 nM, about 2 nM to about 300, about 2 nM to about 250 nM, about 2 nM to about 200 nM, about 2 nM to about 150 nM, about 2 nM to about 100 nM, about 2 nM to about 80 nM, about 2 nM to about 60 nM, about 2 nM to about 50 nM, about 2 nM to about 40 nM, about 2 nM to about 25 nm, about 2 nM to about 20 nM, about 2 nM to about 15 nM, about 2 nM to about 10 nM, about 2 nM to about 8 nM, about 2 nM to about 6 nM, about 2 nM to about 5 nM, about 2 nM to about 4 nM, about 2 nM to about 3 nM, about 5 nM to about 1 µM, about 5 nM to about 800 nM, about 5 nM to about 750 nM, about 5 nM to about 700 nM, about 5 nM to about 650 nM, about 5 nM to about 600 nM, about 5 nM to about 550 nM, about 5 nM to about 500 nM, about 5 nM to about 450 nM, about 5 nM to about 400 nM, about 5 nM to about 350 nM, about 5 nM to about 300, about 5 nM to about 250 nM, about 5 nM to about 200 nM, about 5 nM to about 150 nM, about 5 nM to about 100 nM, about 5 nM to about 80 nM, about 5 nM to about 60 nM, about 5 nM to about 50 nM, about 5 nM to about 40 nM, about 5 nM to about 25 nm, about 5 nM to about 20 nM, about 5 nM to about 15 nM, about 5 nM to about 10 nM, about 5 nM to about 8 nM, about 5 nM to about 6 nM, about 10 nM to about 1 µM, about 10 nM to about 800 nM, about 10 nM to about 750 nM, about 10 nM to about 700 nM, about 10 nM to about 650 nM, about 10 nM to about 600 nM, about 10 nM to about 550 nM, about 10 nM to about 500 nM, about 10 nM to about 450 nM, about 10 nM to about 400 nM, about 10 nM to about 350 nM, about 10 nM to about 300, about 10 nM to about 250 nM, about 10 nM to about 200 nM, about 10 nM to about 150 nM, about 10 nM to about 100 nM, about 10 nM to about 80 nM, about 10 nM to about 60 nM, about 10 nM to about 50 nM, about 10 nM to about 40 nM, about 10 nM to about 25 nm, about 10 nM to about 20 nM, about 10 nM to about 15 nM, about 20 nM to about 1 µM, about 20 nM to about 800 nM, about 20 nM to about 750 nM, about 20 nM to about 700 nM, about 20 nM to about 650 nM, about 20 nM to about 600 nM, about 20 nM to about 550 nM, about 20 nM to about 500 nM, about 20 nM to about 450 nM, about 20 nM to about 400 nM, about 20 nM to about 350 nM, about 20 nM to about 300, about 20 nM to about 250 nM, about 20 nM to about 200 nM, about 20 nM to about 150 nM, about 20 nM to about 100 nM, about 20 nM to about 80 nM, about 20 nM to about 60 nM, about 20 nM to about 50 nM, about 20 nM to about 40 nM, about 50 nM to about 1 µM, about 50 nM to about 800 nM, about 50 nM to about 750 nM, about 50 nM to about 700 nM, about 50 nM to about 650 nM, about 50 nM to about 600 nM, about 50 nM to about 550 nM, about 50 nM to about 500 nM, about 50 nM to about 450 nM, about 50 nM to about 400 nM, about 50 nM to about 350 nM, about 50 nM to about 300, about 50 nM to about 250 nM, about 50 nM to about 200 nM, about 50 nM to about 150 nM, about 50 nM to about 100 nM, about 50 nM to about 80 nM, about 100 nM to about 1 µM, about 100 nM to about 800 nM, about 100 nM to about 750 nM, about 100 nM to about 700 nM, about 100 nM to about 650 nM, about 100 nM to about 600 nM, about 100 nM to about 550 nM, about 100 nM to about 500 nM, about 100 nM to about 450 nM, about 100 nM to about 400 nM, about 100 nM to about 350 nM, about 100 nM to about 300, about 100 nM to about 250 nM, about 100 nM to about 200 nM, about 100 nM to about 150 nM, about 250 nM to about 1 µM, about 250 nM to about 800 nM, about 250 nM to about 750 nM, about 250 nM to about 700 nM, about 250 nM to about 650 nM, about 250 nM to about 600 nM, about 250 nM to about 550 nM, about 250 nM to about 500 nM, about 250 nM to about 450 nM, about 250 nM to about 400 nM, about 250 nM to about 350 nM, about 250 nM to about 300, about 400 nM to about 1 µM, about 400 nM to about 800 nM, about 400 nM to about 750 nM, about 400 nM to about 700 nM, about 400 nM to about 650 nM, about 400 nM to about 600 nM, about 400 nM to about 550 nM, about 400 nM to about 500 nM, about 400 nM to about 450 nM, about 500 nM to about 1 µM, about 500 nM to about 800 nM, about 500 nM to about 750 nM, about 500 nM to about 700 nM, about 500 nM to about 650 nM, about 500 nM to about 600 nM, about 500 nM to about 550 nM, about 600 nM to about 1 µM, about 600 nM to about 800 nM, about 600 nM to about 750 nM, about 600 nM to about 700 nM, about 600 nM to about 650 nM, about 750 nM to about 1 µM, about 750 nM to about 800 nM, about 800 nM to about 1 µM, or about 950 nM to about 1 µM).

In some embodiments, the anti-tissue factor antibody can be an IgG (e.g., IgG1, IgG2, IgG3, and IgG4) (e.g., human IgG1, human IgG2, human IgG3, and human IgG4), an IgA (e.g., a human IgA), an IgE (e.g., a human IgE), or an IgM (e.g., a human IgM).

In some embodiments, the anti-tissue factor antibody is a human or humanized IgG1 antibody. In some embodiments, the anti-tissue factor antibody is a human or humanized IgG4 antibody.

In some embodiments, the anti-tissue factor antibody can include an IgG1 Fc region. In some embodiments, the anti-tissue factor antibody can include a heavy chain constant domain comprising a sequence that is at least 70% identical (e.g., at least 75% identical, at least 80% identical, at least 85% identical, at least 90% identical, at least 95% identical, at least 99% identical, or 100% identical) to SEQ ID NO: 102 or SEQ ID NO: 103.
**Heavy Chain Constant Region (SEQ** ID NO: 102) 
EFASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
**Heavy Chain Constant Region (SEQ ID NO: 103)**

EFASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPSC
PAPEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K

### Affinity Chromatography Resin

Provided herein is an affinity chromatography resin comprising an anti-tissue factor antibody or antigen-binding fragment thereof comprising a heavy chain variable domain comprising CDRs of SEQ ID NOs: 1, 2 or 9, and 3, and a light chain variable domain comprising CDRs of SEQ ID NOs: 4, 5, and 6, attached to a base resin. In some embodiments, the heavy chain variable domain comprises a sequence that is at least 90% identical to SEQ ID NO: 7, and the light chain variable domain comprises a sequence that is at least 90% identical to SEQ ID NO: 8. In some embodiments, the heavy chain variable domain comprises SEQ ID NO: 7, and the light chain variable domain comprises SEQ ID NO: 8.

In some embodiments, the base resin is Sepharose. In some embodiments, the base resin is Capto or agarose. In some embodiments, the base resin (or matrix or stationary phase) is a natural polymer including but not limited to cellulose, agarose or chitosan; a synthetic polymer including but not limited to acrylomido and vinyl copolymers, acrylic polymers or poly-metacrylate, poly(styrene-divinyl-benzene) copolymers; or an inorganic material, including but not limited to hydroxyapatite, silica, glass, or zirconia. In some embodiments, the anti-tissue factor antibody or antigen-binding fragment thereof is non-covalently attached to the base resin. In some embodiments, the anti-tissue factor antibody or antigen-binding fragment thereof is covalently attached to the base resin. In some embodiments, the anti-tissue factor antibody or antigen-binding fragment thereof is covalently attached to the base resin through the formation of a disulfide bond between a cysteine in the anti-tissue factor antibody or antigen-binding fragment thereof and a chemical group on the base resin. In some embodiments, the anti-tissue factor antibody or antigen-binding fragment thereof is covalently attached to the base resin through the formation of a covalent bond between a free amine of the anti-tissue factor antibody or antigen-binding domain and a chemical group on the base resin. In some embodiments, the base resin is CNBr-activated or N-hydroxysuccinimide (NHS)-activated solid support material. In some embodiments, the covalent bond is represented by Formula I below: 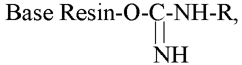 wherein, R represents the anti-tissue factor antibody or antigen-binding fragment thereof.

In some embodiments, NHS-activated Sepharose is designed for the covalent coupling of ligands (often antigens or antibodies) containing primary amino groups (the most common form of attachment). In some embodiments, the matrix of NHS-activated Sepharose is based on highly cross-linked agarose beads with 10-atom spacer arms (6-aminohexanoic acid) attached by epichlorohydrin and activated by N-hydroxysuccinimide (FIG. 7). In some embodiments, the matrix of NHS-activated Sepharose 4 Fast Flow is based on highly cross-linked agarose beads with 14-atom spacer arms. In some embodiments, nonspecific adsorption of proteins to NHS-activated Sepharose (which can reduce binding capacity of the target protein) is negligible due to the excellent hydrophilic properties of the base matrix. In some embodiments, the matrix is stable at high pH to allow stringent washing procedures (subject to the pH stability of the coupled ligand). In some embodiments, ligands containing amino groups such as proteins couple rapidly and spontaneously by nucleophilic attack at the ester linkage to give a very stable amide linkage (FIG. 8). In some embodiments, the amide bond is stable up to pH 13.0, making NHS-activated Sepharose suitable for applications that require conditions at high pH.

In some embodiments, CNBr-activated Sepharose is a pre-activated resin for coupling antibodies or other large proteins containing -NH₂ groups to the Sepharose media, by the cyanogen bromide method, without an intermediate spacer arm. In some embodiments, the CNBr-activated Sepharose is used for immobilizing antibodies or other large proteins containing -NH2 groups by coupling them to the matrix of CNBr-activated Sepharose. In some embodiments, CNBr-activated Sepharose 4 Fast Flow is a bead-formed, highly cross-linked pre-activated matrix produced by reacting Sepharose 4 Fast Flow with cyanogen bromide (CNBr). In some embodiments, CNBr-activated Sepharose 4 Fast Flow is based on antigen-antibody reactions with immobilized monoclonal antibodies as ligands.

In some embodiments, a kit comprises an affinity chromatography resin of any one of the affinity chromatography resins described herein.

Antibody (e.g., monoclonal or polyclonal) based immunoaffinity chromatography is a widely used method/process to purify specific antigens or antigen-containing proteins (e.g., human tissue factor-containing proteins) using affinity chromatography resin comprising an antibody or antigen-binding fragment attached to the base resin. For the immunoaffinity method, an antibody (e.g., monoclonal) or a mixture of antibodies (e.g., polyclonal) is immobilized (e.g., coupled) covalently onto solid support materials (e.g., base resin) such as CNBr-activated Sepharose, NHS-Sepharose, and other related beads/resins suitable for protein coupling or immobilization. In some embodiments, a base resin is any material to which a biospecific ligand is covalently attached. In some embodiments, a base resin is any material to which a biospecific ligand is non-covalently attached. In some embodiments, examples of the base resin used in affinity chromatography include agarose, Capto, cellulose, ceramics, dextran, polystyrene, polyacrylamide, silica, latex, controlled pore glass, and synthetic/organic polymers. Useful base resins can have properties such as high surface-area to volume ratio, chemical groups that are easily modified for covalent attachment of ligands, minimal nonspecific binding properties, good flow characteristics and mechanical and chemical stability.

In some embodiments, the base resin is a sulfhydryl coupling resin, wherein an antibody or antigen-binding fragment thereof is covalently attached to the base resin through free sulfhydryls and an iodoacetyl group on the base resin. In some embodiments, the base resin is an amine coupling resin, wherein the amine coupling resin has reactive aldehyde groups that react with primary amines to form Schiff's bases which are then reduced with a suitable reducing agent (e.g., sodium cyanoborohydride) to form a covalent bond between an antibody or antigen-binding fragment thereof and the base resin. In some embodiments, the base resin is an NHS-activated resin, wherein an antibody or antigen-binding fragment binds to the resin through a reactive NHS group and primary amines forming a covalent bond. In some embodiments, the base resin is an immobilized protein A, immobilized protein B, or immobilized protein A/G, wherein the base resin binds the constant domain of an antibody or antigen-binding fragment thereof ensuring the antigen binding domain is facing away from the base resin for optimal binding.

In some embodiments, a chromatography column is used for affinity chromatography wherein the chromatography column comprises an affinity chromatography resin selected from the group consisting of: CNBr-activated Sepharose, NHS-Sepharose, agarose, Capto, cellulose, ceramics, dextran, polystyrene, polyacrylamide, silica, latex, controlled pore glass, and synthetic/organic polymers.

### Methods of Purifying a Tissue Factor-Containing Protein

Provided herein are methods of purifying a tissue factor-containing protein comprising the use of any of the affinity chromatography resins described herein. In some embodiments, the method comprises: loading the affinity chromatography resin with a liquid comprising the tissue factor-containing protein; washing the affinity chromatography resin using one or more wash buffer(s); and eluting the tissue factor-containing protein using an elution buffer. In some embodiments, the liquid comprising the tissue factor-containing protein is a clarified liquid culture medium. In some embodiments, the liquid comprising the tissue factor-containing protein comprises a cell lysate.

In some embodiments, the one or more wash buffer(s) are: (i) a first wash buffer comprising phosphate buffered saline; and (ii) a second wash buffer comprising about 0.01 M to about 0.2 M citrate and having a pH of about 4.5 to about 5.5. In some embodiments, the first wash buffer is phosphate buffered saline and the second wash buffer is 0.1 M citrate, pH 5.0. In some embodiments, the elution buffer comprises 0.01 M to about 0.2 M acetate and has a pH of about 2.5 to about 3.5. In some embodiments, the elution buffer comprises 0.1 M acetate and has a pH of about 2.9.

In some embodiments, the second wash buffer comprises about 0.01 M to about 0.2 M (e.g., about 0.01 M to about 0.18 M, about 0.01 M to about 0.16 M, about 0.01 M to about 0.14 M, about 0.01 M to about 0.12 M, about 0.01 M to about 0.1 M, about 0.01 M to about 0.08 M, about 0.01 M to about 0.06 M, about 0.01 M to about 0.05 M, about 0.01 M to about 0.04 M, about 0.01 M to about 0.03 M, about 0.01 M to about 0.02 M, about 0.02 M to about 0.2 M, about 0.02 M to about 0.18 M, about 0.02 M to about 0.16 M, about 0.02 M to about 0.14 M, about 0.02 M to about 0.12 M, about 0.02 M to about 0.1 M, about 0.02 M to about 0.08 M, about 0.02 M to about 0.06 M, about 0.02 M to about 0.05 M, about 0.02 M to about 0.04 M, about 0.02 M to about 0.03 M, about 0.03 M to about 0.2 M, about 0.03 M to about 0.18 M, about 0.03 M to about 0.16 M, about 0.03 M to about 0.14 M, about 0.03 M to about 0.12 M, about 0.03 M to about 0.1 M, about 0.03 M to about 0.08 M, about 0.03 M to about 0.06 M, about 0.03 M to about 0.05 M, about 0.03 M to about 0.04 M, about 0.04 M to about 0.2 M, about 0.04 M to about 0.18 M, about 0.04 M to about 0.16 M, about 0.04 M to about 0.14 M, about 0.04 M to about 0.12 M, about 0.04 M to about 0.1 M, about 0.04 M to about 0.08 M, about 0.04 M to about 0.06 M, about 0.04 M to about 0.05 M, about 0.05 M to about 0.2 M, about 0.05 M to about 0.18 M, about 0.05 M to about 0.16 M, about 0.05 M to about 0.14 M, about 0.05 M to about 0.12 M, about 0.05 M to about 0.1 M, about 0.05 M to about 0.08 M, about 0.05 M to about 0.06 M, about 0.06 M to about 0.2 M, about 0.06 M to about 0.18 M, about 0.06 M to about 0.16 M, about 0.06 M to about 0.14 M, about 0.06 M to about 0.12 M, about 0.06 M to about 0.1 M, about 0.06 M to about 0.08 M, about 0.08 M to about 0.2 M, about 0.08 M to about 0.18 M, about 0.08 M to about 0.16 M, about 0.08 M to about 0.14 M, about 0.08 M to about 0.12 M, about 0.08 M to about 0.1 M, about 0.1 M to about 0.2 M, about 0.1 M to about 0.18 M, about 0.1 M to about 0.16 M, about 0.1 M to about 0.14 M, about 0.1 M to about 0.12 M, about 0.12 M to about 0.2 M, about 0.12 M to about 0.18 M, about 0.12 M to about 0.16 M, about 0.12 M to about 0.14 M, about 0.14 M to about 0.2 M, about 0.14 M to about 0.18 M, about 0.14 M to about 0.16 M, about 0.16 M to about 0.2 M, about 0.16 M to about 0.18 M, or about 0.18 M to about 0.2 M) citrate.

In some embodiments, the second wash buffer has a pH of about 4.5 to about 5.5 (e.g., about 4.5 to about 5.4, about 4.5 to about 5.3, about 4.5 to about 5.2, about 4.5 to about 5.1, about 4.5 to about 5.0, about 4.5 to about 4.9, about 4.5 to about 4.8, about 4.5 to about 4.7, about 4.5 to about 4.6, about 4.6 to about 5.5, about 4.6 to about 5.4, about 4.6 to about 5.3, about 4.6 to about 5.2, about 4.6 to about 5.1, about 4.6 to about 5.0, about 4.6 to about 4.9, about 4.6 to about 4.8, about 4.6 to about 4.7, about 4.7 to about 5.5, about 4.7 to about 5.4, about 4.7 to about 5.3, about 4.7 to about 5.2, about 4.7 to about 5.1, about 4.7 to about 5.0, about 4.7 to about 4.9, about 4.7 to about 4.8, about 4.8 to about 5.5, about 4.8 to about 5.4, about 4.8 to about 5.3, about 4.8 to about 5.2, about 4.8 to about 5.1, about 4.8 to about 5.0, about 4.8 to about 4.9, about 4.9 to about 5.5, about 4.9 to about 5.4, about 4.9 to about 5.3, about 4.9 to about 5.2, about 4.9 to about 5.1, about 4.9 to about 5.0, about 5.0 to about 5.5, about 5.0 to about 5.4, about 5.0 to about 5.3, about 5.0 to about 5.2, about 5.0 to about 5.1, about 5.1 to about 5.5, about 5.1 to about 5.4, about 5.1 to about 5.3, about 5.1 to about 5.2, about 5.2 to about 5.5, about 5.2 to about 5.4, about 5.2 to about 5.3, about 5.3 to about 5.5, about 5.3 to about 5.4, or about 5.4 to about 5.5).

In some embodiments, the elution buffer comprises about 0.01 M to about 0.2 M (e.g., about 0.01 M to about 0.18 M, about 0.01 M to about 0.16 M, about 0.01 M to about 0.14 M, about 0.01 M to about 0.12 M, about 0.01 M to about 0.1 M, about 0.01 M to about 0.08 M, about 0.01 M to about 0.06 M, about 0.01 M to about 0.05 M, about 0.01 M to about 0.04 M, about 0.01 M to about 0.03 M, about 0.01 M to about 0.02 M, about 0.02 M to about 0.2 M, about 0.02 M to about 0.18 M, about 0.02 M to about 0.16 M, about 0.02 M to about 0.14 M, about 0.02 M to about 0.12 M, about 0.02 M to about 0.1 M, about 0.02 M to about 0.08 M, about 0.02 M to about 0.06 M, about 0.02 M to about 0.05 M, about 0.02 M to about 0.04 M, about 0.02 M to about 0.03 M, about 0.03 M to about 0.2 M, about 0.03 M to about 0.18 M, about 0.03 M to about 0.16 M, about 0.03 M to about 0.14 M, about 0.03 M to about 0.12 M, about 0.03 M to about 0.1 M, about 0.03 M to about 0.08 M, about 0.03 M to about 0.06 M, about 0.03 M to about 0.05 M, about 0.03 M to about 0.04 M, about 0.04 M to about 0.2 M, about 0.04 M to about 0.18 M, about 0.04 M to about 0.16 M, about 0.04 M to about 0.14 M, about 0.04 M to about 0.12 M, about 0.04 M to about 0.1 M, about 0.04 M to about 0.08 M, about 0.04 M to about 0.06 M, about 0.04 M to about 0.05 M, about 0.05 M to about 0.2 M, about 0.05 M to about 0.18 M, about 0.05 M to about 0.16 M, about 0.05 M to about 0.14 M, about 0.05 M to about 0.12 M, about 0.05 M to about 0.1 M, about 0.05 M to about 0.08 M, about 0.05 M to about 0.06 M, about 0.06 M to about 0.2 M, about 0.06 M to about 0.18 M, about 0.06 M to about 0.16 M, about 0.06 M to about 0.14 M, about 0.06 M to about 0.12 M, about 0.06 M to about 0.1 M, about 0.06 M to about 0.08 M, about 0.08 M to about 0.2 M, about 0.08 M to about 0.18 M, about 0.08 M to about 0.16 M, about 0.08 M to about 0.14 M, about 0.08 M to about 0.12 M, about 0.08 M to about 0.1 M, about 0.1 M to about 0.2 M, about 0.1 M to about 0.18 M, about 0.1 M to about 0.16 M, about 0.1 M to about 0.14 M, about 0.1 M to about 0.12 M, about 0.12 M to about 0.2 M, about 0.12 M to about 0.18 M, about 0.12 M to about 0.16 M, about 0.12 M to about 0.14 M, about 0.14 M to about 0.2 M, about 0.14 M to about 0.18 M, about 0.14 M to about 0.16 M, about 0.16 M to about 0.2 M, about 0.16 M to about 0.18 M, or about 0.18 M to about 0.2 M) acetate.

In some embodiments, the elution buffer has a pH of about 2.5 to about 3.5 (e.g., about 2.5 to about 3.4, about 2.5 to about 3.3, about 2.5 to about 3.2, about 2.5 to about 3.1, about 2.5 to about 3.0, about 2.5 to about 2.9, about 2.5 to about 2.8, about 2.5 to about 2.7, about 2.5 to about 2.6, about 2.6 to about 3.5, about 2.6 to about 3.4, about 2.6 to about 3.3, about 2.6 to about 3.2, about 2.6 to about 3.1, about 2.6 to about 3.0, about 2.6 to about 2.9, about 2.6 to about 2.8, about 2.6 to about 2.7, about 2.7 to about 3.5, about 2.7 to about 3.4, about 2.7 to about 3.3, about 2.7 to about 3.2, about 2.7 to about 3.1, about 2.7 to about 3.0, about 2.7 to about 2.9, about 2.7 to about 2.8, about 2.8 to about 3.5, about 2.8 to about 3.4, about 2.8 to about 3.3, about 2.8 to about 3.2, about 2.8 to about 3.1, about 2.8 to about 3.0, about 2.8 to about 2.9, about 2.9 to about 3.5, about 2.9 to about 3.4, about 2.9 to about 3.3, about 2.9 to about 3.2, about 2.9 to about 3.1, about 2.9 to about 3.0, about 3.0 to about 3.5, about 3.0 to about 3.4, about 3.0 to about 3.3, about 3.0 to about 3.2, about 3.0 to about 3.1, about 3.1 to about 3.5, about 3.1 to about 3.4, about 3.1 to about 3.3, about 3.1 to about 3.2, about 3.2 to about 3.5, about 3.2 to about 3.4, about 3.2 to about 3.3, about 3.3 to about 3.5, about 3.3 to about 3.4, or about 3.4 to about 3.5).

### Methods of Manufacturing a Tissue Factor-Containing Protein

Provided herein are methods of manufacturing a tissue factor-containing protein comprising: (i) purifying a tissue factor-containing protein using any of the methods described herein; and (ii) performing one or more addition unit operations on an eluate obtained from step (i). Non-limiting examples of unit operations that can be performed in a process for manufacturing a tissue factor-containing protein include: capturing the protein, purifying the protein, polishing the protein, inactivating viruses, adjusting the ionic concentration and/or pH of a fluid containing the protein, and filtering a fluid containing the protein. A non-limiting method of manufacturing a tissue factor-containing protein is depicted in FIG. 1.

In some embodiments, the one or more additional unit operations includes, in sequential order: performing low pH viral inactivation, performing depth filtration, performing polishing chromatography, performing nanofiltration, and performing ultrafiltration and diafiltration (UF/DF).

Also provided herein is a tissue factor-containing protein manufactured by any of the methods described herein. In some embodiments, a pharmaceutical composition comprises the manufactured tissue factor-containing protein.

### Single-Chain Chimeric Polypeptides

Non-limiting examples of tissue factor-containing proteins are single-chain chimeric polypeptides that include: (i) a first target-binding domain (e.g., any of the target-binding domains described herein or known in the art), (ii) a soluble tissue factor domain (e.g., any of the exemplary soluble tissue factor domains described herein or known in the art), and (iii) as second target-binding domain (e.g., any of the target-binding domains described herein or known in the art).

In some embodiments of any of the single-chain chimeric polypeptides described herein, the first target-binding domain (e.g., any of the exemplary target-binding domains described herein or known in the art) and the soluble tissue factor domain (e.g., any of the exemplary soluble tissue factor domains described herein) directly abut each other. In some embodiments of any of the single-chain chimeric polypeptides described herein, the single-chain chimeric polypeptide further comprises a linker sequence (e.g., any of the exemplary linker sequences described herein or known in the art) between the first target-binding domain (e.g., any of the exemplary target-binding domains described herein or known in the art) and the soluble tissue factor domain (e.g., any of the exemplary soluble tissue factor domains described herein). In some embodiments of any of the single-chain chimeric polypeptides described herein, the soluble tissue factor domain (e.g., any of the exemplary soluble tissue factor domains described herein) and the second target-binding domain (e.g., any of the exemplary target-binding domains described herein or known in the art) directly abut each other. In some embodiments of any of the single-chain chimeric polypeptides described herein, the single-chain chimeric polypeptide further comprises a linker sequence (e.g., any of the exemplary linker sequences described herein or known in the art) between the soluble tissue factor domain (e.g., any of the exemplary soluble tissue factor domains described herein) and the second target-binding domain (e.g., any of the exemplary target-binding domains described herein or known in the art).

In some embodiments of any of the single-chain chimeric polypeptides described herein, the first target-binding domain (e.g., any of the exemplary target-binding domains described herein or known in the art) and the second target-binding domain (e.g., any of the exemplary target-binding domains described herein or known in the art) directly abut each other. In some embodiments of any of the single-chain chimeric polypeptides described herein, the single-chain chimeric polypeptide further comprises a linker sequence (e.g., any of the exemplary linker sequences described herein or known in the art) between the first target-binding domain (e.g., any of the exemplary target-binding domains described herein or known in the art) and the second target-binding domain (e.g., any of the exemplary target-binding domains described herein or known in the art). In some embodiments of any of the single-chain chimeric polypeptides described herein, the second target-binding domain (e.g., any of the exemplary target-binding domains described herein or known in the art) and the soluble tissue factor domain (e.g., any of the exemplary soluble tissue factor domains described herein) directly abut each other. In some embodiments of any of the single-chain chimeric polypeptides described herein, the single-chain chimeric polypeptide further comprises a linker sequence (e.g., any of the exemplary linker sequences described herein or known in the art) between the second target-binding domain (e.g., any of the exemplary target-binding domains described herein or known in the art) and the soluble tissue factor domain (e.g., any of the exemplary soluble tissue factor domains described herein or known in the art).

Some embodiments of any of the single-chain chimeric polypeptides described herein can further include one or more (e.g., two, three, four, five, six, seven, eight, nine, or ten) additional target-binding domains (e.g., any of the exemplary target-binding domains described herein or known in the art) at its N- and/or C-terminus.

In some embodiments, the single-chain chimeric polypeptides can include one or more (e.g., two, three, four, five, six, seven, eight, nine, or ten) additional target-binding domains (e.g., any of the exemplary target-binding domains described herein or known in the art) at its N-terminus. In some embodiments, one of the one or more additional target-binding domains (e.g., any of the exemplary target-binding domains described herein or known in the art) at the N-terminus of the single-chain chimeric polypeptide can directly abut the first target-binding domain (e.g., any of the exemplary target-binding domains described herein or known in the art), the second target-binding domain (e.g., any of the exemplary target-binding domains described herein or known in the art), or the soluble tissue factor domain (e.g., any of the exemplary soluble tissue factor domains described herein). In some embodiments, the single-chain chimeric polypeptide further includes a linker sequence (e.g., any of the exemplary linker sequences described herein or known in the art) between one of the at least one additional target-binding domains (e.g., any of the exemplary target-binding domains described herein or known in the art) at the N-terminus of the single-chain chimeric polypeptide and the first target-binding domain (e.g., any of the exemplary target-binding domains described herein or known in the art), the second target-binding domain (e.g., any of the exemplary target-binding domains described herein or known in the art), or the soluble tissue factor domain (e.g., any of the exemplary soluble tissue factor domains described herein).

In some embodiments of any of the single-chain chimeric polypeptides described herein, the single-chain chimeric polypeptide includes one or more (e.g., two, three, four, five, six, seven, eight, nine, or ten) additional target-binding domains (e.g., any of the exemplary target-binding domains described herein or known in the art) at its C-terminus. In some embodiments, one of the one or more additional target-binding domains (e.g., any of the exemplary target-binding domains described herein or known in the art) at the C-terminus of the single-chain chimeric polypeptide directly abuts the first target-binding domain (e.g., any of the exemplary target-binding domains described herein or known in the art), the second target-binding domain (e.g., any of the exemplary target-binding domains described herein or known in the art), or the soluble tissue factor domain (e.g., any of the exemplary soluble tissue factor domains described herein or known in the art). In some embodiments, the single-chain chimeric polypeptide further comprises a linker sequence (e.g., any of the exemplary linker sequences described herein or known in the art) between one of the at least one additional target-binding domains (e.g., any of the exemplary target-binding domains described herein or known in the art) at the C-terminus of the single-chain chimeric polypeptide and the first target-binding domain (e.g., any of the exemplary target-binding domains described herein or known in the art), the second target-binding domain (e.g., any of the exemplary target-binding domains described herein or known in the art), or the soluble tissue factor domain (e.g., any of the exemplary soluble tissue factor domains described herein).

In some embodiments of any of the single-chain chimeric polypeptides described herein, the single-chain chimeric polypeptide comprises one or more (e.g., two, three, four, five, six, seven, eight, nine, or ten) additional target binding domains (e.g., any of the exemplary target-binding domains described herein or known in the art) at its N-terminus and its C-terminus. In some embodiments, one of the one or more additional antigen binding domains (e.g., any of the exemplary target-binding domains described herein or known in the art) at the N-terminus of the single-chain chimeric polypeptide directly abuts the first target-binding domain (e.g., any of the exemplary target-binding domains described herein or known in the art), the second target-binding domain (e.g., any of the exemplary target-binding domains described herein or known in the art), or the soluble tissue factor domain (e.g., any of the exemplary soluble tissue factor domains described herein). In some embodiments, the single-chain chimeric polypeptide further includes a linker sequence (e.g., any of the exemplary linker sequences described herein or known in the art) between one of the one or more additional antigen-binding domains (e.g., any of the exemplary target-binding domains described herein or known in the art) at the N-terminus and the first target-binding domain (e.g., any of the exemplary target-binding domains described herein or known in the art), the second target-binding domain (e.g., any of the exemplary target-binding domains described herein or known in the art), or the soluble tissue factor domain (e.g., any of the exemplary soluble tissue factor domains). In some embodiments, one of the one or more additional antigen binding domains (e.g., any of the exemplary target-binding domains described herein or known in the art) at the C-terminus directly abuts the first target-binding domain (e.g., any of the exemplary target-binding domains described herein or known in the art), the second target-binding domain (e.g., any of the exemplary target-binding domains described herein or known in the art), or the soluble tissue factor domain (e.g., any of the exemplary soluble tissue factor domains). In some embodiments, the single-chain chimeric polypeptide further includes a linker sequence (e.g., any of the exemplary linker sequences described herein or known in the art) between one of the one or more additional antigen-binding domains (e.g., any of the exemplary target-binding domains described herein or known in the art) at the C-terminus and the first target-binding domain(e.g., any of the exemplary target-binding domains described herein or known in the art), the second target-binding domain (e.g., any of the exemplary target-binding domains described herein or known in the art), or the soluble tissue factor domain (e.g., any of the exemplary soluble tissue factor domains described herein).

In some embodiments of any of the single-chain chimeric polypeptides described herein, two or more (e.g., three, four, five, six, seven, eight, nine, or ten) of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) bind specifically to the same antigen. In some embodiments, two or more (e.g., three, four, five, six, seven, eight, nine, or ten) of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) bind specifically to the same epitope. In some embodiments, two or more (e.g., three, four, five, six, seven, eight, nine, or ten) of the first target-binding domain (e.g., any of the exemplary target-binding domains described herein or known in the art), the second target-binding domain(e.g., any of the exemplary target-binding domains described herein or known in the art), and the one or more additional target-binding domains (e.g., any of the exemplary target-binding domains described herein or known in the art) include the same amino acid sequence.

In some embodiments of any of the single-chain chimeric polypeptides described herein, the first target-binding domain (e.g., any of the exemplary target-binding domains described herein or known in the art), the second target-binding domain (e.g., any of the exemplary target-binding domains described herein or known in the art), and the one or more (e.g., two, three, four, five, six, seven, eight, nine, or ten) additional target-binding domains (e.g., any of the exemplary target-binding domains described herein or known in the art) each bind specifically to the same antigen. In some embodiments, the first target-binding domain (e.g., any of the exemplary target-binding domains described herein or known in the art), the second target-binding domain(e.g., any of the exemplary target-binding domains described herein or known in the art), and the one or more (e.g., two, three, four, five, six, seven, eight, nine, or ten) additional target-binding domains (e.g., any of the exemplary target-binding domains described herein or known in the art) each bind specifically to the same epitope. In some embodiments, the first target-binding domain, the second target-binding domain, and the one or more (e.g., two, three, four, five, six, seven, eight, nine, or ten) additional target-binding domains each comprise the same amino acid sequence.

In some embodiments of any of the single-chain chimeric polypeptides described herein, the first target-binding domain (e.g., any of the exemplary target-binding domains described herein or known in the art), the second target-binding domain(e.g., any of the exemplary target-binding domains described herein or known in the art), and the one or more (e.g., two, three, four, five, six, seven, eight, nine, or ten) additional target-binding domains (e.g., any of the exemplary target-binding domains described herein or known in the art) bind specifically to different antigens.

In some embodiments of any of the single-chain chimeric polypeptides, one or more of the first target-binding domain, the second target-binding domain, and the one or more target-binding domains is an antigen-binding domain (e.g., any of the exemplary antigen-binding domains described herein or known in the art). In some embodiments of any of the single-chain chimeric polypeptides described herein, the first target-binding domain, the second target-binding domain, and the one or more additional target-binding domains are each an antigen-binding domain (e.g., any of the exemplary antigen-binding domains described herein or known in the art). In some embodiments, the antigen-binding domain can include a scFv or a single domain antibody.

In some embodiments of any of the single-chain chimeric polypeptides described herein, one or more (e.g., two, three, four, five, six, seven, eight, nine, or ten) of the first target-binding domain (e.g., any of the exemplary target-binding domains described herein or known in the art), the second target-binding domain(e.g., any of the exemplary target-binding domains described herein or known in the art), and the one or more additional target-binding domains (e.g., any of the exemplary target-binding domains described herein or known in the art) bind specifically to a target selected from the group consisting of: CD16a, CD28, CD3, CD33, CD20, CD19, CD22, CD123, IL-1R, IL-1, VEGF, IL-6R, IL-4, IL-10, PDL-1, TIGIT, PD-1, TIM3, CTLA4, MICA, MICB, IL-6, IL-8, TNFα, CD26, CD36, ULBP2, CD30, CD200, IGF-1R, MUC4AC, MUC5AC, Trop-2, CMET, EGFR, HER1, HER2, HER3, PSMA, CEA, B7H3, EPCAM, BCMA, P-cadherin, CEACAM5, a UL16-binding protein, HLA-DR, DLL4, TYRO3, AXL, MER, CD122, CD155, PDGF-DD, a ligand of TGF-β receptor II (TGF-βRII), a ligand of TGF-βRIII, a ligand of DNAM1, a ligand of NKp46, a ligand of NKp44, a ligand of NKG2D, a ligand of NKp30, a ligand for a scMHCI, a ligand for a scMHCII, a ligand for a scTCR, a receptor for IL-1, a receptor for IL-2, a receptor for IL-3, a receptor for IL-7, a receptor for IL-8, a receptor for IL-10, a receptor for IL-12, a receptor for IL-15, a receptor for IL-17, a receptor for IL-18, a receptor for IL-21, a receptor for PDGF-DD, a receptor for stem cell factor (SCF), a receptor for stem cell-like tyrosine kinase 3 ligand (FLT3L), a receptor for MICA, a receptor for MICB, a receptor for a ULP16-binding protein, a receptor for CD155, a receptor for CD122, and a receptor for CD28.

In some embodiments of any of the single-chain chimeric polypeptides described herein, one or more of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) is a soluble interleukin or cytokine protein. Non-limiting examples of soluble interleukin proteins and soluble cytokine proteins include: IL-1, IL-2, IL-3, IL-7, IL-8, IL-10, IL-12, IL-15, IL-17, IL-18, IL-21, PDGF-DD, and SCF.

In some embodiments of any of the single-chain chimeric polypeptides described herein, one or more of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) is a soluble interleukin or cytokine receptor. Non-limiting examples of soluble interleukin receptors and soluble cytokine receptors include: a soluble TGF-β receptor II (TGF-βRII), a soluble TGF-βRIII, a soluble NKG2D, a soluble NKP30, a soluble NKp44, a soluble NKp46, a soluble DNAM-1, a scMHCI, a scMHCII, a scTCR, a soluble CD155, a soluble CD122, a soluble CD3, or a soluble CD28.

In some embodiments of any of the single-chain chimeric polypeptides described herein, the first target-binding domain (e.g., any of the target-binding domains described herein), the second target-binding domain (e.g., any of the target-binding domains described herein), and the one or more additional target-binding domains (e.g., any of the target-binding domains described herein) can each, independently, bind specifically to a target selected from the group of: CD16a, CD33, CD20, CD19, CD22, CD123, PDL-1, TIGIT, PD-1, TIM3, CTLA4, MICA, MICB, IL-6, IL-8, TNFα, CD26, CD36, ULBP2, CD30, CD200, IGF-1R, MUC4AC, MUC5AC, Trop-2, CMET, EGFR, HER1, HER2, HER3, PSMA, CEA, B7H3, EPCAM, BCMA, P-cadherin, CEACAM5, a UL16-binding protein, HLA-DR, DLL4, TYRO3, AXL, MER, CD122, CD155, PDGF-DD, a ligand of TGF-β receptor II (TGF-βRII), a ligand of TGF-βRIII, a ligand of DNAM1, a ligand of NKp46, a ligand of NKp44, a ligand of NKG2D, a ligand of NKP30, a ligand for a scMHCI, a ligand for a scMHCII, a ligand for a scTCR, a receptor for PDGF-DD, a receptor for stem cell factor (SCF), a receptor for stem cell-like tyrosine kinase 3 ligand (FLT3L), a receptor for MICA, a receptor for MICB, a receptor for a ULP16-binding protein, a receptor for CD155, and a receptor for CD122.

In some embodiments of any of the single-chain chimeric polypeptides described herein, one or more of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) is a soluble interleukin or cytokine protein. In some embodiments of any of the multi-chain chimeric polypeptides described herein, the soluble interleukin or cytokine protein is selected from the group of: IL-1, IL-2, IL-3, IL-7, IL-8, IL-10, IL-12, IL-15, IL-17, IL-18, IL-21, PDGF-DD, and SCF.

In some embodiments of any of the single-chain chimeric polypeptides described herein, one or more of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) is a soluble interleukin or cytokine receptor. In some embodiments of any of the multi-chain chimeric polypeptides described herein, the soluble receptor is a soluble TGF-β receptor II (TGF-βRII) a soluble TGF-βRIII, a soluble receptor for TNFα, a soluble receptor for IL-4, or a soluble receptor for IL-10.

In some embodiments, a single-chain chimeric polypeptide has a nucleic acid sequence or an amino acid sequence that is at least 80% identical (e.g., at least 82% identical, at least 84% identical, at least 86% identical, at least 88% identical, at least 90% identical, at least 92% identical, at least 94% identical, at least 96% identical, at least 98% identical, at least 99% identical, or 100% identical) to SEQ ID NOs: 22-29.

### Soluble Tissue Factor Domain

In some embodiments of any of the polypeptides, compositions, or methods described herein, the soluble tissue factor domain can be a wildtype tissue factor polypeptide lacking the signal sequence, the transmembrane domain, and the intracellular domain. In some examples, the soluble tissue factor domain can be a tissue factor mutant, wherein a wildtype tissue factor polypeptide lacking the signal sequence, the transmembrane domain, and the intracellular domain, and has been further modified at selected amino acids. In some examples, the soluble tissue factor domain can be a soluble human tissue factor domain. In some examples, the soluble tissue factor domain can be a soluble mouse tissue factor domain. In some examples, the soluble tissue factor domain can be a soluble rat tissue factor domain. Non-limiting examples of soluble human tissue factor domains, a mouse soluble tissue factor domain, a rat soluble tissue factor domain, and mutant soluble tissue factor domains are shown below.
**Exemplary Soluble Human Tissue Factor Domain (SEQ ID NO: 10)** 
SGTTNTVAAYNLTWKSTNFKTILEWEPKPVNQVYTVQISTKSGDWKSKCFYTTD
TECDLTDEIVKDVKQTYLARVFSYPAGNVESTGSAGEPLYENSPEFTPYLETNLGQ
PTIQSFEQVGTKVNVTVEDERTLVRRNNTFLSLRDVFGKDLIYTLYYWKSSSSGK
KTAKTNTNEFLIDVDKGENYCFSVQAVIPSRTVNRKSTDSPVECMGQEKGEFRE
**Exemplary Nucleic Acid Encoding Soluble Human Tissue Factor Domain (SEQ ID NO: 11)** 
AGCGGCACAACCAACACAGTCGCTGCCTATAACCTCACTTGGAAGAGCACCA
ACTTCAAAACCATCCTCGAATGGGAACCCAAACCCGTTAACCAAGTTTACACC
GTGCAGATCAGCACCAAGTCCGGCGACTGGAAGTCCAAATGTTTCTATACCAC
CGACACCGAGTGCGATCTCACCGATGAGATCGTGAAAGATGTGAAACAGACC
TACCTCGCCCGGGTGTTTAGCTACCCCGCCGGCAATGTGGAGAGCACTGGTTC
 
CGCTGGCGAGCCTTTATACGAGAACAGCCCCGAATTTACCCCTTACCTCGAGA
CCAATTTAGGACAGCCCACCATCCAAAGCTTTGAGCAAGTTGGCACAAAGGT
GAATGTGACAGTGGAGGACGAGCGGACTTTAGTGCGGCGGAACAACACCTTT
CTCAGCCTCCGGGATGTGTTCGGCAAAGATTTAATCTACACACTGTATTACTGG
AAGTCCTCTTCCTCCGGCAAGAAGACAGCTAAAACCAACACAAACGAGTTTT
TAATCGACGTGGATAAAGGCGAAAACTACTGTTTCAGCGTGCAAGCTGTGATC
CCCTCCCGGACCGTGAATAGGAAAAGCACCGATAGCCCCGTTGAGTGCATGG
GCCAAGAAAAGGGCGAGTTCCGGGAG
**Exemplary Soluble Mouse Tissue Factor Domain (SEQ ID NO: 12)** 
agipekafnltwistdfktilewqpkptnytytvqisdrsrnwknkcfstt
dtecdltdeivkdvtwayeakvlsvprrnsvhgdgdqlvihgeeppftnap
kflpyrdtnlgqpviqqfeqdgrklnvvvkdsltlvrkngtfltlrqvfgk
dlgyiityrkgsstgkktnitntnefsidveegvsycffvqamifsrktnq
nspgsstvcteqwksflge
**Exemplary Soluble Rat Tissue Factor Domain (SEQ ID NO: 13)** 
agtppgkafnltwistdfktilewqpkptnytytvqisdrsrnwkykctgt
tdtecdltdeivkdvnwtyearvlsvpwrnsthgketlfgthgeeppftna
rkflpyrdtkigqpviqkyeqggtklkvtvkdsftlvrkngtfltlrqvfg
ndlgyiltyrkdsstgrktntthtneflidvekgvsycffaqavifsrktn
hkspesitkcteqwksvlge
**Exemplary Mutant Soluble Human Tissue Factor Domain (SEQ ID NO: 14)** 
SGTTNTVAAYNLTWKSTNFATALEWEPKPVNQVYTVQISTKSGDWKSKCFYTT
DTECALTDEIVKDVKQTYLARVFSYPAGNVESTGSAGEPLYENSPEFTPYLETNL
GQPTIQSFEQVGTKVNVTVEDERTLVARNNTALSLRDVFGKDLIYTLYYWKSSSS
GKKTAKTNTNEFLIDVDKGENYCFSVQAVIPSRTVNRKSTDSPVECMGQEKGEF
RE
**Exemplary Mutant Soluble Human Tissue Factor Domain (SEQ ID NO: 15)**

SGTTNTVAAYNLTWKSTNFATALEWEPKPVNQVYTVQISTKSGDAKSKCFYTTD
TECALTDEIVKDVKQTYLARVFSYPAGNVESTGSAGEPLAENSPEFTPYLETNLG

QPTIQSFEQVGTKVNVTVEDERTLVARNNTALSLRDVFGKDLIYTLYYWKSSSSG
KKTAKTNTNEFLIDVDKGENYCFSVQAVIPSRTVNRKSTDSPVECMGQEKGEFR
E

In some embodiments, a soluble tissue factor domain can include a sequence that is at least 70% identical, at least 72% identical, at least 74% identical, at least 76% identical, at least 78% identical, at least 80% identical, at least 82% identical, at least 84% identical, at least 86% identical, at least 88% identical, at least 90% identical, at least 92% identical, at least 94% identical, at least 96% identical, at least 98% identical, at least 99% identical, or 100% identical to SEQ ID NO: 10, 12, 13, 14, or 15. In some embodiments, a soluble tissue factor domain can include a sequence of SEQ ID NO: 10, 12, 13, 14, or 15, with one to twenty amino acids (e.g., 2, 3, 4, 5, 6, 7, 8, 9, 10, 11, 12, 13, 14, 15, 16, 17, 18, 19, or 20) amino acids removed from its N-terminus and/or one to twenty amino acids (e.g., 2, 3, 4, 5, 6, 7, 8, 9, 10, 11, 12, 13, 14, 15, 16, 17, 18, 19, or 20) amino acids removed from its C-terminus.

As can be appreciated in the art, one skilled in the art would understand that mutation of amino acids that are conserved between different mammalian species is more likely to decrease the activity and/or structural stability of the protein, while mutation of amino acids that are not conserved between different mammalian species is less likely to decrease the activity and/or structural stability of the protein.

In some examples of any of the multi-chain chimeric polypeptides described herein, the soluble tissue factor domain is not capable of binding to Factor VIIa. In some examples of any of the multi-chain chimeric polypeptides described herein, the soluble tissue factor domain does not convert inactive Factor X into Factor Xa. In some embodiments of any of the multi-chain chimeric polypeptides described herein, the multi-chain chimeric polypeptide does not stimulate blood coagulation in a mammal.

In some examples, the soluble tissue factor domain can be a soluble human tissue factor domain. In some embodiments, the soluble tissue factor domain can be a soluble mouse tissue factor domain. In some embodiments, the soluble tissue factor domain can be a soluble rat tissue factor domain.

In some examples, the soluble tissue factor domain does not include one or more (e.g., two, three, four, five, six, or seven) of: a lysine at an amino acid position that corresponds to amino acid position 20 of mature wildtype human tissue factor protein; an isoleucine at an amino acid position that corresponds to amino acid position 22 of mature wildtype human tissue factor protein; a tryptophan at an amino acid position that corresponds to amino acid position 45 of mature wildtype human tissue factor protein; an aspartic acid at an amino acid position that corresponds to amino acid position 58 of mature wildtype human tissue factor protein; a tyrosine at an amino acid position that corresponds to amino acid position 94 of mature wildtype human tissue factor protein; an arginine at an amino acid position that corresponds to amino acid position 135 of mature wildtype human tissue factor protein; and a phenylalanine at an amino acid position that corresponds to amino acid position 140 of mature wildtype human tissue factor protein. In some embodiments, the mutant soluble tissue factor possesses the amino acid sequence of SEQ ID NO: 14 or SEQ ID NO: 15.

In some examples, the soluble tissue factor domain can be encoded by a nucleic acid including a sequence that is at least 70% identical, at least 72% identical, at least 74% identical, at least 76% identical, at least 78% identical, at least 80% identical, at least 82% identical, at least 84% identical, at least 86% identical, at least 88% identical, at least 90% identical, at least 92% identical, at least 94% identical, at least 96% identical, at least 98% identical, at least 99% identical, or 100% identical to SEQ ID NO: 11.

In some embodiments, the soluble tissue factor domain can have a total length of about 20 amino acids to about 220 amino acids, about 20 amino acids to about 215 amino acids, about 20 amino acids to about 210 amino acids, about 20 amino acids to about 205 amino acids, about 20 amino acids to about 200 amino acids, about 20 amino acids to about 195 amino acids, about 20 amino acids to about 190 amino acids, about 20 amino acids to about 185 amino acids, about 20 amino acids to about 180 amino acids, about 20 amino acids to about 175 amino acids, about 20 amino acids to about 170 amino acids, about 20 amino acids to about 165 amino acids, about 20 amino acids to about 160 amino acids, about 20 amino acids to about 155 amino acids, about 20 amino acids to about 150 amino acids, about 20 amino acids to about 145 amino acids, about 20 amino acids to about 140 amino acids, about 20 amino acids to about 135 amino acids, about 20 amino acids to about 130 amino acids, about 20 amino acids to about 125 amino acids, about 20 amino acids to about 120 amino acids, about 20 amino acids to about 115 amino acids, about 20 amino acids to about 110 amino acids, about 20 amino acids to about 105 amino acids, about 20 amino acids to about 100 amino acids, about 20 amino acids to about 95 amino acids, about 20 amino acids to about 90 amino acids, about 20 amino acids to about 85 amino acids, about 20 amino acids to about 80 amino acids, about 20 amino acids to about 75 amino acids, about 20 amino acids to about 70 amino acids, about 20 amino acids to about 60 amino acids, about 20 amino acids to about 50 amino acids, about 20 amino acids to about 40 amino acids, about 20 amino acids to about 30 amino acids.

### Linker Sequences

In some embodiments, the linker sequence can be a flexible linker sequence. Non-limiting examples of linker sequences that can be used are described in Klein et al., Protein Engineering, Design & Selection 27(10):325-330, 2014; Priyanka et al., Protein Sci. 22(2):153-167, 2013. In some examples, the linker sequence is a synthetic linker sequence.

In some embodiments of any of the single-chain chimeric polypeptides described herein can include one, two, three, four, five, six, seven, eight, nine, or ten linker sequence(s) (e.g., the same or different linker sequences, e.g., any of the exemplary linker sequences described herein or known in the art). In some embodiments of any of the single-chain chimeric polypeptides described herein can include one, two, three, four, five, six, seven, eight, nine, or ten linker sequence(s) (e.g., the same or different linker sequences, e.g., any of the exemplary linker sequences described herein or known in the art).

In some embodiments of any of the multi-chain chimeric polypeptides described herein, the first chimeric polypeptide can include one, two, three, four, five, six, seven, eight, nine, or ten linker sequence(s) (e.g., the same or different linker sequences, e.g., any of the exemplary linker sequences described herein or known in the art). In some embodiments of any of the multi-chain chimeric polypeptides described herein, the second chimeric polypeptide can include one, two, three, four, five, six, seven, eight, nine, or ten linker sequence(s) (e.g., the same or different linker sequences, e.g., any of the exemplary linker sequences described herein or known in the art).

In some embodiments, a linker sequence can have a total length of 1 amino acid to about 100 amino acids, 1 amino acid to about 90 amino acids, 1 amino acid to about 80 amino acids, 1 amino acid to about 70 amino acids, 1 amino acid to about 60 amino acids, 1 amino acid to about 50 amino acids, 1 amino acid to about 45 amino acids, 1 amino acid to about 40 amino acids, 1 amino acid to about 35 amino acids, 1 amino acid to about 30 amino acids, 1 amino acid to about 25 amino acids, 1 amino acid to about 24 amino acids, 1 amino acid to about 22 amino acids, 1 amino acid to about 20 amino acids, 1 amino acid to about 18 amino acids, 1 amino acid to about 16 amino acids, 1 amino acid to about 14 amino acids, 1 amino acid to about 12 amino acids, 1 amino acid to about 10 amino acids, 1 amino acid to about 8 amino acids, 1 amino acid to about 6 amino acids, 1 amino acid to about 4 amino acids.

In some embodiments, the linker is rich in glycine (Gly or G) residues. In some embodiments, the linker is rich in serine (Ser or S) residues. In some embodiments, the linker is rich in glycine and serine residues. In some embodiments, the linker has one or more glycine-serine residue pairs (GS), e.g., 1, 2, 3, 4, 5, 6, 7, 8, 9, or 10 or more GS pairs. In some embodiments, the linker has one or more Gly-Gly-Gly-Ser (GGGS) (SEQ ID NO: 16) sequences, e.g., 1, 2, 3, 4, 5, 6, 7, 8, 9, or 10 or more GGGS (SEQ ID NO: 16) sequences. In some embodiments, the linker has one or more Gly-Gly-Gly-Gly-Ser (GGGGS) (SEQ ID NO: 17) sequences, e.g., 1, 2, 3, 4, 5, 6, 7, 8, 9, or 10 or more GGGGS (SEQ ID NO: 17) sequences. In some embodiments, the linker has one or more Gly-Gly-Ser-Gly (GGSG) (SEQ ID NO: 18) sequences, e.g., 1, 2, 3, 4, 5, 6, 7, 8, 9, or 10 or more GGSG (SEQ ID NO: 18) sequences.

In some embodiments, the linker sequence can comprise or consist of GGGGSGGGGSGGGGS (SEQ ID NO: 19). In some embodiments, the linker sequence can be encoded by a nucleic acid comprising or consisting of: GGCGGTGGAGGATCCGGAGGAGGTGGCTCCGGCGGCGGAGGATCT (SEQ ID NO: 20). In some embodiments, the linker sequence can comprise or consist of: GGGSGGGS (SEQ ID NO: 21).

### Target-Binding Domains

In some embodiments of any of the single- or multi-chain chimeric polypeptides described herein, the first target-binding domain, the second target-binding domain, and/or the additional one or more target-binding domains can be an antigen-binding domain (e.g., any of the exemplary antigen-binding domains described herein or known in the art), a soluble interleukin or cytokine protein (e.g., any of the exemplary soluble interleukin proteins or soluble cytokine proteins described herein), and a soluble interleukin or cytokine receptor (e.g., any of the exemplary soluble interleukin receptors or soluble cytokine receptors described herein).

In some embodiments of any of the single- or multi-chain chimeric polypeptides described herein, the first target-binding domain, the second target-binding domain, and/or the one or more additional target-binding domains can each independent have a total number of amino acids of about 5 amino acids to about 1000 amino acids, about 5 amino acids to about 950 amino acids, about 5 amino acids to about 900 amino acids, about 5 amino acids to about 850 amino acids, about 5 amino acids to about 800 amino acids, about 5 amino acids to about 750 amino acids, about 5 amino acids to about 700 amino acids, about 5 amino acids to about 650 amino acids, about 5 amino acids to about 600 amino acids, about 5 amino acids to about 550 amino acids, about 5 amino acids to about 500 amino acids, about 5 amino acids to about 450 amino acids, about 5 amino acids to about 400 amino acids, about 5 amino acids to about 350 amino acids, about 5 amino acids to about 300 amino acids, about 5 amino acids to about 280 amino acids, about 5 amino acids to about 260 amino acids, about 5 amino acids to about 240 amino acids, about 5 amino acids to about 220 amino acids, about 5 amino acids to about 200 amino acids, about 5 amino acids to about 195 amino acids, about 5 amino acids to about 190 amino acids, about 5 amino acids to about 185 amino acids, about 5 amino acids to about 180 amino acids, about 5 amino acids to about 175 amino acids, about 5 amino acids to about 170 amino acids, about 5 amino acids to about 165 amino acids, about 5 amino acids to about 160 amino acids, about 5 amino acids to about 155 amino acids, about 5 amino acids to about 150 amino acids, about 5 amino acids to about 145 amino acids, about 5 amino acids to about 140 amino acids, about 5 amino acids to about 135 amino acids, about 5 amino acids to about 130 amino acids, about 5 amino acids to about 125 amino acids, about 5 amino acids to about 120 amino acids, about 5 amino acids to about 115 amino acids, about 5 amino acids to about 110 amino acids, about 5 amino acids to about 105 amino acids, about 5 amino acids to about 100 amino acids, about 5 amino acids to about 95 amino acids, about 5 amino acids to about 90 amino acids, about 5 amino acids to about 85 amino acids, about 5 amino acids to about 80 amino acids, about 5 amino acids to about 75 amino acids, about 5 amino acids to about 70 amino acids, about 5 amino acids to about 65 amino acids, about 5 amino acids to about 60 amino acids, about 5 amino acids to about 55 amino acids, about 5 amino acids to about 50 amino acids, about 5 amino acids to about 45 amino acids, about 5 amino acids to about 40 amino acids, about 5 amino acids to about 35 amino acids, about 5 amino acids to about 30 amino acids, about 5 amino acids to about 25 amino acids, about 5 amino acids to about 20 amino acids, about 5 amino acids to about 15 amino acids, about 5 amino acids to about 10 amino acids,

Any of the target-binding domains described herein can bind to its target with a dissociation equilibrium constant (K_{D}) of less than 1 x 10⁻⁷M, less than 1 x 10⁻⁸ M, less than 1 x 10⁻⁹ M, less than 1 x 10⁻¹⁰ M, less than 1 x 10⁻¹¹ M, less than 1 x 10⁻¹² M, or less than 1 x 10⁻¹³ M. In some embodiments, the antigen-binding protein construct provided herein can bind to an identifying antigen with a K_{D} of about 1 x 10⁻³ M to about 1 x 10⁻⁵ M, about 1 x 10⁻⁴ M to about 1 x 10⁻⁶ M, about 1 x 10⁻⁵ M to about 1 x 10⁻⁷ M, about 1 x 10⁻⁶ M to about 1 x 10⁻⁸ M, about 1 x 10⁻⁷ M to about 1 x 10⁻⁹ M, about 1 x 10⁻⁸ M to about 1 x 10⁻¹⁰ M, or about 1 x 10⁻⁹ M to about 1 x 10⁻¹¹ M (inclusive).

Any of the target-binding domains described herein can bind to its target with a K_{D} of between about 1 pM to about 30 nM (e.g., about 1 pM to about 25 nM, about 1 pM to about 20 nM, about 1 pM to about 15 nM, about 1 pM to about 10 nM, about 1 pM to about 5 nM, about 1 pM to about 2 nM, about 1 pM to about 1 nM, about 1 pM to about 950 pM, about 1 pM to about 900 pM, about 1 pM to about 850 pM, about 1 pM to about 800 pM, about 1 pM to about 750 pM, about 1 pM to about 700 pM, about 1 pM to about 650 pM, about 1 pM to about 600 pM, about 1 pM to about 550 pM, about 1 pM to about 500 pM, about 1 pM to about 450 pM, about 1 pM to about 400 pM, about 1 pM to about 350 pM, about 1 pM to about 300 pM, about 1 pM to about 250 pM, about 1 pM to about 200 pM, about 1 pM to about 150 pM, about 1 pM to about 100 pM, about 1 pM to about 90 pM, about 1 pM to about 80 pM, about 1 pM to about 70 pM, about 1 pM to about 60 pM, about 1 pM to about 50 pM, about 1 pM to about 40 pM, about 1 pM to about 30 pM, about 1 pM to about 20 pM, about 1 pM to about 10 pM, about 1 pM to about 5 pM, about 1 pM to about 4 pM, about 1 pM to about 3 pM, about 1 pM to about 2 pM).

Any of the target-binding domains described herein can bind to its target with a K_{D} of between about 1 nM to about 10 nM (e.g., about 1 nM to about 9 nM, about 1 nM to about 8 nM, about 1 nM to about 7 nM, about 1 nM to about 6 nM, about 1 nM to about 5 nM, about 1 nM to about 4 nM, about 1 nM to about 3 nM, about 1 nM to about 2 nM, about 2 nM to about 10 nM, about 2 nM to about 9 nM, about 2 nM to about 8 nM, about 2 nM to about 7 nM, about 2 nM to about 6 nM, about 2 nM to about 5 nM, about 2 nM to about 4 nM, about 2 nM to about 3 nM, about 3 nM to about 10 nM, about 3 nM to about 9 nM, about 3 nM to about 8 nM, about 3 nM to about 7 nM, about 3 nM to about 6 nM, about 3 nM to about 5 nM, about 3 nM to about 4 nM, about 4 nM to about 10 nM, about 4 nM to about 9 nM, about 4 nM to about 8 nM, about 4 nM to about 7 nM, about 4 nM to about 6 nM, about 4 nM to about 5 nM, about 5 nM to about 10 nM, about 5 nM to about 9 nM, about 5 nM to about 8 nM, about 5 nM to about 7 nM, about 5 nM to about 6 nM, about 6 nM to about 10 nM, about 6 nM to about 9 nM, about 6 nM to about 8 nM, about 6 nM to about 7 nM, about 7 nM to about 10 nM, about 7 nM to about 9 nM, about 7 nM to about 8 nM, about 8 nM to about 10 nM, about 8 nM to about 9 nM, and about 9 nM to about 10 nM).

A variety of different methods known in the art can be used to determine the K_{D} values of any of the antigen-binding protein constructs described herein (e.g., an electrophoretic mobility shift assay, a filter binding assay, surface plasmon resonance, and a biomolecular binding kinetics assay, etc.).

### Antigen-Binding Domains

In some embodiments of any of the single-chain or multi-chain chimeric polypeptides described herein, the first target-binding domain and the second target-binding domain bind specifically to the same antigen. In some embodiments of these single-chain or multi-chain chimeric polypeptides, the first target-binding domain and the second target-binding domain bind specifically to the same epitope. In some embodiments of these single-chain or multi-chain chimeric polypeptides, the first target-binding domain and the second target-binding domain include the same amino acid sequence.

In some embodiments of any of the single-chain or multi-chain chimeric polypeptides described herein, the first target-binding domain and the second target-binding domain bind specifically to different antigens.

In some embodiments of any of the single-chain or multi-chain chimeric polypeptides described herein, one or both of the first target-binding domain and the second target-binding domain is an antigen-binding domain. In some embodiments of any of the single-chain or multi-chain chimeric polypeptides described herein, the first target-binding domain and the second target-binding domain are each antigen-binding domains. In some embodiments of any of the single-chain or multi-chain chimeric polypeptides described herein, the antigen-binding domain includes or is a scFv or a single domain antibody (e.g., a VHH or a VNAR domain).

In some examples, an antigen-binding domain (e.g., any of the antigen-binding domains described herein) can bind specifically to any one of CD16a (see, e.g., those described in U.S. Patent No. 9,035,026), CD28 (see, e.g., those described in U.S. Patent No. 7,723,482), CD3 (see, e.g., those described in U.S. Patent No. 9,226,962), CD33 (see, e.g., those described in U.S. Patent No. 8,759,494), CD20 (see, e.g., those described in WO 2014/026054), CD19 (see, e.g., those described in U.S. Patent No. 9,701,758), CD22 (see, e.g., those described in WO 2003/104425), CD123 (see, e.g., those described in WO 2014/130635), IL-1R (see, e.g., those described in U.S. Patent No. 8,741,604), IL-1 (see, e.g., those described in WO 2014/095808), VEGF (see, e.g., those described in U.S. Patent No. 9,090,684), IL-6R (see, e.g., those described in U.S. Patent No. 7,482,436), IL-4 (see, e.g., those described in U.S. Patent Application Publication No. 2012/0171197), IL-10 (see, e.g., those described in U.S. Patent Application Publication No. 2016/0340413), PDL-1 (see, e.g., those described in Drees et al., Protein Express. Purif. 94:60-66, 2014), TIGIT (see, e.g., those described in U.S. Patent Application Publication No. 2017/0198042), PD-1 (see, e.g., those described in U.S. Patent No. 7,488,802), TIM3 (see, e.g., those described in U.S. Patent No. 8,552,156), CTLA4 (see, e.g., those described in WO 2012/120125), MICA (see, e.g., those described in WO 2016/154585), MICB (see, e.g., those described in U.S. Patent No. 8,753,640), IL-6 (see, e.g., those described in Gejima et al., Human Antibodies 11(4):121-129, 2002), IL-8 (see, e.g., those described in U.S. Patent No. 6,117,980), TNFα (see, e.g., those described in Geng et al., Immunol. Res. 62(3):377-385, 2015), CD26 (see, e.g., those described in WO 2017/189526), CD36 (see, e.g., those described in U.S. Patent Application Publication No. 2015/0259429), ULBP2 (see, e.g., those described in U.S. Patent No. 9,273,136), CD30 (see, e.g., those described in Homach et al., Scand. J. Immunol. 48(5):497-501, 1998), CD200 (see, e.g., those described in U.S. Patent No. 9,085,623), IGF-1R (see, e.g., those described in U.S. Patent Application Publication No. 2017/0051063), MUC4AC (see, e.g., those described in WO 2012/170470), MUC5AC (see, e.g., those described in U.S. Patent No. 9,238,084), Trop-2 (see, e.g., those described in WO 2013/068946), CMET (see, e.g., those described in Edwardraja et al., Biotechnol. Bioeng. 106(3):367-375, 2010), EGFR (see, e.g., those described in Akbari et al., Protein Expr. Purif. 127:8-15, 2016), HER1 (see, e.g., those described in U.S. Patent Application Publication No. 2013/0274446), HER2 (see, e.g., those described in Cao et al., Biotechnol. Lett. 37(7):1347-1354, 2015), HER3 (see, e.g., those described in U.S. Patent No. 9,505,843), PSMA (see, e.g., those described in Parker et al., Protein Expr. Purif. 89(2):136-145, 2013), CEA (see, e.g., those described in WO 1995/015341), B7H3 (see, e.g., those described in U.S. Patent No. 9,371,395), EPCAM (see, e.g., those described in WO 2014/159531), BCMA (see, e.g., those described in Smith et al., Mol. Ther. 26(6):1447-1456, 2018), P-cadherin (see, e.g., those described in U.S. Patent No. 7,452,537), CEACAM5 (see, e.g., those described in U.S. Patent No. 9,617,345), a UL16-binding protein (see, e.g., those described in WO 2017/083612), HLA-DR (see, e.g., Pistillo et al., Exp. Clin. Immunogenet. 14(2):123-130, 1997), DLL4 (see, e.g., those described in WO 2014/007513), TYRO3 (see, e.g., those described in WO 2016/166348), AXL (see, e.g., those described in WO 2012/175692), MER (see, e.g., those described in WO 2016/106221), CD122 (see, e.g., those described in U.S. Patent Application Publication No. 2016/0367664), CD155 (see, e.g., those described in WO 2017/149538), or PDGF-DD (see, e.g., those described in U.S. Patent No. 9,441,034).

The antigen-binding domains present in any of the single-chain or multi-chain chimeric polypeptides described herein are each independently selected from the group consisting of: a VHH domain, a VNAR domain, and a scFv. In some embodiments, any of the antigen-binding domains described herein is a BiTe, a (scFv)₂, a nanobody, a nanobody-HSA, a DART, a TandAb, a scDiabody, a scDiabody-CH3, scFv-CH-CL-scFv, a HSAbody, scDiabody-HAS, or a tandem-scFv. Additional examples of antigen-binding domains that can be used in any of the single-chain or multi-chain chimeric polypeptide are known in the art.

A VHH domain is a single monomeric variable antibody domain that can be found in camelids. A VNAR domain is a single monomeric variable antibody domain that can be found in cartilaginous fish. Non-limiting aspects of V_{H}H domains and V_{NAR} domains are described in, e.g., Cromie et al., Curr. Top. Med. Chem. 15:2543-2557, 2016; De Genst et al., Dev. Comp. Immunol. 30:187-198, 2006; De Meyer et al., Trends Biotechnol. 32:263-270, 2014; Kijanka et al., Nanomedicine 10:161-174, 2015; Kovaleva et al., Expert. Opin. Biol. Ther. 14:1527-1539, 2014; Krah et al., Immunopharmacol. Immunotoxicol. 38:21-28, 2016; Mujic-Delic et al., Trends Pharmacol. Sci. 35:247-255, 2014; Muyldermans, J. Biotechnol. 74:277-302, 2001; Muyldermans et al., Trends Biochem. Sci. 26:230-235, 2001; Muyldermans, Ann. Rev. Biochem. 82:775-797, 2013; Rahbarizadeh et al., Immunol. Invest. 40:299-338, 2011; Van Audenhove et al., EBioMedicine 8:40-48, 2016; Van Bockstaele et al., Curr. Opin. Investig. Drugs 10:1212-1224, 2009; Vincke et al., Methods Mol. Biol. 911:15-26, 2012; and Wesolowski et al., Med. Microbiol. Immunol. 198:157-174, 2009.

In some embodiments, each of the antigen-binding domains in the single-chain or multi-chain chimeric polypeptides described herein are both VHH domains, or at least one antigen-binding domain is a VHH domain. In some embodiments, each of the antigen-binding domains in the single-chain or multi-chain chimeric polypeptides described herein are both VNAR domains, or at least one antigen-binding domain is a VNAR domain. In some embodiments, each of the antigen-binding domains in the single-chain or multi-chain chimeric polypeptides described herein are both scFv domains, or at least one antigen-binding domain is a scFv domain.

In some embodiments, two or more of polypeptides present in the single-chain or multi-chain chimeric polypeptide can assemble (e.g., non-covalently assemble) to form any of the antigen-binding domains described herein, e.g., an antigen-binding fragment of an antibody (e.g., any of the antigen-binding fragments of an antibody described herein), a VHH-scAb, a VHH-Fab, a Dual scFab, a F(ab')2, a diabody, a crossMab, a DAF (two-in-one), a DAF (four-in-one), a DutaMab, a DT-IgG, a knobs-in-holes common light chain, a knobs-in-holes assembly, a charge pair, a Fab-arm exchange, a SEEDbody, a LUZ-Y, a Fcab, a κλ-body, an orthogonal Fab, a DVD-IgG, a IgG(H)-scFv, a scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG, IgG-2scFv, scFv4-Ig, Zybody, DVI-IgG, Diabody-CH3, a triple body, a miniantibody, a minibody, a TriBi minibody, scFv-CH3 KIH, Fab-scFv, a F(ab')₂-scFv₂, a scFv-KIH, a Fab-scFv-Fc, a tetravalent HCAb, a scDiabody-Fc, a Diabody-Fc, a tandem scFv-Fc, an Intrabody, a dock and lock, a lmmTAC, an IgG-IgG conjugate, a Cov-X-Body, and a scFv1-PEG-scFv2. See, e.g., Spiess et al., Mol. Immunol. 67:95-106, 2015, for a description of these elements. Non-limiting examples of an antigen-binding fragment of an antibody include an Fv fragment, a Fab fragment, a F(ab')₂ fragment, and a Fab' fragment. Additional examples of an antigen-binding fragment of an antibody is an antigen-binding fragment of an IgG (e.g., an antigen-binding fragment of IgG1, IgG2, IgG3, or IgG4) (e.g., an antigen-binding fragment of a human or humanized IgG, e.g., human or humanized IgG1, IgG2, IgG3, or IgG4); an antigen-binding fragment of an IgA (e.g., an antigen-binding fragment of IgA1 or IgA2) (e.g., an antigen-binding fragment of a human or humanized IgA, e.g., a human or humanized IgA1 or IgA2); an antigen-binding fragment of an IgD (e.g., an antigen-binding fragment of a human or humanized IgD); an antigen-binding fragment of an IgE (e.g., an antigen-binding fragment of a human or humanized IgE); or an antigen-binding fragment of an IgM (e.g., an antigen-binding fragment of a human or humanized IgM).

An "Fv" fragment includes a non-covalently-linked dimer of one heavy chain variable domain and one light chain variable domain.

A "Fab" fragment includes, the constant domain of the light chain and the first constant domain (C_{H1}) of the heavy chain, in addition to the heavy and light chain variable domains of the Fv fragment.

A "F(ab')₂" fragment includes two Fab fragments joined, near the hinge region, by disulfide bonds.

A "dual variable domain immunoglobulin" or "DVD-Ig" refers to multivalent and multispecific binding proteins as described, e.g., in DiGiammarino et al., Methods Mol. Biol. 899:145-156, 2012; Jakob et al., MABs 5:358-363, 2013; and U.S. Patent Nos. 7,612,181; 8,258,268; 8,586,714; 8,716,450; 8,722,855; 8,735,546; and 8,822,645.

DARTs are described in, e.g., Garber, Nature Reviews Drug Discovery 13:799-801, 2014.

In some embodiments of any of the antigen-binding domains described herein can bind to an antigen selected from the group consisting of: a protein, a carbohydrate, a lipid, and a combination thereof.

Additional examples and aspects of antigen-binding domains are known in the art.

### Soluble Interleukin or Cytokine Protein

In some embodiments of any of the single-chain or multi-chain chimeric polypeptides described herein, one or both of the first target-binding domain and the second target-binding domain can be a soluble interleukin protein or soluble cytokine protein. In some embodiments, the soluble interleukin or soluble cytokine protein is selected from the group of: IL-2, IL-3, IL-7, IL-8, IL-10, IL-12, IL-15, IL-17, IL-18, IL-21, PDGF-DD, SCF, and FLT3L. Non-limiting examples of soluble IL-2, IL-3, IL-7, IL-8, IL-10, IL-15, IL-17, IL-18, IL-21, PDGF-DD, SCF, and FLT3L are provided below.
**Human Soluble IL-2 (SEQ ID NO: 104)**
**Human Soluble IL-3 (SEQ ID NO: 105)** 
apmtqttplkt swvncsnmid eiithlkqpp lplldfnnln gedqdilmen
nlrrpnleaf nravkslqna saiesilknl lpclplataa ptrhpihikd
gdwnefrrkl tfylktlena qaqqttlsla if
**Human Soluble IL-7 (SEQ ID NO: 106)** 
dcdiegkdgkqyesv lmvsidqlld smkeigsncl nnefnffkrh icdankegmf
lfraarklrq flkmnstgdf dlhllkvseg ttillnctgq vkgrkpaalg
eaqptkslee nkslkeqkkl ndlcflkrll qeiktcwnki lmgtkeh
**Human Soluble IL-8 (SEQ ID NO: 107)** 
egavlprsak elrcqcikty skpfhpkfik elrviesgph canteiivkl
sdgrelcldp kenwvqrvve kflkraens
**Human Soluble IL-10 (SEQ ID NO: 108)** 
spgqgtqsensc thfpgnlpnm lrdlrdafsr vktffqmkdq ldnlllkesl
ledfkgylgc qalsemiqfy leevmpqaen qdpdikahvn slgenlktlr
lrlrrchrfl pcenkskave qvknafnklq ekgiykamse fdifinyiea
ymtmkirn
**Human Soluble IL-15 (SEQ ID NO: 109)** 
Nwvnvisdlkki edliqsmhid atlytesdvh psckvtamkc fllelqvisl
esgdasihdt venliilann slssngnvte sgckeceele eknikeflqs
fvhivqmfin ts
**Human Soluble IL-17 (SEQ ID NO: 110)** 
gitiprn pgcpnsedkn fprtvmvnln ihnrntntnp krssdyynrs
tspwnlhrne dperypsviw eakcrhlgci nadgnvdyhm nsvpiqqeil
vlrrepphcp nsfrlekilv svgctcvtpi vhhva
**Human Soluble IL-18 (SEQ ID NO: 111)** 
yfgklesklsvirn lndqvlfidq gnrplfedmt dsdcrdnapr tifiismykd
sqprgmavti svkcekistl scenkiisfk emnppdnikd tksdiiffqr
svpghdnkmq fesssyegyf lacekerdlf klilkkedel gdrsimftvq ned
**Human Soluble PDGF-DD (SEQ ID NO: 112)** 
rdtsatpqsasi kalrnanlrr desnhltdly rrdetiqvkg ngyvqsprfp
nsyprnlllt wrlhsqentr iqlvfdnqfg leeaendicr ydfvevedis
etstiirgrw cghkevppri ksrtnqikit fksddyfvak pgfkiyysll
edfqpaaase tnwesvtssi sgvsynspsv tdptliadal dkkiaefdtv
edllkyfnpe swqedlenmy ldtpryrgrs yhdrkskvdl drlnddakry
sctprnysvn ireelklanv vffprcllvq rcggncgcgt vnwrsctcns
gktvkkyhev lqfepghikr rgraktmalv diqldhherc dcicssrppr
**Human Soluble SCF (SEQ ID NO: 113)** 
egicrnrvtnnvkdv tklvanlpkd ymitlkyvpg mdvlpshcwi semvvqlsds
ltdlldkfsn iseglsnysi idklvnivdd lvecvkenss kdlkksfksp
eprlftpeef frifnrsida fkdfvvaset sdcvvsstls pekdsrvsvt
kpfmlppvaa sslrndssss nrkaknppgd sslhwaamal palfsliigf
afgalywkkr qpsltraven iqineednei smlqekeref qev
**Human Soluble FLT3L (SEQ ID NO: 114)**

tqdcsfqhspissd favkirelsd yllqdypvtv asnlqdeelc gglwrlvlaq
rwmerlktva gskmqgller vnteihfvtk cafqpppscl rfvqtnisrl
lqetseqlva lkpwitrqnf srclelqcqp dsstlpppws prpleatapt

apqpplllll llpvglllla aawclhwqrt rrrtprpgeq vppvpspqdl
llveh

Non-limiting examples of soluble MICA, MICB, ULBP1, ULBP2, ULBP3, ULBP4, ULBP5, and ULBP6 are provided below.
**Human Soluble MICA (SEQ ID NO: 115)** 
ephslry nltvlswdgs vqsgfltevh ldgqpflrcd rqkcrakpqg
qwaedvlgnk twdretrdlt gngkdlrmtl ahikdqkegl hslqeirvce
ihednstrss qhfyydgelf lsqnletkew tmpqssraqt lamnvrnflk
edamktkthy hamhadclqe lrrylksgvv lrrtvppmvn vtrseasegn
itvtcrasgf ypwnitlswr qdgvslshdt qqwgdvlpdg ngtyqtwvat
ricqgeeqrf tcymehsgnh sthpvpsgkv lvlqshwqtf hvsavaaaai
fviiifyvrc ckkktsaaeg pelvslqvld qhpvgtsdhr datqlgfqpl
msdlgstgst ega
**Human Soluble MICB (SEQ ID NO: 116)** 
aephslry nlmvlsqdes vqsgflaegh ldgqpflryd rqkrrakpqg
qwaedvlgak twdtetedlt engqdlrrtl thikdqkggl hslqeirvce
ihedsstrgs rhfyydgelf lsqnletqes tvpqssraqt lamnvtnfwk
edamktkthy ramqadclqk lqrylksgva irrtvppmvn vtcsevsegn
itvtcrassf yprnitltwr qdgvslshnt qqwgdvlpdg ngtyqtwvat
rirqgeeqrf tcymehsgnh gthpvpsgkv lvlqsqrtdf pyvsaampcf
viiiilcvpc ckkktsaaeg pelvslqvld qhpvgtgdhr
daaqlgfqpl msatgstgst ega
**Human Soluble ULBP1 (SEQ ID NO: 117)** 
wvdthclcydfiit pksrpepqwc evqglvderp flhydcvnhk akafaslgkk
vnvtktweeq tetlrdvvdf lkgqlldiqv enlipieplt lqarmscehe
ahghgrgswq flfngqkfll fdsnnrkwta lhpgakkmte kweknrdvtm
 ffqkislgdc kmwleeflmy weqmldptkp pslapg
**Human Soluble ULBP2 (SEQ ID NO: 118)** 
gradphslcyditvi pkfrpgprwc avqgqvdekt flhydcgnkt vtpvsplgkk
lnvttawkaq npvlrevvdi lteqlrdiql enytpkeplt lqarmsceqk
aeghssgswq fsfdgqifll fdsekrmwtt vhpgarkmke kwendkvvam
sfhyfsmgdc igwledflmg mdstlepsag aplams
**Human Soluble ULBP3 (SEQ ID NO: 119)** 
dahslwynfti ihlprhgqqw cevqsqvdqk nflsydcgsd kvlsmghlee
qlyatdawgk qlemlrevgq rlrleladte ledftpsgpl tlqvrmscec
eadgyirgsw qfsfdgrkfl lfdsnnrkwt vvhagarrmk ekwekdsglt
tffkmvsmrd ckswlrdflm hrkkrlepta pptmapg
**Human Soluble ULBP4 (SEQ ID NO: 120)** 
hslcfnftik slsrpgqpwc eaqvflnknl flqynsdnnm vkplgllgkk
vyatstwgel tqtlgevgrd lrmllcdikp qiktsdpstl qvemfcqrea
erctgaswqf atngeksllf damnmtwtvi nheaskiket wkkdrgleky
frklskgdcd hwlreflghw eampeptvsp vnasdihwss sslpdrwiil
gafillvlmg ivlicvwwqn gewqaglwpl rts
**Human Soluble ULBP5 (SEQ ID NO: 121)** 
gladp hslcyditvi pkfrpgprwc avqgqvdekt flhydcgskt
vtpvsplgkk lnvttawkaq npvlrevvdi lteqlldiql enyipkeplt
lqarmsceqk aeghgsgswq lsfdgqifll fdsenrmwtt vhpgarkmke
kwendkdmtm sfhyismgdc tgwledflmg mdstlepsag apptmssg
**Human Soluble ULBP6 (SEQ ID NO: 122)**

rrddp hslcyditvi pkfrpgprwc avqgqvdekt flhydcgnkt
vtpvsplgkk lnvtmawkaq npvlrevvdi lteqlldiql enytpkeplt

lqarmsceqk aeghssgswq fsidgqtfll fdsekrmwtt vhpgarkmke
kwendkdvam sfhyismgdc igwledflmg mdstlepsag aplamssg

Additional examples of soluble interleukin proteins and soluble cytokine proteins are known in the art.

### Soluble Receptor

In some embodiments of any of the single-chain or multi-chain chimeric polypeptides described herein, one or both of the first target-binding domain and the second target-binding domain is a soluble interleukin receptor or a soluble cytokine receptor. In some embodiments, the soluble receptor is a soluble TGF-β receptor II (TGF-β RII) (see, e.g., those described in Yung et al., Am. J. Resp. Crit. Care Med. 194(9):1140-1151, 2016), a soluble TGF-βRIII (see, e.g., those described in Heng et al., Placenta 57:320, 2017), a soluble NKG2D (see, e.g., Cosman et al., Immunity 14(2):123-133, 2001; Costa et al., Front. Immunol., Vol. 9, Article 1150, May 29, 2018; doi: 10.3389/fimmu.2018.01150), a soluble NKp30 (see, e.g., Costa et al., Front. Immunol., Vol. 9, Article 1150, May 29, 2018; doi: 10.3389/fimmu.2018.01150), a soluble NKp44 (see, e.g., those described in Costa et al., Front. Immunol., Vol. 9, Article 1150, May 29, 2018; doi: 10.3389/fimmu.2018.01150), a soluble NKp46 (see, e.g., Mandelboim et al., Nature 409:1055-1060, 2001; Costa et al., Front. Immunol., Vol. 9, Article 1150, May 29, 2018; doi: 10.3389/fimmu.2018.01150), a soluble DNAM1 (see, e.g., those described in Costa et al., Front. Immunol., Vol. 9, Article 1150, May 29, 2018; doi: 10.3389/fimmu.2018.01150), a scMHCI (see, e.g., those described in Washburn et al., PLoS One 6(3):e18439, 2011), a scMHCII (see, e.g., those described in Bishwajit et al., Cellular Immunol. 170(1):25-33, 1996), a scTCR (see, e.g., those described in Weber et al., Nature 356(6372):793-796, 1992), a soluble CD155 (see, e.g., those described in Tahara-Hanaoka et al., Int. Immunol. 16(4):533-538, 2004), or a soluble CD28 (see, e.g., Hebbar et al., Clin. Exp. Immunol. 136:388-392, 2004). Additional examples of soluble interleukin receptors and soluble cytokine receptors are known in the art.

### Exemplary Embodiments of Single-Chain Chimeric Polypeptides- Type A

In some embodiments of any of the single-chain chimeric polypeptides described herein, the first target-binding domain and/or the second target-binding domain can independently bind specifically to CD3 (e.g., human CD3) or CD28 (e.g., human CD28). In some embodiments, the first target-binding domain binds specifically to CD3 (e.g., human CD3) and the second target-binding domain binds specifically to CD28 (e.g., human CD28). In some embodiments, the first target-binding domain binds specifically to CD28 (e.g., human CD28) and the second target-binding domain binds specifically to CD3 (e.g., human CD3).

### Exemplary Embodiments of Single-Chain Chimeric Polypeptides- Type B

In some embodiments of any of the single-chain chimeric polypeptides described herein, the first target-binding domain and/or the second target-binding domain can independently bind specifically to an IL-2 receptor (e.g., human IL-2 receptor).

### Exemplary Embodiments of Single-Chain Chimeric Polypeptides- Type C

In some embodiments of any of the single-chain chimeric polypeptides described herein, the first target-binding domain and/or the second target-binding domain can independently bind specifically to an IL-15 receptor (e.g., a human IL-15 receptor).

### Sequences of Exemplary Single-Chain Chimeric Polypeptides

The nucleic acid encoding a αCD3scFv/TF/αCD28scFv single-chain polypeptide is as follows (SEQ ID NO: 22):
*(Signal peptide)* 
ATGAAGTGGGTGACCTTCATCAGCTTATTATTTTTATTCAGCTCCGCCT
ATTCC
*(αCD3 light chain variable region)* 
CAGATCGTGCTGACCCAAAGCCCCGCCATCATGAGCGCTAGCCCCGGT
GAGAAGGTGACCATGACATGCTCCGCTTCCAGCTCCGTGTCCTACATGAACT
 
GGTATCAGCAGAAAAGCGGAACCAGCCCCAAAAGGTGGATCTACGACACCA
GCAAGCTGGCCTCCGGAGTGCCCGCTCATTTCCGGGGCTCTGGATCCGGCAC
CAGCTACTCTTTAACCATTTCCGGCATGGAAGCTGAAGACGCTGCCACCTACT
ATTGCCAGCAATGGAGCAGCAACCCCTTCACATTCGGATCTGGCACCAAGCT
CGAAATCAATCGT
*(Linker)*
   GGAGGAGGTGGCAGCGGCGGCGGTGGATCCGGCGGAGGAGGAAGC
*(αCD3 heavy chain variable region)* 
CAAGTTCAACTCCAGCAGAGCGGCGCTGAACTGGCCCGGCCCGGCGC
CTCCGTCAAGATGAGCTGCAAGGCTTCCGGCTATACATTTACTCGTTACACAA
TGCATTGGGTCAAGCAGAGGCCCGGTCAAGGTTTAGAGTGGATCGGATATAT
CAACCCTTCCCGGGGCTACACCAACTATAACCAAAAGTTCAAGGATAAAGCC
ACTTTAACCACTGACAAGAGCTCCTCCACCGCCTACATGCAGCTGTCCTCTTT
AACCAGCGAGGACTCCGCTGTTTACTACTGCGCTAGGTATTACGACGACCAC
TACTGTTTAGACTATTGGGGACAAGGTACCACTTTAACCGTCAGCAGC
*(Human tissue factor 219 form)* 
TCCGGCACCACCAATACCGTGGCCGCTTATAACCTCACATGGAAGAGC
ACCAACTTCAAGACAATTCTGGAATGGGAACCCAAGCCCGTCAATCAAGTTT
ACACCGTGCAGATCTCCACCAAATCCGGAGACTGGAAGAGCAAGTGCTTCTA
CACAACAGACACCGAGTGTGATTTAACCGACGAAATCGTCAAGGACGTCAAG
CAAACCTATCTGGCTCGGGTCTTTTCCTACCCCGCTGGCAATGTCGAGTCCAC
CGGCTCCGCTGGCGAGCCTCTCTACGAGAATTCCCCCGAATTCACCCCTTATT
TAGAGACCAATTTAGGCCAGCCTACCATCCAGAGCTTCGAGCAAGTTGGCAC
CAAGGTGAACGTCACCGTCGAGGATGAAAGGACTTTAGTGCGGCGGAATAAC
ACATTTTTATCCCTCCGGGATGTGTTCGGCAAAGACCTCATCTACACACTGTA
CTATTGGAAGTCCAGCTCCTCCGGCAAAAAGACCGCTAAGACCAACACCAAC
GAGTTTTTAATTGACGTGGACAAAGGCGAGAACTACTGCTTCAGCGTGCAAG
CCGTGATCCCTTCTCGTACCGTCAACCGGAAGAGCACAGATTCCCCCGTTGA
GTGCATGGGCCAAGAAAAGGGCGAGTTCCGGGAG
*(αCD28 light chain variable region)* 
GTCCAGCTGCAGCAGAGCGGACCCGAACTCGTGAAACCCGGTGCTTCC
GTGAAAATGTCTTGTAAGGCCAGCGGATACACCTTCACCTCCTATGTGATCCA
GTGGGTCAAACAGAAGCCCGGACAAGGTCTCGAGTGGATCGGCAGCATCAA
CCCTTACAACGACTATACCAAATACAACGAGAAGTTTAAGGGAAAGGCTACT
TTAACCTCCGACAAAAGCTCCATCACAGCCTACATGGAGTTCAGCTCTTTAAC
ATCCGAGGACAGCGCTCTGTACTATTGCGCCCGGTGGGGCGACGGCAATTAC
TGGGGACGGGGCACAACACTGACCGTGAGCAGC
*(Linker)*
   GGAGGCGGAGGCTCCGGCGGAGGCGGATCTGGCGGTGGCGGCTCC
*(αCD28 light chain variable region)*

GACATCGAGATGACCCAGTCCCCCGCTATCATGTCCGCCTCTTTAGGCGAGC
GGGTCACAATGACTTGTACAGCCTCCTCCAGCGTCTCCTCCTCCTACTTCCAT
TGGTACCAACAGAAACCCGGAAGCTCCCCTAAACTGTGCATCTACAGCACCA
GCAATCTCGCCAGCGGCGTGCCCCCTAGGTTTTCCGGAAGCGGAAGCACCAG
CTACTCTTTAACCATCTCCTCCATGGAGGCTGAGGATGCCGCCACCTACTTTT
GTCACCAGTACCACCGGTCCCCCACCTTCGGAGGCGGCACCAAACTGGAGAC
AAAGAGG

The amino acid sequence of a αCD3scFv/TF/αCD28scFv single-chain chimeric polypeptide is as follows (SEQ ID NO: 23):
*(Signal peptide)*
   MKWVTFISLLFLFSSAYS
*(αCD3 light chain variable region)* 
QIVLTQSPAIMSASPGEKVTMTCSASSSVSYMNWYQQKSGTSPKRWIYDTSKLA
SGVPAHFRGSGSGTSYSLTISGMEAEDAATYYCQQWSSNPFTFGSGTKLEINR
*(Linker)*
   GGGGSGGGGSGGGGS
*(αCD3 heavy chain variable region)*
*(Human tissue factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(αCD28 light chain variable region)* 
VQLQQSGPELVKPGASVKMSCKASGYTFTSYVIQWVKQKPGQGLEWIGSINPYN
DYTKYNEKFKGKATLTSDKSSITAYMEFSSLTSEDSALYYCARWGDGNYWGRG
TTLTVSS
*(Linker)*
   GGGGSGGGGSGGGGS
*(αCD28 heavy chain variable region)*

DIEMTQSPAIMSASLGERVTMTCTASSSVSSSYFHWYQQKPGSSPKLCIYSTSNLA
SGVPPRFSGSGSTSYSLTISSMEAEDAATYFCHQYHRSPTFGGGTKLETKR

The nucleic acid sequence encoding a αCD28scFv/TF/αCD3scFv single-chain polypeptide is as follows (SEQ ID NO: 24):
*(Signal peptide)* 
ATGAAATGGGTCACCTTCATCTCTTTACTGTTTTTATTTAGCAGCGCCT
ACAGC
*(αCD28 light chain variable region)* 
GTGCAGCTGCAGCAGTCCGGACCCGAACTGGTCAAGCCCGGTGCCTCC
GTGAAAATGTCTTGTAAGGCTTCTGGCTACACCTTTACCTCCTACGTCATCCA
ATGGGTGAAGCAGAAGCCCGGTCAAGGTCTCGAGTGGATCGGCAGCATCAAT
CCCTACAACGATTACACCAAGTATAACGAAAAGTTTAAGGGCAAGGCCACTC
TGACAAGCGACAAGAGCTCCATTACCGCCTACATGGAGTTTTCCTCTTTAACT
 
TCTGAGGACTCCGCTTTATACTATTGCGCTCGTTGGGGCGATGGCAATTATTG
GGGCCGGGGAACTACTTTAACAGTGAGCTCC
*(Linker)*
   GGCGGCGGCGGAAGCGGAGGTGGAGGATCTGGCGGTGGAGGCAGC
*(αCD28 heavy chain variable region)* 
GACATCGAGATGACACAGTCCCCGCTATCATGAGCGCCTCTTTAGGA
GAACGTGTGACCATGACTTGTACAGCTTCCTCCAGCGTGAGCAGCTCCTATTT
CCACTGGTACCAGCAGAAACCCGGCTCCTCCCCTAAACTGTGTATCTACTCCA
CAAGCAATTTAGCTAGCGGCGTGCCTCCTCGTTTTAGCGGCTCCGGCAGCACC
TCTTACTCTTTAACCATTAGCTCTATGGAGGCCGAAGATGCCGCCACATACTT
TTGCCATCAGTACCACCGGTCCCCTACCTTTGGCGGAGGCACAAAGCTGGAG
ACCAAGCGG
*(Human tissue factor 219 form)* 
AGCGGCACCACCAACACAGTGGCCGCCTACAATCTGACTTGGAAATCC
ACCAACTTCAAGACCATCCTCGAGTGGGAGCCCAAGCCCGTTAATCAAGTTT
ATACCGTGCAGATTTCCACCAAGAGCGGCGACTGGAAATCCAAGTGCTTCTA
TACCACAGACACCGAGTGCGATCTCACCGACGAGATCGTCAAAGACGTGAAG
CAGACATATTTAGCTAGGGTGTTCTCCTACCCCGCTGGAAACGTGGAGAGCA
CCGGATCCGCTGGAGAGCCTTTATACGAGAACTCCCCCGAATTCACCCCCTAT
CTGGAAACCAATTTAGGCCAGCCCACCATCCAGAGCTTCGAACAAGTTGGCA
CAAAGGTGAACGTCACCGTCGAAGATGAGAGGACTTTAGTGCGGAGGAACA
ATACATTTTTATCCTTACGTGACGTCTTCGGCAAGGATTTAATCTACACACTG
TATTACTGGAAGTCTAGCTCCTCCGGCAAGAAGACCGCCAAGACCAATACCA
ACGAATTTTTAATTGACGTGGACAAGGGCGAGAACTACTGCTTCTCCGTGCA
AGCTGTGATCCCCTCCCGGACAGTGAACCGGAAGTCCACCGACTCCCCCGTG
GAGTGCATGGGCCAAGAGAAGGGAGAGTTTCGTGAG
*(αCD3 light chain variable region)* 
CAGATCGTGCTGACCCAGTCCCCCGCTATTATGAGCGCTAGCCCCGGT
GAAAAGGTGACTATGACATGCAGCGCCAGCTCTTCCGTGAGCTACATGAACT
GGTATCAGCAGAAGTCCGGCACCAGCCCTAAAAGGTGGATCTACGACACCAG
 
CAAGCTGGCCAGCGGCGTCCCCGCTCACTTTCGGGGCTCCGGCTCCGGAACA
AGCTACTCTCTGACCATCAGCGGCATGGAAGCCGAGGATGCCGCTACCTATT
ACTGTCAGCAGTGGAGCTCCAACCCCTTCACCTTTGGATCCGGCACCAAGCTC
GAGATTAATCGT
*(Linker)*
   GGAGGCGGAGGTAGCGGAGGAGGCGGATCCGGCGGTGGAGGTAGC
*(αCD3 heavy chain variable region)*

CAAGTTCAGCTCCAGCAAAGCGGCGCCGAACTCGCTCGGCCCGGCGCT
TCCGTGAAGATGTCTTGTAAGGCCTCCGGCTATACCTTCACCCGGTACACAAT
GCACTGGGTCAAGCAACGGCCCGGTCAAGGTTTAGAGTGGATTGGCTATATC
AACCCCTCCCGGGGCTATACCAACTACAACCAGAAGTTCAAGGACAAAGCCA
CCCTCACCACCGACAAGTCCAGCAGCACCGCTTACATGCAGCTGAGCTCTTT
AACATCCGAGGATTCCGCCGTGTACTACTGCGCTCGGTACTACGACGATCATT
ACTGCCTCGATTACTGGGGCCAAGGTACCACCTTAACAGTCTCCTCC

The amino acid sequence of a αCD28scFv/TF/αCD3scFv single-chain chimeric polypeptide is as follows (SEQ ID NO: 25):
*(Signal peptide)*
   MKWVTFISLLFLFSSAYS
*(αCD28 light chain variable region)* 
VQLQQSGPELVKPGASVKMSCKASGYTFTSYVIQWVKQKPGQGLEWIGS
INPYNDYTKYNEKFKGKATLTSDKSSITAYMEFSSLTSEDSALYYCARWGDGNY
WGRGTTLTVSS
*(Linker)*
   GGGGSGGGGSGGGGS
*(αCD28 heavy chain variable region)* 
DIEMTQSPAIMSASLGERVTMTCTASSSVSSSYFHWYQQKPGSSPKLCIYS
TSNLASGVPPRFSGSGSTSYSLTISSMEAEDAATYFCHQYHRSPTFGGGTKLETKR
*(Human tissue factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(αCD3 light chain variable region)* 
QIVLTQSPAIMSASPGEKVTMTCSASSSVSYMNWYQQKSGTSPKRWIYDT
SKLASGVPAHFRGSGSGTSYSLTISGMEAEDAATYYCQQWSSNPFTFGSGTKLEI
NR
*(Linker)*
   GGGGSGGGGSGGGGS
*(αCD3 heavy chain variable region)*

The nucleic acid sequence encoding an IL-2/TF/IL-2 single-chain chimeric polypeptide is as follows (SEQ ID NO: 26):
*(Signal peptide)* 
ATGAAGTGGGTGACCTTCATCAGCCTGCTGTTCCTGTTCTCCAGCGCCT
ACTCC
*(First IL-2 fragment)* 
GCCCCCACCTCCTCCTCCACCAAGAAGACCCAGCTGCAGCTGGAGCAT
TTACTGCTGGATTTACAGATGATTTTAAACGGCATCAACAACTACAAGAACC
CCAAGCTGACTCGTATGCTGACCTTCAAGTTCTACATGCCCAAGAAGGCCAC
CGAGCTGAAGCATTTACAGTGTTTAGAGGAGGAGCTGAAGCCCCTCGAGGAG
GTGCTGAATTTAGCCCAGTCCAAGAATTTCCATTTAAGGCCCCGGGATTTAAT
CAGCAACATCAACGTGATCGTTTTAGAGCTGAAGGGCTCCGAGACCACCTTC
ATGTGCGAGTACGCCGACGAGACCGCCACCATCGTGGAGTTTTTAAATCGTT
GGATCACCTTCTGCCAGTCCATCATCTCCACTTTAACC
*(Human tissue factor 219 form)* 
AGCGGCACAACCAACACAGTCGCTGCCTATAACCTCACTTGGAAGAG
CACCAACTTCAAAACCATCCTCGAATGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Second IL-2 fragment)*

GCACCTACTTCAAGTTCTACAAAGAAAACACAGCTACAACTGGAGCAT
TTACTGCTGGATTTACAGATGATTTTGAATGGAATTAATAATTACAAGAATCC
CAAACTCACCAGGATGCTCACATTTAAGTTTTACATGCCCAAGAAGGCCACA
GAACTGAAACATCTTCAGTGTCTAGAAGAAGAACTCAAACCTCTGGAGGAAG
TGCTAAATTTAGCTCAAAGCAAAAACTTTCACTTAAGACCCAGGGACTTAAT
CAGCAATATCAACGTAATAGTTCTGGAACTAAAGGGATCTGAAACAACATTC
ATGTGTGAATATGCTGATGAGACAGCAACCATTGTAGAATTTCTGAACAGAT
GGATTACCTTTTGTCAAAGCATCATCTCAACACTAACT

The amino acid sequence of an IL-2/TF/IL-2 single-chain chimeric polypeptide is as follows (SEQ ID NO: 27):
*(Signal peptide)*
   MKWVTFISLLFLFSSAYS
*(Human IL-2)*
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-2)*

The nucleic acid sequence encoding an IL-15/TF/IL-15 single-chain chimeric polypeptide is as follows (SEQ ID NO: 28):
*(Signal peptide)*
   ATGAAGTGGGTGACCTTCATCAGCCTGCTGTTCCTGTTCTCCAGCGCCT ACTCC
*(First IL-15 fragment)* 
AACTGGGTGAACGTGATCAGCGATTTAAAGAAGATCGAGGATTTAATC
CAGAGCATGCACATCGACGCCACTCTGTACACTGAGAGCGACGTGCACCCTA
GCTGCAAGGTGACTGCCATGAAGTGCTTTTTACTGGAGCTGCAAGTTATCTCT
TTAGAGAGCGGCGATGCCAGCATCCACGACACTGTGGAGAATTTAATCATTT
TAGCCAACAACTCTTTAAGCAGCAACGGCAACGTGACAGAGAGCGGCTGCA
AGGAGTGCGAGGAGCTGGAGGAGAAGAACATCAAGGAGTTTTTACAGAGCT
TCGTGCACATCGTGCAGATGTTCATCAACACTAGC
*(Human tissue factor 219 form)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
 
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Second IL-15 fragment)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of an IL-15/TF/IL-15 single-chain chimeric polypeptide is as follows (SEQ ID NO: 29):
*(Signal peptide)*
   MKWVTFISLLFLFSSAYS
*(Human IL-15)* 
NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

### Multi-Chain Chimeric Polypeptides

Non-limiting examples of tissue factor-containing proteins are multi-chain chimeric polypeptides that include: (a) a first chimeric polypeptide including: (i) a first target-binding domain; (ii) a soluble tissue factor domain; and (iii) a first domain of a pair of affinity domains; and (b) a second chimeric polypeptide including: (i) a second domain of a pair of affinity domains; and (ii) a second target-binding domain, where the first chimeric polypeptide and the second chimeric polypeptide associate through the binding of the first domain and the second domain of the pair of affinity domains.

In some embodiments of any of the multi-chain chimeric polypeptides described herein, the first target-binding domain (e.g., any of the first target-binding domains described herein) and the soluble tissue factor domain (e.g., any of the exemplary soluble tissue factor domains described herein) directly abut each other in the first chimeric polypeptide. In some embodiments of any of the multi-chain chimeric polypeptides described herein, the first chimeric polypeptide further comprises a linker sequence (e.g., any of the exemplary linker sequences described herein or known in the art) between the first target-binding domain (e.g., any of the exemplary first target-binding domains described herein) and the soluble tissue factor domain (e.g., any of the exemplary soluble tissue factor domains described herein) in the first chimeric polypeptide.

In some embodiments of any of the multi-chain chimeric polypeptides described herein, the soluble tissue factor domain (e.g., any of the exemplary soluble tissue factor domains described herein) and the first domain of the pair of affinity domains (e.g., any of the exemplary first domains of any of the exemplary pairs of affinity domains described herein) directly abut each other in the first chimeric polypeptide. In some embodiments of any of the multi-chain chimeric polypeptides described herein, the first chimeric polypeptide further comprises a linker sequence (e.g., any of the exemplary linker sequences described herein or known in the art) between the soluble tissue factor domain (e.g., any of the exemplary soluble tissue factor domains described herein) and the first domain of the pair of affinity domains (e.g., any of the exemplary first domains of any of the exemplary pairs of affinity domains described herein) in the first chimeric polypeptide.

In some embodiments of any of the multi-chain chimeric polypeptides described herein, the second domain of the pair of affinity domains (e.g., any of the exemplary second domains of any of the exemplary pairs of affinity domains described herein) and the second target-binding domain (e.g., any of the exemplary second target-binding domains described herein) directly abut each other in the second chimeric polypeptide. In some embodiments of any of the multi-chain chimeric polypeptides described herein, the second chimeric polypeptide further comprises a linker sequence (e.g., any of the exemplary linker sequences described herein or known in the art) between the second domain of the pair of affinity domains (e.g., any of the exemplary second domains of any of the exemplary pairs of affinity domains described herein) and the second target-binding domain (e.g., any of the exemplary second target-binding domains described herein) in the second chimeric polypeptide.

In some embodiments of any of the multi-chain chimeric polypeptides, the first chimeric polypeptide further includes one or more (e.g., two, three, four, five, six, seven, eight, nine, or ten) additional target-binding domain(s) (e.g., any of the exemplary target-binding domains described herein or known in the art), where at least one of the one or more additional antigen-binding domain(s) is positioned between the soluble tissue factor domain (e.g., any of the exemplary soluble tissue factor domains described herein or known in the art) and the first domain of the pair of affinity domains (e.g., any of the exemplary first domains of any of the exemplary pairs of affinity domains described herein). In some embodiments, the first chimeric polypeptide can further include a linker sequence (e.g., any of the exemplary linker sequences described herein or known in the art) between the soluble tissue factor domain (e.g., any of the exemplary soluble tissue factor domains described herein) and the at least one of the one or more additional target-binding domain(s) (e.g., any of the exemplary target-binding domains described herein or known in the art), and/or a linker sequence (e.g., any of the exemplary linker sequences described herein or known in the art) between the at least one of the one or more additional target-binding domain(s) (e.g., any of the exemplary target-binding domains described herein or known in the art) and the first domain of the pair of affinity domains (e.g., any of the exemplary first domains described herein of any of the exemplary pairs of affinity domains described herein).

In some embodiments of any of the multi-chain chimeric polypeptides described herein, the first chimeric polypeptide further includes one or more (e.g., two, three, four, five, six, seven, eight, nine, or ten) additional target-binding domains at the N-terminal and/or C-terminal end of the first chimeric polypeptide. In some embodiments, at least one of the one or more additional target-binding domains (e.g., any of the exemplary target-binding domains described herein or known in the art) directly abuts the first domain of the pair of affinity domains (e.g., any of the exemplary first domains described herein of any of the exemplary pairs of affinity domains described herein) in the first chimeric polypeptide. In some embodiments, the first chimeric polypeptide further includes a linker sequence (e.g., any of the exemplary linker sequences described herein or known in the art) between the at least one of the one or more additional target-binding domains (e.g., any of the exemplary target-binding domains described herein or known in the art) and the first domain of the pair of affinity domains (e.g., any of the exemplary first domains described herein of any of the exemplary pairs of affinity domains described herein). In some embodiments, the at least one of the one or more additional target-binding domains (e.g., any of the exemplary target-binding domains described herein or known in the art) directly abuts the first target-binding domain (e.g., any of the exemplary target-binding domains described herein or known in the art) in the first chimeric polypeptide. In some embodiments, the first chimeric polypeptide further comprises a linker sequence (e.g., any of the exemplary linker sequences described herein or known in the art) between the at least one of the one or more additional target-binding domains (e.g., any of the exemplary target-binding domains described herein or known in the art) and the first target-binding domain (e.g., any of the exemplary target-binding domains described herein or known in the art).

In some embodiments of any of the multi-chain chimeric polypeptides described herein, at least one of the one or more additional target-binding domains (e.g., any of the exemplary target-binding domains described herein or known in the art) is disposed at the N- and/or C-terminus of the first chimeric polypeptide, and at least one of the one or more additional target-binding domains (e.g., any of the exemplary target-binding domains described herein or known in the art) is positioned between the soluble tissue factor domain (e.g., any of the exemplary soluble tissue factor domains described herein or known in the art) and the first domain of the pair of affinity domains (e.g., any of the exemplary first domains of any of the exemplary pairs of affinity domains described herein) in the first chimeric polypeptide. In some embodiments, the at least one additional target-binding domain (e.g., any of the exemplary target-binding domains described herein or known in the art) of the one or more additional target-binding domains disposed at the N-terminus directly abuts the first target-binding domain (e.g., any of the exemplary target-binding domains described herein or known in the art) or the first domain of the pair of affinity domains (e.g., any of the exemplary first domains described herein of any of the exemplary pairs of affinity domains described herein) in the first chimeric polypeptide. In some embodiments, the first chimeric polypeptide further comprises a linker sequence (e.g., any of the linker sequences described herein or known in the art) disposed between the at least one additional target-binding domain (e.g., any of the exemplary target-binding domains described herein or known in the art) and the first target-binding domain (e.g., any of the exemplary target-binding domains described herein or known in the art) or the first domain of the pair of affinity domains (e.g., any of the exemplary first domains described herein of any of the exemplary pairs of affinity domains described herein) in the first chimeric polypeptide. In some embodiments, the at least one additional target-binding domain (e.g., any of the exemplary target-binding domains described herein or known in the art) of the one or more additional target-binding domains disposed at the C-terminus directly abuts the first target-binding domain (e.g., any of the exemplary target-binding domains described herein or known in the art) or the first domain of the pair of affinity domains (e.g., any of the exemplary first domains of any of the exemplary pairs of affinity domains described herein) in the first chimeric polypeptide. In some embodiments, the first chimeric polypeptide further includes a linker sequence (e.g., any of the exemplary linker sequences described herein or known in the art) disposed between the at least one additional target-binding domain (e.g., any of the exemplary target-binding domains described herein or known in the art) and the first target-binding domain (e.g., any of the exemplary target-binding domains described herein or known in the art) or the first domain of the pair of affinity domains (e.g., any of the exemplary first domains described herein of any of the exemplary pairs of affinity domains described herein) in the first chimeric polypeptide. In some embodiments, the at least one of the one or more additional target-binding domains (e.g., any of the exemplary target-binding domains described herein or known in the art) positioned between the soluble tissue factor domain (e.g., any of the exemplary soluble tissue factor domains described herein) and the first domain of the pair of affinity domains (e.g., any of the first domains described herein or any of the exemplary pairs of affinity domains described herein), directly abuts the soluble tissue factor domain and/or the first domain of the pair of affinity domains. In some embodiments, the first chimeric polypeptide further comprises a linker sequence (e.g., any of the exemplary linker sequences described herein or known in the art) disposed (i) between the soluble tissue factor domain (e.g., any of the exemplary soluble tissue factor domains described herein) and the at least one of the one or more additional target-binding domains (e.g., any of the exemplary target-binding domains described herein or known in the art) positioned between the soluble tissue factor domain (e.g., any of the exemplary soluble tissue factor domains described herein) and the first domain of the pair of affinity domains (e.g., any of the exemplary first domains of any of the exemplary pairs of affinity domains described herein), and/or (ii) between the first domain of the pair of affinity domains and the at least one of the one or more additional target-binding domains positioned between the soluble tissue factor domain and the first domain of the pair of affinity domains.

In some embodiments of any of the multi-chain chimeric polypeptides described herein, the second chimeric polypeptide further includes one or more (e.g., two, three, four, five, six, seven, eight, nine, or ten) additional target-binding domains (e.g., any of the exemplary target-binding domains described herein or known in the art) at the N-terminal end and/or the C-terminal end of the second chimeric polypeptide. In some embodiments, at least one of the one or more additional target-binding domains (e.g., any of the exemplary target-binding domains described herein or known in the art) directly abuts the second domain of the pair of affinity domains (e.g., any of the exemplary second domains of any of the exemplary pairs of affinity domains described herein) in the second chimeric polypeptide. In some embodiments, the second chimeric polypeptide further includes a linker sequence (e.g., any of the exemplary linker sequences described herein or known in the art) between at least one of the one or more additional target-binding domains (e.g., any of the exemplary target-binding domains described herein or known in the art) and the second domain of the pair of affinity domains (e.g., any of the second domains described herein of any of the exemplary pairs of affinity domains described herein) in the second chimeric polypeptide. In some embodiments, at least one of the one or more additional target-binding domains (e.g., any of the exemplary target-binding domains described herein or known in the art) directly abuts the second target-binding domain (e.g., any of the target-binding domains described herein or known in the art) in the second chimeric polypeptide. In some embodiments, the second chimeric polypeptide further includes a linker sequence (e.g., any of the exemplary linker sequences described herein or known in the art) between at least one of the one or more additional target-binding domains (e.g., any of the exemplary target binding domains described herein or known in the art) and the second target-binding domain (e.g., any of the exemplary target binding domains described herein or known in the art) in the second chimeric polypeptide.

In some embodiments of any of the multi-chain chimeric polypeptides described herein, two or more (e.g., three or more, four or more, five or more, six or more, seven or more, eight or more, nine or more, or ten or more) of the first target-binding domain, the second target-binding domain, and the one or more additional target-binding domains bind specifically to the same antigen. In some embodiments, two or more (e.g., three or more, four or more, five or more, six or more, seven or more, eight or more, nine or more, or ten or more) of the first target-binding domain, the second target-binding domain, and the one or more additional target-binding domains bind specifically to the same epitope. In some embodiments, two or more (e.g., three or more, four or more, five or more, six or more, seven or more, eight or more, nine or more, or ten or more) of the first target-binding domain, the second target-binding domain, and the one or more additional target-binding domains include the same amino acid sequence. In some embodiments, the first target-binding domain, the second target-binding domain, and the one or more additional target-binding domains each bind specifically to the same antigen. In some embodiments, the first target-binding domain, the second target-binding domain, and the one or more additional target-binding domains each bind specifically to the same epitope. In some embodiments, the first target-binding domain, the second target-binding domain, and the one or more additional target-binding domains each include the same amino acid sequence.

In some embodiments of any of the multi-chain chimeric polypeptides described herein, the first target-binding domain, the second target-binding domain, and the one or more additional target-binding domains bind specifically to different antigens. In some embodiments of any of the multi-chain chimeric polypeptides described herein, one or more (e.g., two or more, three or more, four or more, five or more, six or more, seven or more, eight or more, nine or more, or ten or more) of the first target-binding domain, the second target-binding domain, and the one or more additional target-binding domains is an antigen-binding domain. In some embodiments, the first target-binding domain, the second target-binding domain, and the one or more additional target-binding domains are each an antigen-binding domain (e.g., a scFv or a single-domain antibody).

In some embodiments of any of the multi-chain chimeric polypeptides described herein, one or more (e.g., two, three, four, five, six, seven, eight, nine, or ten) of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) bind specifically to a target selected from the group consisting of: CD16a, CD28, CD3, CD33, CD20, CD19, CD22, CD123, IL-1R, IL-1, VEGF, IL-6R, IL-4, IL-10, PDL-1, TIGIT, PD-1, TIM3, CTLA4, MICA, MICB, IL-6, IL-8, TNFα, CD26, CD36, ULBP2, CD30, CD200, IGF-1R, MUC4AC, MUC5AC, Trop-2, CMET, EGFR, HER1, HER2, HER3, PSMA, CEA, B7H3, EPCAM, BCMA, P-cadherin, CEACAM5, a UL16-binding protein, HLA-DR, DLL4, TYRO3, AXL, MER, CD122, CD155, PDGF-DD, a ligand of TGF-β receptor II (TGF-βRII), a ligand of TGF-βRIII, a ligand of DNAM1, a ligand of NKp46, a ligand of NKp44, a ligand of NKG2D, a ligand of NKP30, a ligand for a scMHCI, a ligand for a scMHCII, a ligand for a scTCR, a receptor for IL-1, a receptor for IL-2, a receptor for IL-3, a receptor for IL-7, a receptor for IL-8, a receptor for IL-10, a receptor for IL-12, a receptor for IL-15, a receptor for IL-17, a receptor for IL-18, a receptor for IL-21, a receptor for PDGF-DD, a receptor for stem cell factor (SCF), a receptor for stem cell-like tyrosine kinase 3 ligand (FLT3L), a receptor for MICA, a receptor for MICB, a receptor for a ULP16-binding protein, a receptor for CD155, a receptor for CD122, and a receptor for CD28.

In some embodiments of any of the multi-chain chimeric polypeptides described herein, one or more of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) is a soluble interleukin or cytokine protein. Non-limiting examples of soluble interleukin proteins and soluble cytokine proteins include: IL-1, IL-2, IL-3, IL-7, IL-8, IL-10, IL-12, IL-15, IL-17, IL-18, IL-21, PDGF-DD, and SCF.

In some embodiments of any of the multi-chain chimeric polypeptides described herein, one or more of the first target-binding domain (e.g., any of the exemplary target-binding domains described herein or known in the art), the second target-binding domain (e.g., any of the exemplary target-binding domains described herein or known in the art), and the one or more additional target-binding domains (e.g., any of the exemplary target-binding domains described herein or known in the art) is a soluble interleukin or cytokine receptor. Non-limiting examples of soluble interleukin receptors and soluble cytokine receptors include: a soluble TGF-β receptor II (TGF-βRII), a soluble TGF-βRIII, a soluble NKG2D, a soluble NKP30, a soluble NKp44, a soluble NKp46, a soluble DNAM1, a scMHCI, a scMHCII, a scTCR, a soluble CD155, a soluble CD122, a soluble CD3, or a soluble CD28.

In some embodiments of any of the multi-chain chimeric polypeptides described herein, the first target-binding domain (e.g., any of the target-binding domains described herein, the second target-binding domain (e.g., any of the target-binding domains described herein), and the one or more additional target-binding domains (e.g., any of the target-binding domains described herein) can each, independently, bind specifically to a target selected from the group of: CD16a, CD33, CD20, CD19, CD22, CD123, PDL-1, TIGIT, PD-1, TIM3, CTLA4, MICA, MICB, IL-6, IL-8, TNFα, CD26, CD36, ULBP2, CD30, CD200, IGF-1R, MUC4AC, MUC5AC, Trop-2, CMET, EGFR, HER1, HER2, HER3, PSMA, CEA, B7H3, EPCAM, BCMA, P-cadherin, CEACAM5, a UL16-binding protein, HLA-DR, DLL4, TYRO3, AXL, MER, CD122, CD155, PDGF-DD, a ligand of TGF-β receptor II (TGF-βRII), a ligand of TGF-βRIII, a ligand of DNAM-1, a ligand of NKp46, a ligand of NKp44, a ligand of NKG2D, a ligand of NKp30, a ligand for a scMHCI, a ligand for a scMHCII, a ligand for a scTCR, a receptor for PDGF-DD, a receptor for stem cell factor (SCF), a receptor for stem cell-like tyrosine kinase 3 ligand (FLT3L), a receptor for MICA, a receptor for MICB, a receptor for a ULP16-binding protein, a receptor for CD155, and a receptor for CD122.

In some embodiments of any of the multi-chain chimeric polypeptides described herein, one or both of the first target-binding domain (e.g., any of the target-binding domains described herein), the second target-binding domain (e.g., any of the target-binding domains described herein), and the one or more additional binding domains (e.g., any of the target-binding described herein) is a soluble interleukin or cytokine protein. In some embodiments of any of the multi-chain chimeric polypeptides described herein, the soluble interleukin or cytokine protein is selected from the group of: IL-1, IL-2, IL-3, IL-7, IL-8, IL-10, IL-12, IL-15, IL-17, IL-18, IL-21, PDGF-DD, and SCF.

In some embodiments of any of the multi-chain chimeric polypeptides described herein, one or both of the first target-binding domain and the second target-binding domain is a soluble interleukin or cytokine receptor. In some embodiments of any of the multi-chain chimeric polypeptides described herein, the soluble receptor is a soluble TGF-β receptor II (TGF-βRII) a soluble TGF-βRIII, a soluble receptor for TNFα, a soluble receptor for IL-4, or a soluble receptor for IL-10.

Non-limiting examples of cell activating agents are multi-chain chimeric polypeptides that include: (a) a first and second chimeric polypeptide each including: (i) a first target-binding domain; (ii) a Fc domain; and (iii) a first domain of a pair of affinity domains; and (b) a third and fourth chimeric polypeptide each including: (i) a second domain of a pair of affinity domains; and (ii) a second target-binding domain, where the first and second chimeric polypeptides and the third and fourth chimeric polypeptides associate through the binding of the first domain and the second domain of the pair of affinity domains, and the first and second chimeric polypeptides associate through their Fc domains.

In some embodiments of any of the multi-chain chimeric polypeptides described herein, the first target-binding domain (e.g., any of the first target-binding domains described herein) and the Fc domain (e.g., any of the exemplary Fc domains described herein) directly abut each other in the first and second chimeric polypeptides. In some embodiments of any of the multi-chain chimeric polypeptides described herein, the first and second chimeric polypeptides further comprise a linker sequence (e.g., any of the exemplary linker sequences described herein or known in the art) between the first target-binding domain (e.g., any of the exemplary first target-binding domains described herein) and the Fc domain (e.g., any of the exemplary Fc domains described herein) in the first and second chimeric polypeptides.

In some embodiments of any of the multi-chain chimeric polypeptides described herein, the Fc domain (e.g., any of the exemplary Fc domains described herein) and the first domain of the pair of affinity domains (e.g., any of the exemplary first domains of any of the exemplary pairs of affinity domains described herein) directly abut each other in the first and second chimeric polypeptide. In some embodiments of any of the multi-chain chimeric polypeptides described herein, the first and second chimeric polypeptide further comprises a linker sequence (e.g., any of the exemplary linker sequences described herein or known in the art) between the Fc domain (e.g., any of the exemplary Fc domains described herein) and the first domain of the pair of affinity domains (e.g., any of the exemplary first domains of any of the exemplary pairs of affinity domains described herein) in the first and second chimeric polypeptide.

In some embodiments of any of the multi-chain chimeric polypeptides described herein, the second domain of the pair of affinity domains (e.g., any of the exemplary second domains of any of the exemplary pairs of affinity domains described herein) and the second target-binding domain (e.g., any of the exemplary second target-binding domains described herein) directly abut each other in the third and fourth chimeric polypeptide. In some embodiments of any of the multi-chain chimeric polypeptides described herein, the third and fourth chimeric polypeptide further comprise a linker sequence (e.g., any of the exemplary linker sequences described herein or known in the art) between the second domain of the pair of affinity domains (e.g., any of the exemplary second domains of any of the exemplary pairs of affinity domains described herein) and the second target-binding domain (e.g., any of the exemplary second target-binding domains described herein) in the third and fourth chimeric polypeptide.

In some embodiments of any of the multi-chain chimeric polypeptides described herein, the first target-binding domain and the second target-binding domain bind specifically to the same antigen. In some embodiments of any of the multi-chain chimeric polypeptides described herein, the first target-binding domain and the second target-binding domain bind specifically to the same epitope. In some embodiments of any of the multi-chain chimeric polypeptides described herein, the first target-binding domain and the second target-binding domain include the same amino acid sequence. In some embodiments of any of the multi-chain chimeric polypeptides described herein, the first target-binding domain and the second target-binding domain bind specifically to different antigens. In some embodiments of any of the multi-chain chimeric polypeptides described herein, one or both of the first target-binding domain and the second target-binding domain is an antigen-binding domain (e.g., any of the exemplary second target-binding domains described herein). In some embodiments of any of the multi-chain chimeric polypeptides described herein, the first target-binding domain and the second target-binding domain are each antigen-binding domains (e.g., any of the exemplary second target-binding domains described herein). In some embodiments of any of the multi-chain chimeric polypeptides described herein, the antigen-binding domain (e.g., any of the exemplary second target-binding domains described herein) includes a scFv or a single domain antibody.

In some embodiments of any of the multi-chain chimeric polypeptides described herein, one or both of the first target-binding domain (e.g., any of the exemplary target-binding domains described herein or known in the art) and the second target-binding domain (e.g., any of the exemplary target-binding domains described herein or known in the art) bind specifically to a target selected from the group consisting of: CD16a, CD28, CD3, CD33, CD20, CD19, CD22, CD123, IL-1R, IL-1, VEGF, IL-6R, IL-4, IL-10, PDL-1, TIGIT, PD-1, TIM3, CTLA4, MICA, MICB, IL-6, IL-8, TNFα, CD26, CD36, ULBP2, CD30, CD200, IGF-1R, MUC4AC, MUC5AC, Trop-2, CMET, EGFR, HER1, HER2, HER3, PSMA, CEA, B7H3, EPCAM, BCMA, P-cadherin, CEACAM5, a UL16-binding protein, HLA-DR, DLL4, TYRO3, AXL, MER, CD122, CD155, PDGF-DD, a ligand of TGF-β receptor II (TGF-βRII), a ligand of TGF-βRIII, a ligand of DNAM1, a ligand of NKp46, a ligand of NKp44, a ligand of NKG2D, a ligand of NKp30, a ligand for a scMHCI, a ligand for a scMHCII, a ligand for a scTCR, a receptor for IL-1, a receptor for IL-2, a receptor for IL-3, a receptor for IL-7, a receptor for IL-8, a receptor for IL-10, a receptor for IL-12, a receptor for IL-15, a receptor for IL-17, a receptor for IL-18, a receptor for IL-21, a receptor for PDGF-DD, a receptor for stem cell factor (SCF), a receptor for stem cell-like tyrosine kinase 3 ligand (FLT3L), a receptor for MICA, a receptor for MICB, a receptor for a ULP16-binding protein, a receptor for CD155, a receptor for CD122, and a receptor for CD28.

In some embodiments of any of the multi-chain chimeric polypeptides described herein, one or both of the first target-binding domain (e.g., any of the exemplary target-binding domains described herein or known in the art) and the second target-binding domain (e.g., any of the exemplary target-binding domains described herein or known in the art) is a soluble interleukin or cytokine protein. Non-limiting examples of soluble interleukin proteins and soluble cytokine proteins include: IL-1, IL-2, IL-3, IL-7, IL-8, IL-10, IL-12, IL-15, IL-17, IL-18, IL-21, PDGF-DD, and SCF.

In some embodiments of any of the multi-chain chimeric polypeptides described herein, one or both of the first target-binding domain and the second target-binding domain (e.g., any of the exemplary target-binding domains described herein or known in the art) is a soluble interleukin or cytokine receptor. Non-limiting examples of soluble interleukin receptors and soluble cytokine receptors include: a soluble TGF-β receptor II (TGF-βRII), a soluble TGF-βRIII, a soluble NKG2D, a soluble NKp30, a soluble NKp44, a soluble NKp46, a soluble DNAM1, a scMHCI, a scMHCII, a scTCR, a soluble CD155, a soluble CD122, a soluble CD3, or a soluble CD28.

In some embodiments of any of the multi-chain chimeric polypeptides described herein, the first target-binding domain and the second target-binding domain can each, independently, bind specifically to a target selected from the group of: CD16a, CD33, CD20, CD19, CD22, CD123, PDL-1, TIGIT, PD-1, TIM3, CTLA4, MICA, MICB, IL-6, IL-8, TNFα, CD26, CD36, ULBP2, CD30, CD200, IGF-1R, MUC4AC, MUC5AC, Trop-2, CMET, EGFR, HER1, HER2, HER3, PSMA, CEA, B7H3, EPCAM, BCMA, P-cadherin, CEACAM5, a UL16-binding protein, HLA-DR, DLL4, TYRO3, AXL, MER, CD122, CD155, PDGF-DD, a ligand of TGF-β receptor II (TGF-βRII), a ligand of TGF-βRIII, a ligand of DNAM1, a ligand of NKp46, a ligand of NKp44, a ligand of NKG2D, a ligand of NKp30, a ligand for a scMHCI, a ligand for a scMHCII, a ligand for a scTCR, a receptor for PDGF-DD, a receptor for stem cell factor (SCF), a receptor for stem cell-like tyrosine kinase 3 ligand (FLT3L), a receptor for MICA, a receptor for MICB, a receptor for a ULP16-binding protein, a receptor for CD155, and a receptor for CD122.

In some embodiments of any of the multi-chain chimeric polypeptides described herein, one or both of the first target-binding domain and the second target-binding domain is a soluble interleukin or cytokine protein. In some embodiments of any of the multi-chain chimeric polypeptides described herein, the soluble interleukin or cytokine protein is selected from the group of: IL-1, IL-2, IL-3, IL-7, IL-8, IL-10, IL-12, IL-15, IL-17, IL-18, IL-21, PDGF-DD, and SCF.

In some embodiments of any of the multi-chain chimeric polypeptides described herein, one or both of the first target-binding domain and the second target-binding domain is a soluble interleukin or cytokine receptor. In some embodiments of any of the multi-chain chimeric polypeptides described herein, the soluble receptor is a soluble TGF-β receptor II (TGF-βRII) a soluble TGF-βRIII, a soluble receptor for TNFα, a soluble receptor for IL-4, or a soluble receptor for IL-10.

In some embodiments, a multi-chain chimeric polypeptide has a nucleic acid sequence or an amino acid that is at least 80% identical (e.g., at least 82% identical, at least 84% identical, at least 86% identical, at least 88% identical, at least 90% identical, at least 92% identical, at least 94% identical, at least 96% identical, at least 98% identical, at least 99% identical, or 100% identical) to: SEQ ID NOs: 30-101.

### Additional Antigen-Binding Domains

In some embodiments of any of the single- or multi-chain chimeric polypeptides, the first chimeric polypeptide further includes one or more (e.g., two, three, four, five, six, seven, eight, nine, or ten) additional target-binding domain(s) (e.g., any of the exemplary target-binding domains described herein or known in the art). In some embodiments of any of the multi-chain chimeric polypeptides, at least one of the one or more additional antigen-binding domain(s) can be positioned between the soluble tissue factor domain (e.g., any of the exemplary soluble tissue factor domains described herein or known in the art) and the first domain of the pair of affinity domains (e.g., any of the exemplary first domains of any of the exemplary pairs of affinity domains described herein). In some embodiments, the first chimeric polypeptide can further include a linker sequence (e.g., any of the exemplary linker sequences described herein or known in the art) between the soluble tissue factor domain (e.g., any of the exemplary soluble tissue factor domains described herein) and the at least one of the one or more additional target-binding domain(s) (e.g., any of the exemplary target-binding domains described herein or known in the art), and/or a linker sequence (e.g., any of the exemplary linker sequences described herein or known in the art) between the at least one of the one or more additional target-binding domain(s) (e.g., any of the exemplary target-binding domains described herein or known in the art) and the first domain of the pair of affinity domains (e.g., any of the exemplary first domains described herein of any of the exemplary pairs of affinity domains described herein).

In some embodiments of any of the multi-chain chimeric polypeptides described herein, the first chimeric polypeptide further includes one or more (e.g., two, three, four, five, six, seven, eight, nine, or ten) additional target-binding domains at the N-terminal and/or C-terminal end of the first chimeric polypeptide. In some embodiments, at least one of the one or more additional target-binding domains (e.g., any of the exemplary target-binding domains described herein or known in the art) directly abuts the first domain of the pair of affinity domains (e.g., any of the exemplary first domains described herein of any of the exemplary pairs of affinity domains described herein) in the first chimeric polypeptide. In some embodiments, the first chimeric polypeptide further includes a linker sequence (e.g., any of the exemplary linker sequences described herein or known in the art) between the at least one of the one or more additional target-binding domains (e.g., any of the exemplary target-binding domains described herein or known in the art) and the first domain of the pair of affinity domains (e.g., any of the exemplary first domains described herein of any of the exemplary pairs of affinity domains described herein). In some embodiments, the at least one of the one or more additional target-binding domains (e.g., any of the exemplary target-binding domains described herein or known in the art) directly abuts the first target-binding domain (e.g., any of the exemplary target-binding domains described herein or known in the art) in the first chimeric polypeptide. In some embodiments, the first chimeric polypeptide further comprises a linker sequence (e.g., any of the exemplary linker sequences described herein or known in the art) between the at least one of the one or more additional target-binding domains (e.g., any of the exemplary target-binding domains described herein or known in the art) and the first target-binding domain (e.g., any of the exemplary target-binding domains described herein or known in the art).

In some embodiments of any of the multi-chain chimeric polypeptides described herein, at least one of the one or more additional target-binding domains (e.g., any of the exemplary target-binding domains described herein or known in the art) is disposed at the N- and/or C-terminus of the first chimeric polypeptide, and at least one of the one or more additional target-binding domains (e.g., any of the exemplary target-binding domains described herein or known in the art) is positioned between the soluble tissue factor domain (e.g., any of the exemplary soluble tissue factor domains described herein or known in the art) and the first domain of the pair of affinity domains (e.g., any of the exemplary first domains of any of the exemplary pairs of affinity domains described herein) in the first chimeric polypeptide. In some embodiments, the at least one additional target-binding domain (e.g., any of the exemplary target-binding domains described herein or known in the art) of the one or more additional target-binding domains disposed at the N-terminus directly abuts the first target-binding domain (e.g., any of the exemplary target-binding domains described herein or known in the art) or the first domain of the pair of affinity domains (e.g., any of the exemplary first domains described herein of any of the exemplary pairs of affinity domains described herein) in the first chimeric polypeptide. In some embodiments, the first chimeric polypeptide further comprises a linker sequence (e.g., any of the linker sequences described herein or known in the art) disposed between the at least one additional target-binding domain (e.g., any of the exemplary target-binding domains described herein or known in the art) and the first target-binding domain (e.g., any of the exemplary target-binding domains described herein or known in the art) or the first domain of the pair of affinity domains (e.g., any of the exemplary first domains described herein of any of the exemplary pairs of affinity domains described herein) in the first chimeric polypeptide. In some embodiments, the at least one additional target-binding domain (e.g., any of the exemplary target-binding domains described herein or known in the art) of the one or more additional target-binding domains disposed at the C-terminus directly abuts the first target-binding domain (e.g., any of the exemplary target-binding domains described herein or known in the art) or the first domain of the pair of affinity domains (e.g., any of the exemplary first domains of any of the exemplary pairs of affinity domains described herein) in the first chimeric polypeptide. In some embodiments, the first chimeric polypeptide further includes a linker sequence (e.g., any of the exemplary linker sequences described herein or known in the art) disposed between the at least one additional target-binding domain (e.g., any of the exemplary target-binding domains described herein or known in the art) and the first target-binding domain (e.g., any of the exemplary target-binding domains described herein or known in the art) or the first domain of the pair of affinity domains (e.g., any of the exemplary first domains described herein of any of the exemplary pairs of affinity domains described herein) in the first chimeric polypeptide. In some embodiments, the at least one of the one or more additional target-binding domains (e.g., any of the exemplary target-binding domains described herein or known in the art) positioned between the soluble tissue factor domain (e.g., any of the exemplary soluble tissue factor domains described herein) and the first domain of the pair of affinity domains (e.g., any of the first domains described herein or any of the exemplary pairs of affinity domains described herein), directly abuts the soluble tissue factor domain and/or the first domain of the pair of affinity domains. In some embodiments, the first chimeric polypeptide further comprises a linker sequence (e.g., any of the exemplary linker sequences described herein or known in the art) disposed (i) between the soluble tissue factor domain (e.g., any of the exemplary soluble tissue factor domains described herein) and the at least one of the one or more additional target-binding domains (e.g., any of the exemplary target-binding domains described herein or known in the art) positioned between the soluble tissue factor domain (e.g., any of the exemplary soluble tissue factor domains described herein) and the first domain of the pair of affinity domains (e.g., any of the exemplary first domains of any of the exemplary pairs of affinity domains described herein), and/or (ii) between the first domain of the pair of affinity domains and the at least one of the one or more additional target-binding domains positioned between the soluble tissue factor domain and the first domain of the pair of affinity domains.

In some embodiments of any of the multi-chain chimeric polypeptides described herein, the second chimeric polypeptide further includes one or more (e.g., two, three, four, five, six, seven, eight, nine, or ten) additional target-binding domains (e.g., any of the exemplary target-binding domains described herein or known in the art) at the N-terminal end and/or the C-terminal end of the second chimeric polypeptide. In some embodiments, at least one of the one or more additional target-binding domains (e.g., any of the exemplary target-binding domains described herein or known in the art) directly abuts the second domain of the pair of affinity domains (e.g., any of the exemplary second domains of any of the exemplary pairs of affinity domains described herein) in the second chimeric polypeptide. In some embodiments, the second chimeric polypeptide further includes a linker sequence (e.g., any of the exemplary linker sequences described herein or known in the art) between at least one of the one or more additional target-binding domains (e.g., any of the exemplary target-binding domains described herein or known in the art) and the second domain of the pair of affinity domains (e.g., any of the second domains described herein of any of the exemplary pairs of affinity domains described herein) in the second chimeric polypeptide. In some embodiments, at least one of the one or more additional target-binding domains (e.g., any of the exemplary target-binding domains described herein or known in the art) directly abuts the second target-binding domain (e.g., any of the target-binding domains described herein or known in the art) in the second chimeric polypeptide. In some embodiments, the second chimeric polypeptide further includes a linker sequence (e.g., any of the exemplary linker sequences described herein or known in the art) between at least one of the one or more additional target-binding domains (e.g., any of the exemplary target binding domains described herein or known in the art) and the second target-binding domain (e.g., any of the exemplary target binding domains described herein or known in the art) in the second chimeric polypeptide.

In some embodiments of any of the multi-chain chimeric polypeptides described herein, two or more (e.g., three or more, four or more, five or more, six or more, seven or more, eight or more, nine or more, or ten or more) of the first target-binding domain, the second target-binding domain, and the one or more additional target-binding domains bind specifically to the same antigen. In some embodiments, two or more (e.g., three or more, four or more, five or more, six or more, seven or more, eight or more, nine or more, or ten or more) of the first target-binding domain, the second target-binding domain, and the one or more additional target-binding domains bind specifically to the same epitope. In some embodiments, two or more (e.g., three or more, four or more, five or more, six or more, seven or more, eight or more, nine or more, or ten or more) of the first target-binding domain, the second target-binding domain, and the one or more additional target-binding domains include the same amino acid sequence. In some embodiments, the first target-binding domain, the second target-binding domain, and the one or more additional target-binding domains each bind specifically to the same antigen. In some embodiments, the first target-binding domain, the second target-binding domain, and the one or more additional target-binding domains each bind specifically to the same epitope. In some embodiments, the first target-binding domain, the second target-binding domain, and the one or more additional target-binding domains each include the same amino acid sequence.

In some embodiments of any of the multi-chain chimeric polypeptides described herein, the first target-binding domain, the second target-binding domain, and the one or more additional target-binding domains bind specifically to different antigens. In some embodiments of any of the multi-chain chimeric polypeptides described herein, one or more (e.g., two or more, three or more, four or more, five or more, six or more, seven or more, eight or more, nine or more, or ten or more) of the first target-binding domain, the second target-binding domain, and the one or more target-binding domains is an antigen-binding domain. In some embodiments, the first target-binding domain, the second target-binding domain, and the one or more additional target-binding domains are each an antigen-binding domain (e.g., a scFv or a single-domain antibody).

### Pairs of Affinity Domains

In some embodiments, a multi-chain chimeric polypeptide includes: 1) a first chimeric polypeptide that includes a first domain of a pair of affinity domains, and 2) a second chimeric polypeptide that includes a second domain of a pair of affinity domains such that the first chimeric polypeptide and the second chimeric polypeptide associate through the binding of the first domain and the second domain of the pair of affinity domains. In some embodiments, the pair of affinity domains is a sushi domain from an alpha chain of human IL-15 receptor (IL15Rα) and a soluble IL-15. A sushi domain, also known as a short consensus repeat or type 1 glycoprotein motif, is a common motif in protein-protein interaction. Sushi domains have been identified on a number of protein-binding molecules, including complement components C1r, C1s, factor H, and C2m, as well as the nonimmunologic molecules factor XIII and β2-glycoprotein. A typical Sushi domain has approximately 60 amino acid residues and contains four cysteines (Ranganathan, Pac. Symp Biocomput. 2000:155-67). The first cysteine can form a disulfide bond with the third cysteine, and the second cysteine can form a disulfide bridge with the fourth cysteine. In some embodiments in which one member of the pair of affinity domains is a soluble IL-15, the soluble IL15 has a D8N or D8A amino acid substitution. In some embodiments in which one member of the pair of affinity domains is an alpha chain of human IL-15 receptor (IL15Rα), the human IL15Rα is a mature full-length IL15Rα. In some embodiments, the pair of affinity domains is barnase and barnstar. In some embodiments, the pair of affinity domains is a PKA and an AKAP. In some embodiments, the pair of affinity domains is an adapter/docking tag module based on mutated RNase I fragments (Rossi, Proc Natl Acad Sci USA. 103:6841-6846, 2006; Sharkey et al., Cancer Res. 68:5282-5290, 2008; Rossi et al., Trends Pharmacol Sci. 33:474-481, 2012) or SNARE modules based on interactions of the proteins syntaxin, synaptotagmin, synaptobrevin, and SNAP25 (Deyev et al., Nat Biotechnol. 1486-1492, 2003).

In some embodiments, a first chimeric polypeptide of a multi-chain chimeric polypeptide includes a first domain of a pair of affinity domains and a second chimeric polypeptide of the multi-chain chimeric polypeptide includes a second domain of a pair of affinity domains, wherein the first domain of the pair of affinity domains and the second domain of the pair of affinity domains bind to each other with a dissociation equilibrium constant (K_{D}) of less than 1 x 10⁻⁷ M, less than 1 x 10⁻⁸ M, less than 1 x 10⁻⁹ M, less than 1 x 10⁻¹⁰ M, less than 1 x 10⁻¹¹ M, less than 1 x 10⁻¹² M, or less than 1 x 10⁻¹³ M. In some embodiments, the first domain of the pair of affinity domains and the second domain of the pair of affinity domains bind to each other with a K_{D} of about 1 x 10⁻⁴ M to about 1 x 10⁻⁶ M, about 1 x 10⁻⁵ M to about 1 x 10⁻⁷ M, about 1 x 10⁻⁶ M to about 1 x 10⁻⁸ M, about 1 x 10⁻⁷ M to about 1 x 10⁻⁹ M, about 1 x 10⁻⁸ M to about 1 x 10⁻¹⁰ M, about 1 x 10⁻⁹ M to about 1 x 10⁻¹¹ M, about 1 x 10⁻¹⁰ M to about 1 x 10⁻¹² M, about 1 x 10⁻¹¹ M to about 1 x 10⁻¹³ M, about 1 x 10⁻⁴ M to about 1 x 10⁻⁵ M, about 1 x 10⁻⁵ M to about 1 x 10⁻⁶ M, about 1 x 10⁻⁶ M to about 1 x 10⁻⁷ M, about 1 x 10⁻⁷ M to about 1 x 10⁻⁸ M, about 1 x 10⁻⁸ M to about 1 x 10⁻⁹ M, about 1 x 10⁻⁹ M to about 1 x 10⁻¹⁰ M, about 1 x 10⁻¹⁰ M to about 1 x 10⁻¹¹ M, about 1 x 10⁻¹¹ M to about 1 x 10⁻¹² M, or about 1 x 10⁻¹² M to about 1 x 10⁻¹³ M (inclusive). Any of a variety of different methods known in the art can be used to determine the K_{D} value of the binding of the first domain of the pair of affinity domains and the second domain of the pair of affinity domains (e.g., an electrophoretic mobility shift assay, a filter binding assay, surface plasmon resonance, and a biomolecular binding kinetics assay, etc.).

In some embodiments, a first chimeric polypeptide of a multi-chain chimeric polypeptide includes a first domain of a pair of affinity domains and a second chimeric polypeptide of the multi-chain chimeric polypeptide includes a second domain of a pair of affinity domains, wherein the first domain of the pair of affinity domains, the second domain of the pair of affinity domains, or both is about 10 to 100 amino acids in length. For example, a first domain of a pair of affinity domains, a second domain of a pair of affinity domains, or both can be about 10 to 100 amino acids in length, about 15 to 100 amino acids in length, about 20 to 100 amino acids in length, about 25 to 100 amino acids in length, about 30 to 100 amino acids in length, about 35 to 100 amino acids in length, about 40 to 100 amino acids in length, about 45 to 100 amino acids in length, about 50 to 100 amino acids in length, about 55 to 100 amino acids in length, about 60 to 100 amino acids in length, about 65 to 100 amino acids in length, about 70 to 100 amino acids in length, about 75 to 100 amino acids in length, about 80 to 100 amino acids in length, about 85 to 100 amino acids in length, about 90 to 100 amino acids in length, about 95 to 100 amino acids in length, about 10 to 95 amino acids in length, about 10 to 90 amino acids in length, about 10 to 85 amino acids in length, about 10 to 80 amino acids in length, about 10 to 75 amino acids in length, about 10 to 70 amino acids in length, about 10 to 65 amino acids in length, about 10 to 60 amino acids in length, about 10 to 55 amino acids in length, about 10 to 50 amino acids in length, about 10 to 45 amino acids in length, about 10 to 40 amino acids in length, about 10 to 35 amino acids in length, about 10 to 30 amino acids in length, about 10 to 25 amino acids in length, about 10 to 20 amino acids in length, about 10 to 15 amino acids in length, about 20 to 30 amino acids in length, about 30 to 40 amino acids in length, about 40 to 50 amino acids in length, about 50 to 60 amino acids in length, about 60 to 70 amino acids in length, about 70 to 80 amino acids in length, about 80 to 90 amino acids in length, about 90 to 100 amino acids in length, about 20 to 90 amino acids in length, about 30 to 80 amino acids in length, about 40 to 70 amino acids in length, about 50 to 60 amino acids in length, or any range in between. In some embodiments, a first domain of a pair of affinity domains, a second domain of a pair of affinity domains, or both is about 10, 15, 20, 25, 30, 35, 40, 45, 50, 55, 60, 65, 70, 75, 80, 85, 90, 95, or 100 amino acids in length.

In some embodiments, any of the first and/or second domains of a pair of affinity domains disclosed herein can include one or more additional amino acids (e.g., 1, 2, 3, 5, 6, 7, 8, 9, 10, or more amino acids) at its N-terminus and/or C-terminus, so long as the function of the first and/or second domains of a pair of affinity domains remains intact. For example, a sushi domain from an alpha chain of human IL-15 receptor (IL15Rα) can include one or more additional amino acids at the N-terminus and/or the C-terminus, while still retaining the ability to bind to a soluble IL-15. Additionally or alternatively, a soluble IL-15 can include one or more additional amino acids at the N-terminus and/or the C-terminus, while still retaining the ability to bind to a sushi domain from an alpha chain of human IL-15 receptor (IL15Rα).

A non-limiting example of a sushi domain from an alpha chain of IL-15 receptor alpha (IL15Rα) can include a sequence that is at least 70% identical, at least 75% identical, at least 80% identical, at least 85% identical, at least 90% identical, at least 95% identical, at least 99% identical, or 100% identical to ITCPPPMSVEHADIWVKSYSLYSRERYICNSGFKRKAGTSSLTECVLNKATNVAH WTTPSLKCIR (SEQ ID NO: 123). In some embodiments, a sushi domain from an alpha chain of IL15Rα can be encoded by a nucleic acid including

ATTACATGCCCCCCTCCCATGAGCGTGGAGCACGCCGACATCTGGGTGAAGAG
CTATAGCCTCTACAGCCGGGAGAGGTATATCTGTAACAGCGGCTTCAAGAGGA

AGGCCGGCACCAGCAGCCTCACCGAGTGCGTGCTGAATAAGGCTACCAACGT
GGCTCACTGGACAACACCCTCTTTAAAGTGCATCCGG (SEQ ID NO: 124).

In some embodiments, a soluble IL-15 can include a sequence that is at least 70% identical, at least 75% identical, at least 80% identical, at least 85% identical, at least 90% identical, at least 95% identical, at least 99% identical, or 100% identical to NWVNVISDLKKIEDLIQSMHIDATLYTESDVHPSCKVTAMKCFLLELQVISLESGD ASIHDTVENLIILANNSLSSNGNVTESGCKECEELEEKNIKEFLQSFVHIVQMFINT S (SEQ ID NO: 125). In some embodiments, a soluble IL-15 can be encoded by a nucleic acid including the sequence of

AACTGGGTGAACGTCATCAGCGATTTAAAGAAGATCGAAGATTAATTCAGTC
CATGCATATCGACGCCACTTTATACACAGAATCCGACGTGCACCCCTCTTGTAA
GGTGACCGCCATGAAATGTTTTTACTGGAGCTGCAAGTTATCTCTTAGAGAG
CGGAGACGCTAGCATCCACGACACCGTGGAGAATTTAATCATTTAGCCAATA
ACTCTTTATCCAGCAACGGCAACGTGACAGAGTCCGGCTGCAAGGAGTGCGA
AGAGCTGGAGGAGAAGAACATCAAGGAGTTTCTGCAATCCTTTGTGCACATTG
TCCAGATGTTCATCAATACCTCC (SEQ ID NO: 126).

### Exemplary Multi-Chain Chimeric Polypeptides- Type A

In some embodiments of any of the multi-chain chimeric polypeptides described herein, the first target-binding domain and the second targeting-binding domain each independently bind specifically to a receptor of IL-18 or a receptor of IL-12. In some examples of these multi-chain chimeric polypeptides, the first target-binding domain and the soluble tissue factor domain directly abut each other in the first chimeric polypeptide. In some examples of these multi-chain chimeric polypeptides, the first chimeric polypeptide further comprises a linker sequence (e.g., any of the exemplary linkers described herein) between the first target-binding domain and the soluble tissue factor domain in the first chimeric polypeptide.

In some embodiments of these multi-chain chimeric polypeptides, one or both of the first target-binding domain and the second target-binding domain is a soluble IL-15 or a soluble IL-18. In some embodiments of these multi-chain chimeric polypeptides, the first target-binding domain and the second target-binding domain are each independently a soluble IL-15 or a soluble IL-18. In some embodiments of these multi-chain chimeric polypeptides, the first target-binding domain and the second target-binding domain both bind specifically to a receptor of IL-18 or a receptor of IL-12. In some embodiments of these multi-chain chimeric polypeptides, the first target-binding domain and the second target-binding domain bind specifically to the same epitope. In some embodiments of these multi-chain chimeric polypeptides, the first target-binding domain and the second target-binding domain include the same amino acid sequence.

In some embodiments of these multi-chain chimeric polypeptides, the first target-binding domain binds specifically to a receptor for IL-12, and the second target-binding domain binds specifically to a receptor for IL-18. In some embodiments of these multi-chain chimeric polypeptides, the first target-binding domain binds specifically to a receptor for IL-18, and the second target-binding domain bind specifically to a receptor for IL-12.

In some embodiments of these multi-chain chimeric polypeptides, the first target-binding domain includes a soluble IL-18 (e.g., a soluble human IL-18).

In some embodiments of these multi-chain chimeric polypeptides, the second target-binding domain includes a soluble IL-12 (e.g., a soluble human IL-12). In some embodiments of these multi-chain chimeric polypeptides, the soluble human IL-15 includes a sequence of soluble human IL-12β (p40) and a sequence of soluble human IL-12α (p35). In some embodiments of these multi-chain chimeric polypeptides, the soluble IL-15 human IL-15 further includes a linker sequence (e.g., any of the exemplary linker sequences described herein) between the sequence of soluble IL-12β (p40) and the sequence of soluble human IL-12α (p35).

### Exemplary Multi-Chain Chimeric Polypeptides- Type B

In some embodiments of any of the multi-chain chimeric polypeptides described herein, the first target-binding domain and the second targeting-binding domain each independently bind specifically to a receptor of IL-21 or to TGF-β. In some examples of these multi-chain chimeric polypeptides, the first target-binding domain and the soluble tissue factor domain directly abut each other in the first chimeric polypeptide. In some examples of these multi-chain chimeric polypeptides, the first chimeric polypeptide further comprises a linker sequence (e.g., any of the exemplary linkers described herein) between the first target-binding domain and the soluble tissue factor domain in the first chimeric polypeptide.

In some embodiments of these multi-chain chimeric polypeptides, one or both of the first target-binding domain and the second target-binding domain is a soluble IL-21 (e.g., a soluble human IL-21 polypeptide) or a soluble TGF-β receptor (e.g., a soluble TGFRβRII receptor). In some embodiments of these multi-chain chimeric polypeptides, the first target-binding domain and the second target-binding domain are each independently a soluble IL-21 or a soluble TGF-β receptor (e.g., a soluble TGFRβRII receptor). In some embodiments of these multi-chain chimeric polypeptides, the first target-binding domain and the second target-binding domain both bind specifically to a receptor of IL-21 or to TGF-β. In some embodiments of these multi-chain chimeric polypeptides, the first target-binding domain and the second target-binding domain bind specifically to the same epitope. In some embodiments of these multi-chain chimeric polypeptides, the first target-binding domain and the second target-binding domain include the same amino acid sequence.

In some embodiments of these multi-chain chimeric polypeptides, the first target-binding domain binds specifically to a receptor for IL-21, and the second target-binding domain binds specifically to TGF-β. In some embodiments of these multi-chain chimeric polypeptides, the first target-binding domain binds specifically to TGF-β, and the second target-binding domain bind specifically to a receptor for IL-21.

In some embodiments of these multi-chain chimeric polypeptides, the second target-binding domain includes a soluble TGF-β receptor (e.g., a soluble TGFRβRII receptor (e.g., a soluble human TGFRβRII receptor)). In some embodiments of these multi-chain chimeric polypeptides, the soluble human TGFRβRII includes a first sequence of soluble human TGFRβRII and a second sequence of soluble human TGFRβRII. In some embodiments of these multi-chain chimeric polypeptides, the soluble human TGFRβRII includes a linker disposed between the first sequence of soluble human TGFRβRII and the second sequence of soluble human TGFRβRII.

### Exemplary Multi-Chain Chimeric Polypeptides- Type C

In some embodiments of any of the multi-chain chimeric polypeptides described herein, the first target-binding domain and the second targeting-binding domain each independently bind specifically to a receptor of IL-7 or a receptor of IL-21. In some examples of these multi-chain chimeric polypeptides, the first target-binding domain and the soluble tissue factor domain directly abut each other in the first chimeric polypeptide. In some examples of these multi-chain chimeric polypeptides, the first chimeric polypeptide further comprises a linker sequence (e.g., any of the exemplary linkers described herein) between the first target-binding domain and the soluble tissue factor domain in the first chimeric polypeptide.

In some embodiments of these multi-chain chimeric polypeptides, one or both of the first target-binding domain and the second target-binding domain is a soluble IL-21 (e.g., a soluble human IL-21 polypeptide) or a soluble IL-7 (e.g., a soluble human IL-7 polypeptide). In some embodiments of these multi-chain chimeric polypeptides, the first target-binding domain and the second target-binding domain are each independently a soluble IL-21 or a soluble IL-7. In some embodiments of these multi-chain chimeric polypeptides, the first target-binding domain and the second target-binding domain both bind specifically to a receptor of IL-21 or a receptor of IL-7. In some embodiments of these multi-chain chimeric polypeptides, the first target-binding domain and the second target-binding domain bind specifically to the same epitope. In some embodiments of these multi-chain chimeric polypeptides, the first target-binding domain and the second target-binding domain include the same amino acid sequence.

In some embodiments of these multi-chain chimeric polypeptides, the first target-binding domain binds specifically to a receptor for IL-21, and the second target-binding domain binds specifically to a receptor for IL-7. In some embodiments of these multi-chain chimeric polypeptides, the first target-binding domain binds specifically to a receptor for IL-7, and the second target-binding domain binds specifically to a receptor for IL-21.

In some embodiments of these multi-chain chimeric polypeptides, the first target-binding domain includes a soluble IL-21 (e.g., a soluble human IL-21).

### Exemplary Multi-Chain Chimeric Polypeptides- Type D

In some embodiments of any of the multi-chain chimeric polypeptides described herein, the first target-binding domain and the second targeting-binding domain each independently bind specifically to a receptor of IL-7 or a receptor of IL-21. In some examples of these multi-chain chimeric polypeptides, the first target-binding domain and the soluble tissue factor domain directly abut each other in the first chimeric polypeptide. In some examples of these multi-chain chimeric polypeptides, the first chimeric polypeptide further comprises a linker sequence (e.g., any of the exemplary linkers described herein) between the first target-binding domain and the soluble tissue factor domain in the first chimeric polypeptide.

In some embodiments of these multi-chain chimeric polypeptides, one or both of the first target-binding domain and the second target-binding domain is a soluble IL-21 (e.g., a soluble human IL-21 polypeptide) or a soluble IL-7 (e.g., a soluble human IL-7 polypeptide). In some embodiments of these multi-chain chimeric polypeptides, the first target-binding domain and the second target-binding domain are each independently a soluble IL-21 or a soluble IL-7. In some embodiments of these multi-chain chimeric polypeptides, the first target-binding domain and the second target-binding domain both bind specifically to a receptor of IL-21 or a receptor of IL-7. In some embodiments of these multi-chain chimeric polypeptides, the first target-binding domain and the second target-binding domain bind specifically to the same epitope. In some embodiments of these multi-chain chimeric polypeptides, the first target-binding domain and the second target-binding domain include the same amino acid sequence.

In some embodiments of these multi-chain chimeric polypeptides, the first target-binding domain binds specifically to a receptor for IL-21, and the second target-binding domain binds specifically to a receptor for IL-7. In some embodiments of these multi-chain chimeric polypeptides, the first target-binding domain binds specifically to a receptor for IL-7, and the second target-binding domain binds specifically to a receptor for IL-21.

### Exemplary Multi-Chain Chimeric Polypeptides- Type E

In some embodiments of any of the multi-chain chimeric polypeptides described herein, the first target-binding domain and the second targeting-binding domain each independently bind specifically to a receptor for IL-18 (e.g., a soluble human IL-18), a receptor for IL-12 (e.g., a soluble human IL-12), or CD16 (e.g., an anti-CD16 scFv). In some embodiments of these multi-chain chimeric polypeptides described herein, the first chimeric polypeptide further includes the additional target-binding domain. In some embodiments of these multi-chain chimeric polypeptides described herein, the second chimeric polypeptide further includes the additional target-binding domain. In some embodiments of these multi-chain chimeric polypeptides described herein, the additional target-binding domain binds specifically to CD16 or a receptor for IL-12.

In some embodiments of these multi-chain chimeric polypeptides, one or both of the first target-binding domain and the second target-binding domain is a soluble IL-15 or a soluble IL-18. In some embodiments of these multi-chain chimeric polypeptides, the first target-binding domain and the second target-binding domain are each independently a soluble IL-15 or a soluble IL-18. In some embodiments of these multi-chain chimeric polypeptides, the first target-binding domain and the second target-binding domain both bind specifically to a receptor of IL-18 or a receptor of IL-12. In some embodiments of these multi-chain chimeric polypeptides, the first target-binding domain and the second target-binding domain bind specifically to the same epitope. In some embodiments of these multi-chain chimeric polypeptides, the first target-binding domain and the second target-binding domain include the same amino acid sequence.

In some embodiments of these multi-chain chimeric polypeptides, the first target-binding domain binds specifically to a receptor for IL-12, and the second target-binding domain binds specifically to a receptor for IL-18. In some embodiments of these multi-chain chimeric polypeptides, the first target-binding domain binds specifically to a receptor for IL-18, and the second target-binding domain bind specifically to a receptor for IL-12. In some embodiments of these multi-chain chimeric polypeptides, the first target-binding domain binds specifically to CD16, and the second target-binding domain binds specifically to a receptor for IL-18. In some embodiments of these multi-chain chimeric polypeptides, the first target-binding domain binds specifically to a receptor for IL-18, and the second target-binding domain bind specifically to CD16.

In some embodiments of these multi-chain chimeric polypeptides, two or more of the first target-binding domain, the second target-binding domain, and the one or more additional target-binding domains bind specifically to the same antigen. In some embodiments, two or more of the first target-binding domain, the second target-binding domain, and the one or more additional target-binding domains bind specifically to the same epitope. In some embodiments, two or more of the first target-binding domain, the second target-binding domain, and the one or more additional target-binding domains comprise the same amino acid sequence.

In some embodiments of these multi-chain chimeric polypeptides, the first target-binding domain includes a soluble IL-18 (e.g., a soluble human IL-18).

### Exemplary Multi-Chain Chimeric Polypeptides- Type F

In some embodiments of any of the multi-chain chimeric polypeptides described herein, the first target-binding domain and the second targeting-binding domain each independently bind specifically to a receptor for IL-7 (e.g., a soluble human IL-7), CD16 (e.g., an anti-CD16 scFv), or a receptor for IL-21 (e.g., a soluble human IL-21). In some embodiments of these multi-chain chimeric polypeptides described herein, the first chimeric polypeptide further includes the additional target-binding domain. In some embodiments of these multi-chain chimeric polypeptides described herein, the second chimeric polypeptide further includes the additional target-binding domain. In some embodiments of these multi-chain chimeric polypeptides described herein, the additional target-binding domain binds specifically to CD16 or a receptor for IL-21.

In some embodiments of any of the multi-chain chimeric polypeptides described herein, the first target-binding domain binds specifically to a receptor IL-7 and the second target-binding domain binds specifically to CD16 or a receptor for IL-21. In some embodiments of any of the multi-chain chimeric polypeptides described herein, the first target-binding domain includes a soluble IL-7 protein. In some embodiments of any of the multi-chain chimeric polypeptides described herein, the soluble IL-7 protein is a soluble human IL-7. In some embodiments of any of the multi-chain chimeric polypeptides described herein, the second antigen-binding domain includes a target-binding domain that binds specifically to CD16. In some embodiments of any of the multi-chain chimeric polypeptides described herein, the second target-binding domain includes an scFv that binds specifically to CD 16. In some embodiments of any of the multi-chain chimeric polypeptides described herein, the second target-binding domain binds specifically to a receptor for IL-21. In some embodiments of any of the multi-chain chimeric polypeptides described herein, the second target-binding domain includes a soluble IL-21. In some embodiments of any of the multi-chain chimeric polypeptides described herein, the soluble IL-21 is a soluble human IL-21. In some embodiments of any of the multi-chain chimeric polypeptides described herein, the second chimeric polypeptide further includes an additional target-binding domain that binds specifically to a receptor for IL-21. In some embodiments of any of the multi-chain chimeric polypeptides described herein, the additional target-binding domain includes a soluble IL-21. In some embodiments of any of the multi-chain chimeric polypeptides described herein, the soluble IL-21 is a soluble human IL-21. In some embodiments of any of the multi-chain chimeric polypeptides described herein, the second chimeric polypeptide further includes an additional target-binding domain that binds specifically to CD16.

In some embodiments of these multi-chain chimeric polypeptides, two or more of the first target-binding domain, the second target-binding domain, and the one or more additional target-binding domains bind specifically to the same antigen. In some embodiments, two or more of the first target-binding domain, the second target-binding domain, and the one or more additional target-binding domains bind specifically to the same epitope. In some embodiments, two or more of the first target-binding domain, the second target-binding domain, and the one or more additional target-binding domains comprise the same amino acid sequence.

In some embodiments of these multi-chain chimeric polypeptides, the first target-binding domain includes a soluble IL-7 (e.g., a soluble human IL-7).

### Exemplary Multi-Chain Chimeric Polypeptides- Type G

In some embodiments of any of the multi-chain chimeric polypeptides described herein, the first target-binding domain and the second targeting-binding domain each independently bind specifically to TGFβ (e.g., a human TGFβRII receptor), CD16 (e.g., an anti-CD16 scFv), or a receptor for IL-21 (e.g., a soluble human IL-21). In some embodiments of these multi-chain chimeric polypeptides described herein, the first chimeric polypeptide further includes the additional target-binding domain. In some embodiments of these multi-chain chimeric polypeptides described herein, the second chimeric polypeptide further includes the additional target-binding domain. In some embodiments of these multi-chain chimeric polypeptides described herein, the additional target-binding domain binds specifically to CD16 or a receptor for IL-21.

In some embodiments of any of the multi-chain chimeric polypeptides described herein, the first target-binding domain and the second targeting-binding domain each independently bind specifically to TGF-β, CD16, or a receptor for IL-21. In some embodiments of any of the multi-chain chimeric polypeptides described herein, the first target-binding domain binds specifically to a TGF-β and the second target-binding domain binds specifically to CD16 or a receptor of IL-21. In some embodiments of any of the multi-chain chimeric polypeptides described herein, the first target-binding domain is a soluble TGF-β receptor. In some embodiments of any of the multi-chain chimeric polypeptides described herein, soluble TGF-β receptor is a soluble TGFβRII receptor. In some embodiments of any of the multi-chain chimeric polypeptides described herein, the second target-binding domain binds specifically to CD16. In some embodiments of any of the multi-chain chimeric polypeptides described herein, the second antigen-binding domain includes an antigen-binding domain that binds specifically to CD16. In some embodiments of any of the multi-chain chimeric polypeptides described herein, the second antigen-binding domain includes an scFv that binds specifically to CD16. In some embodiments of any of the multi-chain chimeric polypeptides described herein, the second target-binding domain binds specifically to a receptor for IL-21. In some embodiments of any of the multi-chain chimeric polypeptides described herein, the second target-binding domain includes a soluble IL-21. In some embodiments of any of the multi-chain chimeric polypeptides described herein, the second target-binding domain includes a soluble human IL-21. In some embodiments of any of the multi-chain chimeric polypeptides described herein, the second chimeric polypeptide further includes an additional target-binding domain that binds specifically to a receptor for IL-21. In some embodiments of any of the multi-chain chimeric polypeptides described herein, the additional target-binding domain includes a soluble IL-21. In some embodiments of any of the multi-chain chimeric polypeptides described herein, the soluble IL-21 is a soluble human IL-21. In some embodiments of any of the multi-chain chimeric polypeptides described herein, the second chimeric polypeptide further includes an additional target-binding domain that binds specifically to CD16.

In some embodiments of these multi-chain chimeric polypeptides, two or more of the first target-binding domain, the second target-binding domain, and the one or more additional target-binding domains bind specifically to the same antigen. In some embodiments, two or more of the first target-binding domain, the second target-binding domain, and the one or more additional target-binding domains bind specifically to the same epitope. In some embodiments, two or more of the first target-binding domain, the second target-binding domain, and the one or more additional target-binding domains comprise the same amino acid sequence.

In some embodiments of these multi-chain chimeric polypeptides, the first target-binding domain includes a TGFβRII receptor (e.g., a soluble human TGFβRII receptor). In some embodiments of these multi-chain chimeric polypeptides, the soluble human TGFRβRII includes a first sequence of soluble human TGFRβRII and a second sequence of soluble human TGFRβRII. In some embodiments of these multi-chain chimeric polypeptides, the soluble human TGFRβRII includes a linker disposed between the first sequence of soluble human TGFRβRII and the second sequence of soluble human TGFRβRII.

### Exemplary Multi-Chain Chimeric Polypeptides- Type H

In some embodiments of any of the multi-chain chimeric polypeptides described herein, the first target-binding domain and the second targeting-binding domain each independently bind specifically to a receptor of IL-7. In some examples of these multi-chain chimeric polypeptides, the first target-binding domain and the soluble tissue factor domain directly abut each other in the first chimeric polypeptide. In some examples of these multi-chain chimeric polypeptides, the first chimeric polypeptide further comprises a linker sequence (e.g., any of the exemplary linkers described herein) between the first target-binding domain and the soluble tissue factor domain in the first chimeric polypeptide.

### Exemplary Multi-Chain Chimeric Polypeptides- Type I

In some embodiments of any of the multi-chain chimeric polypeptides described herein, the first target-binding domain and the second targeting-binding domain each independently bind specifically to TGF-β. In some examples of these multi-chain chimeric polypeptides, the first target-binding domain and the soluble tissue factor domain directly abut each other in the first chimeric polypeptide. In some examples of these multi-chain chimeric polypeptides, the first chimeric polypeptide further comprises a linker sequence (e.g., any of the exemplary linkers described herein) between the first target-binding domain and the soluble tissue factor domain in the first chimeric polypeptide.

In some embodiments of these multi-chain chimeric polypeptides, the first target-binding domain and the second target-binding domain each independently bind specifically to TGF-β. In some embodiments of these multi-chain chimeric polypeptides, the first target-binding domain and the second target-binding domain bind specifically to the same epitope. In some embodiments of these multi-chain chimeric polypeptides, the first target-binding domain and the second target-binding domain include the same amino acid sequence.

In some embodiments of these multi-chain chimeric polypeptides, the first target-binding domain and the second target-binding domain is a soluble TGF-β receptor (e.g., a soluble TGFβRII receptor, e.g., a soluble human TGFβRII). In some embodiments of these multi-chain chimeric polypeptides, the soluble human TGFRβRII includes a first sequence of soluble human TGFRβRII and a second sequence of soluble human TGFRβRII. In some embodiments of these multi-chain chimeric polypeptides, the soluble human TGFRβRII includes a linker disposed between the first sequence of soluble human TGFRβRII and the second sequence of soluble human TGFRβRII.

### Exemplary Multi-Chain Chimeric Polypeptides- Type J

In some embodiments of any of the multi-chain chimeric polypeptides described herein, the first target-binding domain and the second targeting-binding domain each independently bind specifically to a receptor of IL-7, a receptor of IL-21, or a receptor of CD137L. In some embodiments of these multi-chain chimeric polypeptides described herein, the second chimeric polypeptide further includes the additional target-binding domain. In some embodiments of these multi-chain chimeric polypeptides described herein, the additional target-binding domain binds specifically to a receptor for IL-21 (e.g., a soluble IL-21, e.g., a soluble human IL-21) or a receptor for CD137L (e.g., a soluble CD137L, e.g., a soluble human CD137L).

In some embodiments of these multi-chain chimeric polypeptides, the first target-binding domain binds specifically to a receptor for IL-7, and the second target-binding domain binds specifically to a receptor for IL-21 or a receptor for CD137L. In some embodiments, the additional target-binding domain binds specifically to a receptor for IL-21 or a receptor for CD137L.

### Exemplary Multi-Chain Chimeric Polypeptides- Type K

In some embodiments of any of the multi-chain chimeric polypeptides described herein, the first target-binding domain and the second targeting-binding domain each independently bind specifically to a receptor of IL-7 or TGF-β. In some examples of these multi-chain chimeric polypeptides, the first target-binding domain and the soluble tissue factor domain directly abut each other in the first chimeric polypeptide. In some examples of these multi-chain chimeric polypeptides, the first chimeric polypeptide further comprises a linker sequence (e.g., any of the exemplary linkers described herein) between the first target-binding domain and the soluble tissue factor domain in the first chimeric polypeptide.

In some embodiments of these multi-chain chimeric polypeptides, the first target-binding domain binds specifically to a receptor for IL-7, and the second target-binding domain binds specifically to TGF-β. In some embodiments of these multi-chain chimeric polypeptides, the first target-binding domain binds specifically to TGF-β, and the second target-binding domain binds specifically to a receptor for IL-7.

In some embodiments of these multi-chain chimeric polypeptides, the second target-binding domain comprises a target-binding domain that binds specifically to TGF-β. In some embodiments of these multi-chain chimeric polypeptides, the second target-binding domain is a soluble TGF-β receptor (e.g., a soluble TGFβRII receptor, e.g., a soluble human TGFβRII receptor). In some embodiments of these multi-chain chimeric polypeptides, the soluble human TGFRβRII includes a first sequence of soluble human TGFRβRII and a second sequence of soluble human TGFRβRII. In some embodiments of these multi-chain chimeric polypeptides, the soluble human TGFRβRII includes a linker disposed between the first sequence of soluble human TGFRβRII and the second sequence of soluble human TGFRβRII.

### Exemplary Multi-Chain Chimeric Polypeptides- Type L

In some embodiments of any of the multi-chain chimeric polypeptides described herein, the first target-binding domain and the second targeting-binding domain each independently bind specifically to TGF-β, a receptor of IL-21, or a receptor of CD137L. In some embodiments of these multi-chain chimeric polypeptides described herein, the second chimeric polypeptide further includes the additional target-binding domain. In some embodiments of these multi-chain chimeric polypeptides described herein, the additional target-binding domain binds specifically to a receptor for IL-21 (e.g., a soluble IL-21, e.g., a soluble human IL-21) or a receptor for CD137L (e.g., a soluble CD137L, e.g., a soluble human CD137L).

In some embodiments of these multi-chain chimeric polypeptides, the first target-binding domain binds specifically to TGF-β and the second target-binding domain binds specifically to a receptor for IL-21 or a receptor for CD137L.

In some embodiments of these multi-chain chimeric polypeptides, the first target-binding domain is a soluble TGF-β receptor (e.g., a soluble TGFβRII receptor, e.g., a soluble human TGFβRII receptor). In some embodiments of these multi-chain chimeric polypeptides, the soluble human TGFRβRII includes a first sequence of soluble human TGFRβRII and a second sequence of soluble human TGFRβRII. In some embodiments of these multi-chain chimeric polypeptides, the soluble human TGFRβRII includes a linker disposed between the first sequence of soluble human TGFRβRII and the second sequence of soluble human TGFRβRII.

### Exemplary Multi-Chain Chimeric Polypeptides- Type M

In some embodiments of any of the multi-chain chimeric polypeptides described herein, the first target-binding domain and the second targeting-binding domain each independently bind specifically to TGF-β or a receptor of IL-21. In some embodiments of these multi-chain chimeric polypeptides described herein, the second chimeric polypeptide further includes the additional target-binding domain. In some embodiments of these multi-chain chimeric polypeptides described herein, the additional target-binding domain binds specifically to a receptor for IL-21 (e.g., a soluble IL-21, e.g., a soluble human IL-21) or a TGF-β (e.g., a soluble TGF-β receptor, e.g., a soluble TGFβRII receptor).

In some embodiments of these multi-chain chimeric polypeptides, the first target-binding domain binds specifically to TGF-β, and the second target-binding domain binds specifically to TGF-β or a receptor for IL-21. In some embodiments of these multi-chain chimeric polypeptides, the first target-binding domain is a soluble TGF-β receptor (e.g., a soluble TGFβRII receptor, e.g., a soluble human TGFβRII receptor). In some embodiments of these multi-chain chimeric polypeptides, the soluble human TGFRβRII includes a first sequence of soluble human TGFRβRII and a second sequence of soluble human TGFRβRII. In some embodiments of these multi-chain chimeric polypeptides, the soluble human TGFRβRII includes a linker disposed between the first sequence of soluble human TGFRβRII and the second sequence of soluble human TGFRβRII.

### Exemplary Multi-Chain Chimeric Polypeptides- Type N

In some embodiments of any of the multi-chain chimeric polypeptides described herein, the first target-binding domain and the second targeting-binding domain each independently bind specifically to TGF-β or CD16. In some embodiments of these multi-chain chimeric polypeptides described herein, the second chimeric polypeptide further includes the additional target-binding domain. In some embodiments of these multi-chain chimeric polypeptides described herein, the additional target-binding domain binds specifically to CD16 (e.g., an anti-CD16 scFv) or a TGF- β (e.g., a soluble TGF-β receptor, e.g., a soluble TGFβRII receptor).

In some embodiments of these multi-chain chimeric polypeptides, the first target-binding domain binds specifically to TGF-β, and the second target-binding domain binds specifically to TGF-β or CD16. In some embodiments of these multi-chain chimeric polypeptides, the first target-binding domain is a soluble TGF-β receptor (e.g., a soluble TGFβRII receptor, e.g., a soluble human TGFβRII receptor). In some embodiments of these multi-chain chimeric polypeptides, the soluble human TGFRβRII includes a first sequence of soluble human TGFRβRII and a second sequence of soluble human TGFRβRII. In some embodiments of these multi-chain chimeric polypeptides, the soluble human TGFRβRII includes a linker disposed between the first sequence of soluble human TGFRβRII and the second sequence of soluble human TGFRβRII.

### Exemplary Multi-Chain Chimeric Polypeptides- Type O

In some embodiments of any of the multi-chain chimeric polypeptides described herein, the first target-binding domain and the second targeting-binding domain each independently bind specifically to TGF-β or a receptor of CD137L. In some embodiments of these multi-chain chimeric polypeptides described herein, the second chimeric polypeptide further includes the additional target-binding domain. In some embodiments of these multi-chain chimeric polypeptides described herein, the additional target-binding domain binds specifically to a receptor to TGF- β (e.g., a soluble TGF-β receptor, e.g., a soluble TGFβRII receptor) or CD137L.

In some examples of these multi-chain chimeric polypeptides, the first target-binding domain and the soluble tissue factor domain directly abut each other in the first chimeric polypeptide. In some examples of these multi-chain chimeric polypeptides, the first chimeric polypeptide further comprises a linker sequence (e.g., any of the exemplary linkers described herein) between the first target-binding domain and the soluble tissue factor domain in the first chimeric polypeptide.

In some embodiments of these multi-chain chimeric polypeptides, the soluble tissue factor domain and the first domain of the pair of affinity domains directly abut each other in the first chimeric polypeptide. In some embodiments of these multi-chain chimeric polypeptides, the first chimeric polypeptide further includes a linker sequence (e.g., any of the exemplary linkers described herein) between the soluble tissue factor domain and the first domain of the pair of affinity domains in the first chimeric polypeptide.

In some embodiments of these multi-chain chimeric polypeptides, the second domain of the pair of affinity domains and the second target-binding domain directly abut each other in the second chimeric polypeptide. In some embodiments of these multi-chain chimeric polypeptides, the second chimeric polypeptide further includes a linker sequence (e.g., any of the exemplary linkers described herein) between the second domain of the pair of affinity domains and the second target-binding domain in the second chimeric polypeptide.

In some embodiments, the second chimeric polypeptide further comprises one or more additional target-binding domains at the N-terminal end or the C-terminal end of the second chimeric polypeptide.

In some embodiments of these multi-chain chimeric polypeptides, the additional target-binding domain and the second domain of the pair of affinity domains directly abut each other in the second chimeric polypeptide. In some embodiments of these multi-chain chimeric polypeptides, the second chimeric polypeptide further includes a linker sequence (e.g., any of the exemplary linkers described herein) between the second domain of the pair of affinity domains and the additional target-binding domain in the second chimeric polypeptide.

In some embodiments of these multi-chain chimeric polypeptides, the additional target-binding domain and the second target-binding domain directly abut each other in the second chimeric polypeptide. In some embodiments of these multi-chain chimeric polypeptides, the second chimeric polypeptide further includes a linker sequence (e.g., any of the exemplary linkers described herein) between the second target-binding domain and the additional target-binding domain in the second chimeric polypeptide.

In some embodiments of these multi-chain chimeric polypeptides, the soluble tissue factor domain can be any of the exemplary soluble tissue factor domains described herein. In some embodiments of these multi-chain chimeric polypeptides, the pair of affinity domains can be any of the exemplary pairs of affinity domains described herein.

In some embodiments of these multi-chain chimeric polypeptides, the first target-binding domain binds specifically to TGF-β, and the second target-binding domain binds specifically to CD137L. In some embodiments of these multi-chain chimeric polypeptides, the first target-binding domain or the additional target-binding domain is a soluble TGF-β receptor (e.g., a soluble TGFβRII receptor, e.g., a soluble human TGFβRII receptor).

In some embodiments of these multi-chain chimeric polypeptides, the soluble human TGFRβRII includes a first sequence of soluble human TGFRβRII and a second sequence of soluble human TGFRβRII. In some embodiments of these multi-chain chimeric polypeptides, the soluble human TGFRβRII includes a linker disposed between the first sequence of soluble human TGFRβRII and the second sequence of soluble human TGFRβRII.

### Sequences of Exemplary Multi-Chain Chimeric Polypeptides

The nucleic acid sequence of an IL12/IL-15RαSu construct (including signal peptide sequence) is as follows (SEQ ID NO: 30):
*(Signal peptide)* 
ATGAAATGGGTGACCTTTATTTCTTTACTGTTCCTCTTTAGCAGCGCCT
ACTCC
*(Human IL-12 subunit beta (p40))* 
ATTTGGGAACTGAAGAAGGACGTCTACGTGGTCGAACTGGACTGGTAT
CCCGATGCTCCCGGCGAAATGGTGGTGCTCACTTGTGACACCCCCGAAGAAG
ACGGCATCACTTGGACCCTCGATCAGAGCAGCGAGGTGCTGGGCTCCGGAAA
GACCCTCACAATCCAAGTTAAGGAGTTCGGAGACGCTGGCCAATACACATGC
CACAAGGGAGGCGAGGTGCTCAGCCATTCCTTATTATTATTACACAAGAAGG
AAGACGGAATCTGGTCCACCGACATTTTAAAAGATCAGAAGGAGCCCAAGA
ATAAGACCTTTTTAAGGTGTGAGGCCAAAAACTACAGCGGTCGTTTCACTTGT
TGGTGGCTGACCACCATTTCCACCGATTTAACCTTCTCCGTGAAAAGCAGCCG
GGGAAGCTCCGACCCTCAAGGTGTGACATGTGGAGCCGCTACCCTCAGCGCT
GAGAGGGTTCGTGGCGATAACAAGGAATACGAGTACAGCGTGGAGTGCCAA
GAAGATAGCGCTTGTCCCGCTGCCGAAGAATCTTTACCCATTGAGGTGATGG
TGGACGCCGTGCACAAACTCAAGTACGAGAACTACACCTCCTCCTTCTTTATC
CGGGACATCATTAAGCCCGATCCTCCTAAGAATTTACAGCTGAAGCCTCTCA
AAAATAGCCGGCAAGTTGAGGTCTCTTGGGAATATCCCGACACTTGGAGCAC
ACCCCACAGCTACTTCTCTTTAACCTTTTGTGTGCAAGTTCAAGGTAAAAGCA
AGCGGGAGAAGAAAGACCGGGTGTTTACCGACAAAACCAGCGCCACCGTCA
TCTGTCGGAAGAACGCCTCCATCAGCGTGAGGGCTCAAGATCGTTATTACTCC
AGCAGCTGGTCCGAGTGGGCCAGCGTGCCTTGTTCC
*(Linker)*
   GGCGGTGGAGGATCCGGAGGAGGTGGCTCCGGCGGCGGAGGATCT
*(Human IL-12 subunit alpha (p35))* 
CGTAACCTCCCCGTGGCTACCCCCGATCCCGGAATGTTCCCTTGTTTAC
ACCACAGCCAGAATTTACTGAGGGCCGTGAGCAACATGCTGCAGAAAGCTAG
GCAGACTTTAGAATTTTACCCTTGCACCAGCGAGGAGATCGACCATGAAGAT
ATCACCAAGGACAAGACATCCACCGTGGAGGCTTGTTTACCTCTGGAGCTGA
CAAAGAACGAGTCTTGTCTCAACTCTCGTGAAACCAGCTTCATCACAAATGG
CTCTTGTTTAGCTTCCCGGAAGACCTCCTTTATGATGGCTTTATGCCTCAGCTC
 
CATCTACGAGGATTTAAAGATGTACCAAGTGGAGTTCAAGACCATGAACGCC
AAGCTGCTCATGGACCCTAAACGGCAGATCTTTTTAGACCAGAACATGCTGG
CTGTGATTGATGAGCTGATGCAAGCTTTAAACTTCAACTCCGAGACCGTCCCT
CAGAAGTCCTCCCTCGAGGAGCCCGATTTTTACAAGACAAAGATCAAACTGT
GCATTTTACTCCACGCCTTTAGGATCCGGGCCGTGACCATTGACCGGGTCATG
AGCTATTTAAACGCCAGC
*(Human IL-15R α sushi domain)*

ATTACATGCCCCCCTCCCATGAGCGTGGAGCACGCCGACATCTGGGTG
AAGAGCTATAGCCTCTACAGCCGGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG

The amino acid sequence of an IL12/IL-15RαSu fusion protein (including signal peptide sequence) is as follows (SEQ ID NO: 31):
*(Signal peptide)*
   MKWVTFISLLFLFSSAYS
*(Human IL-12 subunit beta (p40))* 
IWELKKDVYVVELDWYPDAPGEMVVLTCDTPEEDGITWTLDQSSEVLGS
GKTLTIQVKEFGDAGQYTCHKGGEVLSHSLLLLHKKEDGIWSTDILKDQKEPKN
KTFLRCEAKNYSGRFTCWWLTTISTDLTFSVKSSRGSSDPQGVTCGAATLSAERV
RGDNKEYEYSVECQEDSACPAAEESLPIEVMVDAVHKLKYENYTSSFFIRDIIKPD
PPKNLQLKPLKNSRQVEVSWEYPDTWSTPHSYFSLTFCVQVQGKSKREKKDRVF
TDKTSATVICRKNASISVRAQDRYYSSSWSEWASVPCS
*(Linker)*
   GGGGSGGGGSGGGGS
*(Human IL-12 subunit alpha (p35))* 
RNLPVATPDPGMFPCLHHSQNLLRAVSNMLQKARQTLEFYPCTSEEIDHE
DITKDKTSTVEACLPLELTKNESCLNSRETSFITNGSCLASRKTSFMMALCLSSIYE
DLKMYQVEFKTMNAKLLMDPKRQIFLDQNMLAVIDELMQALNFNSETVPQKSS
LEEPDFYKTKIKLCILLHAFRIRAVTIDRVMSYLNAS
*(Human IL-15R α sushi domain)*

ITCPPPMSVEHADIWVKSYSLYSRERYICNSGFKRKAGTSSLTECVLNKAT
NVAHWTTPSLKCIR

The nucleic acid sequence of an IL-18/TF/IL-15 construct is as follows (SEQ ID NO: 32):
*(Signal peptide)* 
ATGAAGTGGGTCACATTTATCTCTTTACTGTTCCTCTTCTCCAGCGCCT
ACAGC
*(Human IL-18)* 
TACTTCGGCAAACTGGAATCCAAGCTGAGCGTGATCCGGAATTTAAAC
GACCAAGTTCTGTTTATCGATCAAGGTAACCGGCCTCTGTTCGAGGACATGAC
CGACTCCGATTGCCGGGACAATGCCCCCCGGACCATCTTCATTATCTCCATGT
ACAAGGACAGCCAGCCCCGGGGCATGGCTGTGACAATTAGCGTGAAGTGTGA
GAAAATCAGCACTTTATCTTGTGAGAACAAGATCATCTCCTTTAAGGAAATG
AACCCCCCCGATAACATCAAGGACACCAAGTCCGATATCATCTTCTTCCAGC
GGTCCGTGCCCGGTCACGATAACAAGATGCAGTTCGAATCCTCCTCCTACGA
GGGCTACTTTTTAGCTTGTGAAAAGGAGAGGGATTTATTCAAGCTGATCCTCA
AGAAGGAGGACGAGCTGGGCGATCGTTCCATCATGTTCACCGTCCAAAACGA
GGAT
*(Human Tissue Factor 219)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
 
GTATTACTGGAAGTCCTCTTCCTCCGGCAAGAAGACAGCTAAAACCAACACA
AACGAGTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of an IL-18/TF/IL-15 fusion protein (including signal peptide sequence) is as follows (SEQ ID NO: 33):
*(Signal peptide)*
   MKWVTFISLLFLFSSAYS
*(Human IL-18)* 
YFGKLESKLSVIRNLNDQVLFIDQGNRPLFEDMTDSDCRDNAPRTIFIISM
YKDSQPRGMAVTISVKCEKISTLSCENKIISFKEMNPPDNIKDTKSDIIFFQRSVPG
HDNKMQFESSSYEGYFLACEKERDLFKLILKKEDELGDRSIMFTVQNED
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of an IL-12/IL-15RαSu/αCD16scFv construct is as follows (SEQ ID NO: 34):
*(Signal peptide)*
   ATGAAATGGGTGACCTTTATTTCTTTACTGTTCCTCTTTAGCAGCGCCTACTCC
*(Human IL-12 subunit beta (p40))* 
ATTTGGGAACTGAAGAAGGACGTCTACGTGGTCGAACTGGACTGGTAT
CCCGATGCTCCCGGCGAAATGGTGGTGCTCACTTGTGACACCCCCGAAGAAG
ACGGCATCACTTGGACCCTCGATCAGAGCAGCGAGGTGCTGGGCTCCGGAAA
GACCCTCACAATCCAAGTTAAGGAGTTCGGAGACGCTGGCCAATACACATGC
CACAAGGGAGGCGAGGTGCTCAGCCATTCCTTATTATTATTACACAAGAAGG
AAGACGGAATCTGGTCCACCGACATTTTAAAAGATCAGAAGGAGCCCAAGA
ATAAGACCTTTTTAAGGTGTGAGGCCAAAAACTACAGCGGTCGTTTCACTTGT
TGGTGGCTGACCACCATTTCCACCGATTTAACCTTCTCCGTGAAAAGCAGCCG
GGGAAGCTCCGACCCTCAAGGTGTGACATGTGGAGCCGCTACCCTCAGCGCT
GAGAGGGTTCGTGGCGATAACAAGGAATACGAGTACAGCGTGGAGTGCCAA
GAAGATAGCGCTTGTCCCGCTGCCGAAGAATCTTTACCCATTGAGGTGATGG
TGGACGCCGTGCACAAACTCAAGTACGAGAACTACACCTCCTCCTTCTTTATC
CGGGACATCATTAAGCCCGATCCTCCTAAGAATTTACAGCTGAAGCCTCTCA
AAAATAGCCGGCAAGTTGAGGTCTCTTGGGAATATCCCGACACTTGGAGCAC
ACCCCACAGCTACTTCTCTTTAACCTTTTGTGTGCAAGTTCAAGGTAAAAGCA
AGCGGGAGAAGAAAGACCGGGTGTTTACCGACAAAACCAGCGCCACCGTCA
TCTGTCGGAAGAACGCCTCCATCAGCGTGAGGGCTCAAGATCGTTATTACTCC
AGCAGCTGGTCCGAGTGGGCCAGCGTGCCTTGTTCC
*(Linker)*
   GGCGGTGGAGGATCCGGAGGAGGTGGCTCCGGCGGCGGAGGATCT
*(Human IL-12 subunit alpha (p35))* 
CGTAACCTCCCCGTGGCTACCCCCGATCCCGGAATGTTCCCTTGTTTAC
ACCACAGCCAGAATTTACTGAGGGCCGTGAGCAACATGCTGCAGAAAGCTAG
GCAGACTTTAGAATTTTACCCTTGCACCAGCGAGGAGATCGACCATGAAGAT
ATCACCAAGGACAAGACATCCACCGTGGAGGCTTGTTTACCTCTGGAGCTGA
CAAAGAACGAGTCTTGTCTCAACTCTCGTGAAACCAGCTTCATCACAAATGG
CTCTTGTTTAGCTTCCCGGAAGACCTCCTTTATGATGGCTTTATGCCTCAGCTC
CATCTACGAGGATTTAAAGATGTACCAAGTGGAGTTCAAGACCATGAACGCC
AAGCTGCTCATGGACCCTAAACGGCAGATCTTTTTAGACCAGAACATGCTGG
CTGTGATTGATGAGCTGATGCAAGCTTTAAACTTCAACTCCGAGACCGTCCCT
CAGAAGTCCTCCCTCGAGGAGCCCGATTTTTACAAGACAAAGATCAAACTGT
GCATTTTACTCCACGCCTTTAGGATCCGGGCCGTGACCATTGACCGGGTCATG
AGCTATTTAAACGCCAGC
*(Human IL-15R α sushi domain)* 
ATTACATGCCCCCCTCCCATGAGCGTGGAGCACGCCGACATCTGGGTG
AAGAGCTATAGCCTCTACAGCCGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*(anti-Human CD16 light chain variable domain)* 
TCCGAGCTGACCCAGGACCCTGCTGTGTCCGTGGCTCTGGGCCAGACC
GTGAGGATCACCTGCCAGGGCGACTCCCTGAGGTCCTACTACGCCTCCTGGT
ACCAGCAGAAGCCCGGCCAGGCTCCTGTGCTGGTGATCTACGGCAAGAACAA
CAGGCCCTCCGGCATCCCTGACAGGTTCTCCGGATCCTCCTCCGGCAACACCG
CCTCCCTGACCATCACAGGCGCTCAGGCCGAGGACGAGGCTGACTACTACTG
CAACTCCAGGGACTCCTCCGGCAACCATGTGGTGTTCGGCGGCGGCACCAAG
CTGACCGTGGGCCAT
*(Linker)*
   GGCGGCGGCGGCTCCGGAGGCGGCGGCAGCGGCGGAGGAGGATCC
*(anti-Human CD16 heavy chain variable domain)*

GAGGTGCAGCTGGTGGAGTCCGGAGGAGGAGTGGTGAGGCCTGGAGG
CTCCCTGAGGCTGAGCTGTGCTGCCTCCGGCTTCACCTTCGACGACTACGGCA
TGTCCTGGGTGAGGCAGGCTCCTGGAAAGGGCCTGGAGTGGGTGTCCGGCAT
CAACTGGAACGGCGGATCCACCGGCTACGCCGATTCCGTGAAGGGCAGGTTC
ACCATCAGCAGGGACAACGCCAAGAACTCCCTGTACCTGCAGATGAACTCCC
TGAGGGCCGAGGACACCGCCGTGTACTACTGCGCCAGGGGCAGGTCCCTGCT
GTTCGACTACTGGGGACAGGGCACCCTGGTGACCGTGTCCAGG

The amino acid sequence of an IL-12/IL-15RαSu/αCD16scFv fusion protein (including signal peptide sequence) is as follows (SEQ ID NO: 35):
*(Signal peptide)*
   MKWVTFISLLFLFSSAYS
*(Human IL-12 subunit beta (p40))* 
IWELKKDVYVVELDWYPDAPGEMVVLTCDTPEEDGITWTLDQSSEVLGS
GKTLTIQVKEFGDAGQYTCHKGGEVLSHSLLLLHKKEDGIWSTDILKDQKEPKN
KTFLRCEAKNYSGRFTCWWLTTISTDLTFSVKSSRGSSDPQGVTCGAATLSAERV
RGDNKEYEYSVECQEDSACPAAEESLPIEVMVDAVHKLKYENYTSSFFIRDIIKPD
PPKNLQLKPLKNSRQVEVSWEYPDTWSTPHSYFSLTFCVQVQGKSKREKKDRVF
TDKTSATVICRKNASISVRAQDRYYSSSWSEWASVPCS
*(Linker)*
   GGGGSGGGGSGGGGS
*(Human IL-12 subunit alpha (p35))* 
RNLPVATPDPGMFPCLHHSQNLLRAVSNMLQKARQTLEFYPCTSEEIDHE
DITKDKTSTVEACLPLELTKNESCLNSRETSFITNGSCLASRKTSFMMALCLSSIYE
DLKMYQVEFKTMNAKLLMDPKRQIFLDQNMLAVIDELMQALNFNSETVPQKSS
LEEPDFYKTKIKLCILLHAFRIRAVTIDRVMSYLNAS
*(Human IL-15R α sushi domain)* 
ITCPPPMSVEHADIWVKSYSLYSRERYICNSGFKRKAGTSSLTECVLNKAT
NVAHWTTPSLKCIR
*(anti-Human CD16 light chain variable domain)* 
SELTQDPAVSVALGQTVRITCQGDSLRSYYASWYQQKPGQAPVLVIYGK
NNRPSGIPDRFSGSSSGNTASLTITGAQAEDEADYYCNSRDSSGNHVVFGGGTKL
TVGH
*(Linker)*
   GGGGSGGGGSGGGGS
*(anti-Human CD16 heavy chain variable domain)*

EVQLVESGGGVVRPGGSLRLSCAASGFTFDDYGMSWVRQAPGKGLEWV
SGINWNGGSTGYADSVKGRFTISRDNAKNSLYLQMNSLRAEDTAVYYCARGRS
LLFDYWGQGTLVTVSR

The nucleic acid sequence of an IL-18/IL-15RαSu construct (including signal peptide sequence) is as follows (SEQ ID NO: 36):
*(Signal peptide)* 
ATGAAGTGGGTCACATTTATCTCTTTACTGTTCCTCTTCTCCAGCGCCT
ACAGC
*(Human IL-18)* 
TACTTCGGCAAACTGGAATCCAAGCTGAGCGTGATCCGGAATTTAAAC
GACCAAGTTCTGTTTATCGATCAAGGTAACCGGCCTCTGTTCGAGGACATGAC
CGACTCCGATTGCCGGGACAATGCCCCCCGGACCATCTTCATTATCTCCATGT
ACAAGGACAGCCAGCCCCGGGGCATGGCTGTGACAATTAGCGTGAAGTGTGA
GAAAATCAGCACTTTATCTTGTGAGAACAAGATCATCTCCTTTAAGGAAATG
AACCCCCCCGATAACATCAAGGACACCAAGTCCGATATCATCTTCTTCCAGC
GGTCCGTGCCCGGTCACGATAACAAGATGCAGTTCGAATCCTCCTCCTACGA
GGGCTACTTTTTAGCTTGTGAAAAGGAGAGGGATTTATTCAAGCTGATCCTCA
AGAAGGAGGACGAGCTGGGCGATCGTTCCATCATGTTCACCGTCCAAAACGA
GGAT
*(Human IL-15R α sushi domain)*

ATTACATGCCCCCCTCCCATGAGCGTGGAGCACGCCGACATCTGGGTG
AAGAGCTATAGCCTCTACAGCCGGGAGAGGTATATCTGTAACAGCGGCTTCA

AGAGGAAGGCCGGCACCAGCAGCCTCACCGAGTGCGTGCTGAATAAGGCTA
CCAACGTGGCTCACTGGACAACACCCTCTTTAAAGTGCATCCGG

The amino acid sequence of an IL-18/IL-15RαSu fusion protein (including signal peptide sequence) is as follows (SEQ ID NO: 37):
(Signal peptide)
   MKWVTFISLLFLFSSAYS
(Human IL-18) 
YFGKLESKLSVIRNLNDQVLFIDQGNRPLFEDMTDSDCRDNAPRTIFIISM
YKDSQPRGMAVTISVKCEKISTLSCENKIISFKEMNPPDNIKDTKSDIIFFQRSVPG
HDNKMQFESSSYEGYFLACEKERDLFKLILKKEDELGDRSIMFTVQNED
(Human IL-15R α sushi domain)

ITCPPPMSVEHADIWVKSYSLYSRERYICNSGFKRKAGTSSLTECVLNKAT
NVAHWTTPSLKCIR

The nucleic acid sequence of an IL-12/TF/IL-15 construct (including leader sequence) is as follows (SEQ ID NO: 38):
*(Signal peptide)* 
ATGAAATGGGTGACCTTTATTTCTTTACTGTTCCTCTTTAGCAGCGCCT
ACTCC
*(Human IL-12 subunit beta (p40))* 
ATTTGGGAACTGAAGAAGGACGTCTACGTGGTCGAACTGGACTGGTAT
CCCGATGCTCCCGGCGAAATGGTGGTGCTCACTTGTGACACCCCCGAAGAAG
ACGGCATCACTTGGACCCTCGATCAGAGCAGCGAGGTGCTGGGCTCCGGAAA
GACCCTCACAATCCAAGTTAAGGAGTTCGGAGACGCTGGCCAATACACATGC
CACAAGGGAGGCGAGGTGCTCAGCCATTCCTTATTATTACACAAGAAGG
AAGACGGAATCTGGTCCACCGACATTTTAAAAGATCAGAAGGAGCCCAAGA
ATAAGACCTTTTTAAGGTGTGAGGCCAAAAACTACAGCGGTCGTTTCACTTGT
TGGTGGCTGACCACCATTTCCACCGATTTAACCTTCTCCGTGAAAAGCAGCCG
GGGAAGCTCCGACCCTCAAGGTGTGACATGTGGAGCCGCTACCCTCAGCGCT
GAGAGGGTTCGTGGCGATAACAAGGAATACGAGTACAGCGTGGAGTGCCAA
 
GAAGATAGCGCTTGTCCCGCTGCCGAAGAATCTTTACCCATTGAGGTGATGG
TGGACGCCGTGCACAAACTCAAGTACGAGAACTACACCTCCTCCTTCTTTATC
CGGGACATCATTAAGCCCGATCCTCCTAAGAATTTACAGCTGAAGCCTCTCA
AAAATAGCCGGCAAGTTGAGGTCTCTTGGGAATATCCCGACACTTGGAGCAC
ACCCCACAGCTACTTCTCTTTAACCTTTTGTGTGCAAGTTCAAGGTAAAAGCA
AGCGGGAGAAGAAAGACCGGGTGTTTACCGACAAAACCAGCGCCACCGTCA
TCTGTCGGAAGAACGCCTCCATCAGCGTGAGGGCTCAAGATCGTTATTACTCC
AGCAGCTGGTCCGAGTGGGCCAGCGTGCCTTGTTCC
*(Linker)*
   GGCGGTGGAGGATCCGGAGGAGGTGGCTCCGGCGGCGGAGGATCT
*(Human IL-12 subunit alpha (p35))* 
CGTAACCTCCCCGTGGCTACCCCCGATCCCGGAATGTTCCCTTGTTTAC
ACCACAGCCAGAATTTACTGAGGGCCGTGAGCAACATGCTGCAGAAAGCTAG
GCAGACTTTAGAATTTTACCCTTGCACCAGCGAGGAGATCGACCATGAAGAT
ATCACCAAGGACAAGACATCCACCGTGGAGGCTTGTTTACCTCTGGAGCTGA
CAAAGAACGAGTCTTGTCTCAACTCTCGTGAAACCAGCTTCATCACAAATGG
CTCTTGTTTAGCTTCCCGGAAGACCTCCTTTATGATGGCTTTATGCCTCAGCTC
CATCTACGAGGATTTAAAGATGTACCAAGTGGAGTTCAAGACCATGAACGCC
AAGCTGCTCATGGACCCTAAACGGCAGATCTTTTTAGACCAGAACATGCTGG
CTGTGATTGATGAGCTGATGCAAGCTTTAAACTTCAACTCCGAGACCGTCCCT
CAGAAGTCCTCCCTCGAGGAGCCCGATTTTTACAAGACAAAGATCAAACTGT
GCATTTTACTCCACGCCTTTAGGATCCGGGCCGTGACCATTGACCGGGTCATG
AGCTATTTAAACGCCAGC
*(Human Tissue Factor 219)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
 
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of an IL-12/TF/IL-15 fusion protein (including leader sequence) is as follows (SEQ ID NO: 39):
*(Signal peptide)*
   MKWVTFISLLFLFSSAYS
*(Human IL-12 subunit beta (p40))* 
IWELKKDVYVVELDWYPDAPGEMVVLTCDTPEEDGITWTLDQSSEVLGS
GKTLTIQVKEFGDAGQYTCHKGGEVLSHSLLLLHKKEDGIWSTDILKDQKEPKN
KTFLRCEAKNYSGRFTCWWLTTISTDLTFSVKSSRGSSDPQGVTCGAATLSAERV
RGDNKEYEYSVECQEDSACPAAEESLPIEVMVDAVHKLKYENYTSSFFIRDIIKPD
PPKNLQLKPLKNSRQVEVSWEYPDTWSTPHSYFSLTFCVQVQGKSKREKKDRVF
TDKTSATVICRKNASISVRAQDRYYSSSWSEWASVPCS
*(Linker)*
   GGGGSGGGGSGGGGS
*(Human IL-12 subunit alpha (p35))* 
RNLPVATPDPGMFPCLHHSQNLLRAVSNMLQKARQTLEFYPCTSEEIDHE
DITKDKTSTVEACLPLELTKNESCLNSRETSFITNGSCLASRKTSFMMALCLSSIYE
DLKMYQVEFKTMNAKLLMDPKRQIFLDQNMLAVIDELMQALNFNSETVPQKSS
LEEPDFYKTKIKLCILLHAFRIRAVTIDRVMSYLNAS
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequences of a TGFβRII/IL-15RαSu construct (including signal sequence) is as follows (SEQ ID NO: 40):
*(Signal peptide)* 
ATGAAGTGGGTGACCTTCATCAGCCTGCTGTTCCTGTTCTCCAGCGCCT
ACTCC
*(Human TGFβRII-1^{st}fragment*) 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCACGATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGAT
*(Linker)*
   GGAGGTGGCGGATCCGGAGGTGGAGGTTCTGGTGGAGGTGGGAGT
*(Human TGFβRII-2^{nd}fragment*) 
ATTCCTCCCCACGTGCAGAAGAGCGTGAATAATGACATGATCGTGACC
GATAACAATGGCGCCGTGAAATTTCCCCAGCTGTGCAAATTCTGCGATGTGA
GGTTTTCCACCTGCGACAACCAGAAGTCCTGTATGAGCAACTGCACAATCAC
CTCCATCTGTGAGAAGCCTCAGGAGGTGTGCGTGGCTGTCTGGCGGAAGAAT
GACGAGAATATCACCCTGGAAACCGTCTGCCACGATCCCAAGCTGCCCTACC
ACGATTTCATCCTGGAAGACGCCGCCAGCCCTAAGTGCATCATGAAAGAGAA
AAAGAAGCCTGGCGAGACCTTTTTCATGTGCTCCTGCAGCAGCGACGAATGC
AACGACAATATCATCTTTAGCGAGGAATACAATACCAGCAACCCCGAC
*(Human IL-15R α sushi domain*)

ATCACGTGTCCTCCTCCTATGTCCGTGGAACACGCAGACATCTGGGTC
AAGAGCTACAGCTTGTACTCCAGGGAGCGGTACATTTGTAACTCTGGTTTCAA
GCGTAAAGCCGGCACGTCCAGCCTGACGGAGTGCGTGTTGAACAAGGCCACG
AATGTCGCCCACTGGACAACCCCCAGTCTCAAATGTATTAGA

The amino acid sequence of a TGFβRII/IL-15RαSu construct (including signal peptide sequence) is as follows (SEQ ID NO: 41):
*(Signal peptide)*
   MKWVTFISLLFLFSSAYS
*(Human TGFβRII-1^{st}fragment)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPD
*(Linker)* GGGGSGGGGSGGGGS
*(Human TGFβRII-2^{nd}fragment*) 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPD
*(Human IL-15R α sushi domain)*

ITCPPPMSVEHADIWVKSYSLYSRERYICNSGFKRKAGTSSLTECVLNKAT
NVAHWTTPSLKCIR

The nucleic acid sequence of the IL-21/TF/IL-15 construct (including leader sequence) is as follows (SEQ ID NO: 42):
*(Signal peptide)* 
ATGAAGTGGGTGACCTTCATCAGCCTGCTGTTCCTGTTCTCCAGCGCCT
ACTCC
*(Human IL-21)* 
CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC
*(Human Tissue Factor 219)* 
TCCGGCACCACCAATACCGTGGCCGCTTATAACCTCACATGGAAGAGC
ACCAACTTCAAGACAATTCTGGAATGGGAACCCAAGCCCGTCAATCAAGTTT
ACACCGTGCAGATCTCCACCAAATCCGGAGACTGGAAGAGCAAGTGCTTCTA
CACAACAGACACCGAGTGTGATTTAACCGACGAAATCGTCAAGGACGTCAAG
CAAACCTATCTGGCTCGGGTCTTTTCCTACCCCGCTGGCAATGTCGAGTCCAC
CGGCTCCGCTGGCGAGCCTCTCTACGAGAATTCCCCCGAATTCACCCCTTATT
TAGAGACCAATTTAGGCCAGCCTACCATCCAGAGCTTCGAGCAAGTTGGCAC
CAAGGTGAACGTCACCGTCGAGGATGAAAGGACTTTAGTGCGGCGGAATAAC
ACATTTTTATCCCTCCGGGATGTGTTCGGCAAAGACCTCATCTACACACTGTA
CTATTGGAAGTCCAGCTCCTCCGGCAAAAAGACCGCTAAGACCAACACCAAC
GAGTTTTTAATTGACGTGGACAAAGGCGAGAACTACTGCTTCAGCGTGCAAG
 
CCGTGATCCCTTCTCGTACCGTCAACCGGAAGAGCACAGATTCCCCCGTTGA
GTGCATGGGCCAAGAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of the mature IL-21/TF/IL-15 fusion protein (including signal peptide sequence) is as follows (SEQ ID NO: 43):
*(Signal peptide)*
   MKWVTFISLLFLFSSAYS
*(Human IL-21)*
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of the IL-21/TF mutant/IL-15 construct (including signal peptide sequence) is as follows (SEQ ID NO: 44, bold nucleotides are mutant and the mutant codons are underlined):
*(Signal sequence)* 
ATGAAGTGGGTGACCTTCATCAGCCTGCTGTTCCTGTTCTCCAGCGCCT
ACTCC
*(Human IL-21)* 
CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC
*(Human Tissue Factor 219 mutants)* 
TCCGGCACCACCAATACCGTGGCCGCTTATAACCTCACATGGAAGAGC
ACCAACTTCGCGACAGCTCTGGAATGGGAACCCAAGCCCGTCAATCAAGTTT
ACACCGTGCAGATCTCCACCAAATCCGGAGACTGGAAGAGCAAGTGCTTCTA
CACAACAGACACCGAGTGTGCTTTAACCGACGAAATCGTCAAGGACGTCAAG
CAAACCTATCTGGCTCGGGTCTTTTCCTACCCCGCTGGCAATGTCGAGTCCAC
CGGCTCCGCTGGCGAGCCTCTCTACGAGAATTCCCCCGAATTCACCCCTTATT
TAGAGACCAATTTAGGCCAGCCTACCATCCAGAGCTTCGAGCAAGTTGGCAC
CAAGGTGAACGTCACCGTCGAGGATGAAAGGACTTTAGTGGCGCGGAATAA
CACAGCTTTATCCCTCCGGGATGTGTTCGGCAAAGACCTCATCTACACACTGT
ACTATTGGAAGTCCAGCTCCTCCGGCAAAAAGACCGCTAAGACCAACACCAA
CGAGTTTTTAATTGACGTGGACAAAGGCGAGAACTACTGCTTCAGCGTGCAA
GCCGTGATCCCTTCTCGTACCGTCAACCGGAAGAGCACAGATTCCCCCGTTG
AGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of the IL-21/TF mutant/IL-15 construct (including signal peptide sequence) is as follows (SEQ ID NO: 45, substituted residues are shown in bold and underlined):
*(Signal peptide)*
   MKWVTFISLLFLFSSAYS
*(Human IL-21)*
*(Human Tissue Factor 219)* 
SGTTNTVAAYNLTWKSTNFATALEWEPKPVNQVYTVQISTKSGDWKSKC
FYTTDTECALTDEIVKDVKQTYLARVFSYPAGNVESTGSAGEPLYENSPEFTPYL
ETNLGQPTIQSFEQVGTKVNVTVEDERTLVARNNTALSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of the IL-21/IL-15RαSu construct (including signal sequence) is as follows (SEQ ID NO: 46):
*(Signal sequence)* 
ATGAAGTGGGTGACCTTCATCAGCCTGCTGTTCCTGTTCTCCAGCGCCT
ACTCC
*(Human IL-21)* 
CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC
*(Human IL-15R α sushi domain)*

ATTACATGCCCCCCTCCCATGAGCGTGGAGCACGCCGACATCTGGGTG
AAGAGCTATAGCCTCTACAGCCGGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG

The amino acid sequence of the IL-21/IL-15RαSu construct (including signal peptide sequence) is as follows (SEQ ID NO: 101):
*(Signal peptide)*
   MKWVTFISLLFLFSSAYS
*(Human IL-21)*
*(Human IL-15R α sushi domain)*

ITCPPPMSVEHADIWVKSYSLYSRERYICNSGFKRKAGTSSLTECVLNKAT
NVAHWTTPSLKCIR

The nucleic acid sequence of the TGFβRII/TF/IL-15 construct (including leader sequence) is as follows (SEQ ID NO: 47):
*(Signal peptide)* 
ATGAAGTGGGTGACCTTCATCAGCCTGCTGTTCCTGTTCTCCAGCGCCT
ACTCC
*(Human TGFβRII-1^{st}fragment*) 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCACGATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGAT
*(Linker)*
   GGAGGTGGCGGATCCGGAGGTGGAGGTTCTGGTGGAGGTGGGAGT
*(Human TGFβRII-2^{nd}fragment*) 
ATTCCTCCCCACGTGCAGAAGAGCGTGAATAATGACATGATCGTGACC
GATAACAATGGCGCCGTGAAATTTCCCCAGCTGTGCAAATTCTGCGATGTGA
GGTTTTCCACCTGCGACAACCAGAAGTCCTGTATGAGCAACTGCACAATCAC
CTCCATCTGTGAGAAGCCTCAGGAGGTGTGCGTGGCTGTCTGGCGGAAGAAT
GACGAGAATATCACCCTGGAAACCGTCTGCCACGATCCCAAGCTGCCCTACC
ACGATTTCATCCTGGAAGACGCCGCCAGCCCTAAGTGCATCATGAAAGAGAA
AAAGAAGCCTGGCGAGACCTTTTTCATGTGCTCCTGCAGCAGCGACGAATGC
AACGACAATATCATCTTTAGCGAGGAATACAATACCAGCAACCCCGAC
*(Human Tissue Factor 219)* 
TCCGGCACCACCAATACCGTGGCCGCTTATAACCTCACATGGAAGAGC
ACCAACTTCAAGACAATTCTGGAATGGGAACCCAAGCCCGTCAATCAAGTTT
ACACCGTGCAGATCTCCACCAAATCCGGAGACTGGAAGAGCAAGTGCTTCTA
CACAACAGACACCGAGTGTGATTTAACCGACGAAATCGTCAAGGACGTCAAG
 
CAAACCTATCTGGCTCGGGTCTTTCCTACCCCGCTGGCAATGTCGAGTCCAC
CGGCTCCGCTGGCGAGCCTCTCTACGAGAATTCCCCCGAATTCACCCCTTATT
TAGAGACCAATTTAGGCCAGCCTACCATCCAGAGCTTCGAGCAAGTTGGCAC
CAAGGTGAACGTCACCGTCGAGGATGAAAGGACTTTAGTGCGGCGGAATAAC
ACATTTTTATCCCTCCGGGATGTGTTCGGCAAAGACCTCATCTACACACTGTA
CTATTGGAAGTCCAGCTCCTCCGGCAAAAAGACCGCTAAGACCAACACCAAC
GAGTTTTTAATTGACGTGGACAAAGGCGAGAACTACTGCTTCAGCGTGCAAG
CCGTGATCCCTTCTCGTACCGTCAACCGGAAGAGCACAGATTCCCCCGTTGA
GTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of the TGFβRII/TF/IL-15 fusion protein (including signal peptide) is as follows (SEQ ID NO: 48):
*(Signal peptide)*
   MKWVTFISLLFLFSSAYS
*(Human TGFβRII-1^{st}fragment*) 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPD
*(Linker)*
   GGGGSGGGGSGGGGS
*(Human TGFβRII-2^{nd}fragment)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPD
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence encoding the second chimeric polypeptide of IL-7/IL-15RαSu construct (including signal peptide sequence) is as follows (SEQ ID NO: 49):
*(Signal peptide)* 
ATGGGAGTGAAAGTTCTTTTTGCCCTTATTTGTATTGCTGTGGCCGAGG
CC
*(Human IL-7)* 
GATTGTGATATTGAAGGTAAAGATGGCAAACAATATGAGAGTGTTCTA
ATGGTCAGCATCGATCAATTATTGGACAGCATGAAAGAAATTGGTAGCAATT
GCCTGAATAATGAATTTAACTTTTTAAAAGACATATCTGTGATGCTAATAAG
GAAGGTATGTTTTATTCCGTGCTGCTCGCAAGTTGAGGCAATTTCTTAAAAT
GAATAGCACTGGTGATTTTGATCTCCACTTATTAAAAGTTTCAGAAGGCACAA
CAATACTGTTGAACTGCACTGGCCAGGTTAAAGGAAGAAAACCAGCTGCCCT
GGGTGAAGCCCAACCAACAAAGAGTTTGGAAGAAAATAAATCTTTAAAGGA
ACAGAAAAAACTGAATGACTTGTGTTTCCTAAAGAGACTATTACAAGAGATA
AAAACTTGTTGGAATAAAATTTTGATGGGCACTAAAGAACAC
*(Human IL-15R α sushi domain)*

ATCACGTGCCCTCCCCCCATGTCCGTGGAACACGCAGACATCTGGGTC
AAGAGCTACAGCTTGTACTCCAGGGAGCGGTACATTTGTAACTCTGGTTTCAA
GCGTAAAGCCGGCACGTCCAGCCTGACGGAGTGCGTGTTGAACAAGGCCACG
AATGTCGCCCACTGGACAACCCCCAGTCTCAAATGCATTAGA

The second chimeric polypeptide of IL-7/IL-15RαSu construct (including signal peptide sequence) is as follows (SEQ ID NO: 50):
(Signal peptide)
   MKWVTFISLLFLFSSAYS
(Human IL-7) 
DCDIEGKDGKQYESVLMVSIDQLLDSMKEIGSNCLNNEFNFFKRHICDAN
KEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEH
(Human Tissue Factor 219) 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
(Human IL-15)

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence encoding the first chimeric polypeptide of IL-21/TF/IL-15 construct (including leader sequence), synthesized by Genewiz, is as follows (SEQ ID NO: 51):
*(Signal peptide)* 
ATGGGAGTGAAAGTTCTTTTTGCCCTTATTTGTATTGCTGTGGCCGAGG
CC
*(Human IL-21 fragment*) 
CAAGGTCAAGATCGCCACATGATTAGAATGCGTCAACTTATAGATATT
GTTGATCAGCTGAAAAATTATGTGAATGACTTGGTCCCTGAATTTCTGCCAGC
TCCAGAAGATGTAGAGACAAACTGTGAGTGGTCAGCTTTTTCCTGTTTTCAGA
AGGCCCAACTAAAGTCAGCAAATACAGGAAACAATGAAAGGATAATCAATG
TATCAATTAAAAAGCTGAAGAGGAAACCACCTTCCACAAATGCAGGGAGAA
GACAGAAACACAGACTAACATGCCCTTCATGTGATTCTTATGAGAAAAAACC
ACCCAAAGAATTCCTAGAAAGATTCAAATCACTTCTCCAAAAGATGATTCAT
CAGCATCTGTCCTCTAGAACACACGGAAGTGAAGATTCC
*(Human Tissue Factor 219)* 
TCAGGCACTACAAATACTGTGGCAGCATATAATTTAACTTGGAAATCA
ACTAATTTCAAGACAATTTTGGAGTGGAACCCAAACCCGTCAATCAAGTCT
ACACTGTTCAAATAAGCACTAAGTCAGGAGATTGGAAAAGCAAATGCTTTTA
CACAACAGACACAGAGTGTGACCTCACCGACGAGATTGTGAAGGATGTGAA
GCAGACGTACTTGGCACGGGTCTTCTCCTACCCGGCAGGGAATGTGGAGAGC
ACCGGTTCTGCTGGGGAGCCTCTGTATGAGAACTCCCCAGAGTTCACACCTTA
CCTGGAGACAAACCTCGGACAGCCAACAATTCAGAGTTTTGAACAGGTGGGA
ACAAAAGTGAATGTGACCGTAGAAGATGAACGGACTTTAGTCAGAAGGAAC
AACACTTTCCTAAGCCTCCGGGATGTTTTTGGCAAGGACTTAATTTATACACT
TTATTATTGGAAATCTTCAAGTTCAGGAAAGAAAACAGCCAAAACAAACACT
AATGAGTTTTTGATTGATGTGGATAAAGGAGAAAACTACTGTTTCAGTGTTCA
AGCAGTGATTCCCTCCCGAACAGTTAACCGGAAGAGTACAGACAGCCCGGTA
GAGTGTATGGGCCAGGAGAAAGGGGAATTCAGAGAA
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG

GAGTGCGAAGAGCTGGAGGAGAAGAACATCAAGGAGTTTCTGCAATCCTTTG
TGCACATTGTCCAGATGTTCATCAATACCTCC

The first chimeric polypeptide of IL-21/TF/IL-15 construct including leader sequence is as follows (SEQ ID NO: 52):
*(Signal peptide)*
   MGVKVLFALICIAVAEA
*(Human IL-21)*
*(Human Tissue Factor 219)* 
SGTTNTVAAYNLTWKSTNFKTILEWEPKPVNQVYTVQISTKSGDWKSKCFYTTD
TECDLTDEIVKDVKQTYLARVFSYPAGNVESTGSAGEPLYENSPEFTPYLETNLG
QPTIQSFEQVGTKVNVTVEDERTLVRRNNTFLSLRDVFGKDLIYTLYYWKSSSSG
KKTAKTNTNEFLIDVDKGENYCFSVQAVIPSRTVNRKSTDSPVECMGQEKGEFR
E
*(Human IL-15)*

NWVNVISDLKKIEDLIQSMHIDATLYTESDVHPSCKVTAMKCFLLELQVISLESG
DASIHDTVENLIILANNSLSSNGNVTESGCKECEELEEKNIKEFLQSFVHIVQMFIN
TS

The nucleic acid sequence encoding the second chimeric polypeptide of IL-21/IL-15RαSu domain (including leader sequence), synthesized by Genewiz, is as follows (SEQ ID NO: 53):
*(Signal peptide)* 
ATGAAGTGGGTGACCTTCATCAGCCTGCTGTTCCTGTTCTCCAGCGCCTACTC
C
*(Human IL-21)* 
CAGGGCCAGGACAGGCACATGATCCGGATGAGGCAGCTCATCGACATCGTCG
ACCAGCTGAAGAACTACGTGAACGACCTGGTGCCCGAGTTTCTGCCTGCCCC
CGAGGACGTGGAGACCAACTGCGAGTGGTCCGCCTTCTCCTGCTTTCAGAAG
GCCCAGCTGAAGTCCGCCAACACCGGCAACAACGAGCGGATCATCAACGTG
AGCATCAAGAAGCTGAAGCGGAAGCCTCCCTCCACAAACGCCGGCAGGAGG
CAGAAGCACAGGCTGACCTGCCCCAGCTGTGACTCCTACGAGAAGAAGCCCC
CCAAGGAGTTCCTGGAGAGGTTCAAGTCCCTGCTGCAGAAGATGATCCATCA
GCACCTGTCCTCCAGGACCCACGGCTCCGAGGACTCC
*(Human IL-15R α sushi domain*)

ATTACATGCCCCCCTCCCATGAGCGTGGAGCACGCCGACATCTGGGTGAAGA
GCTATAGCCTCTACAGCCGGGAGAGGTATATCTGTAACAGCGGCTTCAAGAG
GAAGGCCGGCACCAGCAGCCTCACCGAGTGCGTGCTGAATAAGGCTACCAAC
GTGGCTCACTGGACAACACCCTCTTTAAAGTGCATCCGG

The second chimeric polypeptide of IL-21/IL-15Rα sushi domain (including leader sequence) is as follows (SEQ ID NO: 54):
*(Signal Sequence)*
   MKWVTFISLLFLFSSAYS
*(Human IL-21)*
*(Human IL-15Rα sushi domain)*

ITCPPPMSVEHADIWVKSYSLYSRERYICNSGFKRKAGTSSLTECVLNKATNVAH
WTTPSLKCIR

The nucleic acid sequence encoding the first chimeric polypeptide of IL-7/TF/IL-15 is as follows (SEQ ID NO: 55):
*(Signal peptide)* 
ATGAAGTGGGTGACCTTCATCAGCCTGCTGTTCCTGTTCTCCAGCGCCTACTC
C
*(Human IL-7fragment*) 
GATTGCGACATCGAGGGCAAGGACGGCAAGCAGTACGAGAGCGTGCTGATG
GTGTCCATCGACCAGCTGCTGGACAGCATGAAGGAGATCGGCTCCAACTGCC
TCAACAACGAGTTCAACTTCTTCAAGCGGCACATCTGCGACGCCAACAAGGA
GGGCATGTTCCTGTTCAGGGCCGCCAGGAAACTGCGGCAGTTCCTGAAGATG
AACTCCACCGGCGACTTCGACCTGCACCTGCTGAAGGTGTCCGAGGGCACCA
CCATCCTGCTGAACTGCACCGGACAGGTGAAGGGCCGGAAACCTGCTGCTCT
GGGAGAGGCCCAACCCACCAAGAGCCTGGAGGAGAACAAGTCCCTGAAGGA
GCAGAAGAAGCTGAACGACCTGTGCTTCCTGAAGAGGCTGCTGCAGGAGATC
AAGACCTGCTGGAACAAGATCCTGATGGGCACCAAGGAGCAT
*(Human Tissue Factor 219)* 
AGCGGCACAACCAACACAGTCGCTGCCTATAACCTCACTTGGAAGAGCACCA
ACTTCAAAACCATCCTCGAATGGGAACCCAAACCCGTTAACCAAGTTTACAC
CGTGCAGATCAGCACCAAGTCCGGCGACTGGAAGTCCAAATGTTTCTATACC
ACCGACACCGAGTGCGATCTCACCGATGAGATCGTGAAAGATGTGAAACAGA
CCTACCTCGCCCGGGTGTTTAGCTACCCCGCCGGCAATGTGGAGAGCACTGG
TTCCGCTGGCGAGCCTTTATACGAGAACAGCCCCGAATTTACCCCTTACCTCG
AGACCAATTTAGGACAGCCCACCATCCAAAGCTTTGAGCAAGTTGGCACAAA
GGTGAATGTGACAGTGGAGGACGAGCGGACTTTAGTGCGGCGGAACAACAC
CTTTCTCAGCCTCCGGGATGTGTTCGGCAAAGATTTAATCTACACACTGTATT
ACTGGAAGTCCTCTTCCTCCGGCAAGAAGACAGCTAAAACCAACACAAACGA
GTTTTTAATCGACGTGGATAAAGGCGAAAACTACTGTTTCAGCGTGCAAGCT
GTGATCCCCTCCCGGACCGTGAATAGGAAAAGCACCGATAGCCCCGTTGAGT
GCATGGGCCAAGAAAAGGGCGAGTTCCGGGAG
*(Human IL-15)*

AACTGGGTGAACGTCATCAGCGATTTAAAGAAGATCGAAGATTTAATTCAGT
CCATGCATATCGACGCCACTTTATACACAGAATCCGACGTGCACCCCTCTTGT
AAGGTGACCGCCATGAAATGTTTTTACTGGAGCTGCAAGTTATCTCTTTAGA

GAGCGGAGACGCTAGCATCCACGACACCGTGGAGAATTTAATCATTTTAGCC
AATAACTCTTTATCCAGCAACGGCAACGTGACAGAGTCCGGCTGCAAGGAGT
GCGAAGAGCTGGAGGAGAAGAACATCAAGGAGTTTCTGCAATCCTTTGTGCA
CATTGTCCAGATGTTCATCAATACCTCC

The chimeric polypeptide of IL-7/TF/IL-15 (including leader sequence), is as follows (SEQ ID NO: 56):
*(Signal peptide)*
   MKWVTFISLLFLFSSAYS
*(Human IL-7)* 
DCDIEGKDGKQYESVLMVSIDQLLDSMKEIGSNCLNNEFNFFKRHICDANKEGM
FLFRAARKLRQFLKMNSTGDFDLHLLKVSEGTTILLNCTGQVKGRKPAALGEAQ
PTKSLEENKSLKEQKKLNDLCFLKRLLQEIKTCWNKILMGTKEH
*(Human Tissue Factor 219)* 
SGTTNTVAAYNLTWKSTNFKTILEWEPKPVNQVYTVQISTKSGDWKSKCFYTTD
TECDLTDEIVKDVKQTYLARVFSYPAGNVESTGSAGEPLYENSPEFTPYLETNLG
QPTIQSFEQVGTKVNVTVEDERTLVRRNNTFLSLRDVFGKDLIYTLYYWKSSSSG
KKTAKTNTNEFLIDVDKGENYCFSVQAVIPSRTVNRKSTDSPVECMGQEKGEFR
E
*(Human IL-15)*

NWVNVISDLKKIEDLIQSMHIDATLYTESDVHPSCKVTAMKCFLLELQVISLESG
DASIHDTVENLIILANNSLSSNGNVTESGCKECEELEEKNIKEFLQSFVHIVQMFIN
TS

The nucleic acid sequence of an IL-7/TF/IL-15 construct (including signal peptide sequence) is as follows (SEQ ID NO: 57):
*(Signal peptide)* 
ATGAAGTGGGTGACCTTCATCAGCCTGCTGTTCCTGTTCTCCAGCGCCT
ACTCC
*(Human IL-7)* 
GATTGCGACATCGAGGGCAAGGACGGCAAGCAGTACGAGAGCGTGCT
GATGGTGTCCATCGACCAGCTGCTGGACAGCATGAAGGAGATCGGCTCCAAC
TGCCTCAACAACGAGTTCAACTTCTTCAAGCGGCACATCTGCGACGCCAACA
AGGAGGGCATGTTCCTGTTCAGGGCCGCCAGGAAACTGCGGCAGTTCCTGAA
GATGAACTCCACCGGCGACTTCGACCTGCACCTGCTGAAGGTGTCCGAGGGC
ACCACCATCCTGCTGAACTGCACCGGACAGGTGAAGGGCCGGAAACCTGCTG
CTCTGGGAGAGGCCCAACCCACCAAGAGCCTGGAGGAGAACAAGTCCCTGA
AGGAGCAGAAGAAGCTGAACGACCTGTGCTTCCTGAAGAGGCTGCTGCAGG
AGATCAAGACCTGCTGGAACAAGATCCTGATGGGCACCAAGGAGCAT
*(Human Tissue Factor 219)* 
AGCGGCACAACCAACACAGTCGCTGCCTATAACCTCACTTGGAAGAG
CACCAACTTCAAAACCATCCTCGAATGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG

GAGTGCGAAGAGCTGGAGGAGAAGAACATCAAGGAGTTTCTGCAATCCTTTG
TGCACATTGTCCAGATGTTCATCAATACCTCC

The amino acid sequence of IL-7/TF/IL-15 fusion protein (including the leader sequence) is as follows (SEQ ID NO: 58):
*(Signal peptide)*
   MKWVTFISLLFLFSSAYS
*(Human IL-7)* 
DCDIEGKDGKQYESVLMVSIDQLLDSMKEIGSNCLNNEFNFFKRHICDAN
KEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEH
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of an anti-CD16SscFv/IL-15 RαSu/IL-21 construct (including signal peptide sequence) is as follows (SEQ ID NO: 59):
*(Signal peptide)* 
ATGAAGTGGGTGACCTTCATCAGCCTGCTGTTCCTGTTCTCCAGCGCCT
ACTCC
*((Anti-human CD16scFv)* 
TCCGAGCTGACCCAGGACCCTGCTGTGTCCGTGGCTCTGGGCCAGACC
GTGAGGATCACCTGCCAGGGCGACTCCCTGAGGTCCTACTACGCCTCCTGGT
 
ACCAGCAGAAGCCCGGCCAGGCTCCTGTGCTGGTGATCTACGGCAAGAACAA
CAGGCCCTCCGGCATCCCTGACAGGTTCTCCGGATCCTCCTCCGGCAACACCG
CCTCCCTGACCATCACAGGCGCTCAGGCCGAGGACGAGGCTGACTACTACTG
CAACTCCAGGGACTCCTCCGGCAACCATGTGGTGTTCGGCGGCGGCACCAAG
CTGACCGTGGGCCATGGCGGCGGCGGCTCCGGAGGCGGCGGCAGCGGCGGA
GGAGGATCCGAGGTGCAGCTGGTGGAGTCCGGAGGAGGAGTGGTGAGGCCT
GGAGGCTCCCTGAGGCTGAGCTGTGCTGCCTCCGGCTTCACCTTCGACGACTA
CGGCATGTCCTGGGTGAGGCAGGCTCCTGGAAAGGGCCTGGAGTGGGTGTCC
GGCATCAACTGGAACGGCGGATCCACCGGCTACGCCGATTCCGTGAAGGGCA
GGTTCACCATCAGCAGGGACAACGCCAAGAACTCCCTGTACCTGCAGATGAA
CTCCCTGAGGGCCGAGGACACCGCCGTGTACTACTGCGCCAGGGGCAGGTCC
CTGCTGTTCGACTACTGGGGACAGGGCACCCTGGTGACCGTGTCCAGG
*(Human IL-15R α sushi domain)* 
ATTACATGCCCCCCTCCCATGAGCGTGGAGCACGCCGACATCTGGGTG
AAGAGCTATAGCCTCTACAGCCGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*(Human IL-21)*

CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC

The amino acid sequence of the anti-CD16scFv/IL-15RαSu/IL-21 construct (including signal peptide sequence) is as follows (SEQ ID NO: 60):
*(Signal peptide)*
   MKWVTFISLLFLFSSAYS
*(Anti-human CD16scFv)* 
SELTQDPAVSVALGQTVRITCQGDSLRSYYASWYQQKPGQAPVLVIYGK
NNRPSGIPDRFSGSSSGNTASLTITGAQAEDEADYYCNSRDSSGNHVVFGGGTKL
TVGHGGGGSGGGGSGGGGSEVQLVESGGGVVRPGGSLRLSCAASGFTFDDYGM
SWVRQAPGKGLEWVSGINWNGGSTGYADSVKGRFTISRDNAKNSLYLQMNSLR
AEDTAVYYCARGRSLLFDYWGQGTLVTVSR
*(Human IL-15R α sushi domain)* 
ITCPPPMSVEHADIWVKSYSLYSRERYICNSGFKRKAGTSSLTECVLNKAT
NVAHWTTPSLKCIR
*(Human IL-21)*

The nucleic acid sequence of the two TGFβ Receptor II/TF/IL-15 construct (including signal peptide sequence) is as follows (SEQ ID NO: 61):
*(Signal peptide)* 
ATGAAGTGGGTGACCTTCATCAGCCTGCTGTTCCTGTTCTCCAGCGCCT
ACTCC
*(Two Human TGFβ Receptor II fragments)* 
ATCCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
 
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human Tissue Factor 219)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of TGFβ Receptor II/TF/IL-15 fusion protein (including the leader sequence) is as follows (SEQ ID NO: 62):
*(Signal peptide)*
   MKWVTFISLLFLFSSAYS
*(Human TGFβ Receptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of the anti-CD16scFv/IL-15 RαSu/IL-21 construct (including signal peptide sequence) is as follows (SEQ ID NO: 63):
*(Signal peptide)* 
ATGAAGTGGGTGACCTTCATCAGCCTGCTGTTCCTGTTCTCCAGCGCCT
ACTCC
*(Anti-human CD16scFv)* 
TCCGAGCTGACCCAGGACCCTGCTGTGTCCGTGGCTCTGGGCCAGACC
GTGAGGATCACCTGCCAGGGCGACTCCCTGAGGTCCTACTACGCCTCCTGGT
 
ACCAGCAGAAGCCCGGCCAGGCTCCTGTGCTGGTGATCTACGGCAAGAACAA
CAGGCCCTCCGGCATCCCTGACAGGTTCTCCGGATCCTCCTCCGGCAACACCG
CCTCCCTGACCATCACAGGCGCTCAGGCCGAGGACGAGGCTGACTACTACTG
CAACTCCAGGGACTCCTCCGGCAACCATGTGGTGTTCGGCGGCGGCACCAAG
CTGACCGTGGGCCATGGCGGCGGCGGCTCCGGAGGCGGCGGCAGCGGCGGA
GGAGGATCCGAGGTGCAGCTGGTGGAGTCCGGAGGAGGAGTGGTGAGGCCT
GGAGGCTCCCTGAGGCTGAGCTGTGCTGCCTCCGGCTTCACCTTCGACGACTA
CGGCATGTCCTGGGTGAGGCAGGCTCCTGGAAAGGGCCTGGAGTGGGTGTCC
GGCATCAACTGGAACGGCGGATCCACCGGCTACGCCGATTCCGTGAAGGGCA
GGTTCACCATCAGCAGGGACAACGCCAAGAACTCCCTGTACCTGCAGATGAA
CTCCCTGAGGGCCGAGGACACCGCCGTGTACTACTGCGCCAGGGGCAGGTCC
CTGCTGTTCGACTACTGGGGACAGGGCACCCTGGTGACCGTGTCCAGG
*(Human IL-15R a sushi domain)* 
ATTACATGCCCCCCTCCCATGAGCGTGGAGCACGCCGACATCTGGGTG
AAGAGCTATAGCCTCTACAGCCGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*(Human IL-21)*

CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC

The amino acid sequence of the anti-CD16scFv/IL-15RαSu/IL-21 construct (including signal peptide sequence) is as follows (SEQ ID NO: 64):
*(Signal peptide)*
   MKWVTFISLLFLFSSAYS
*(Anti-human CD16scFv)* 
SELTQDPAVSVALGQTVRITCQGDSLRSYYASWYQQKPGQAPVLVIYGK
NNRPSGIPDRFSGSSSGNTASLTITGAQAEDEADYYCNSRDSSGNHVVFGGGTKL
TVGHGGGGSGGGGSGGGGSEVQLVESGGGVVRPGGSLRLSCAASGFTFDDYGM
SWVRQAPGKGLEWVSGINWNGGSTGYADSVKGRFTISRDNAKNSLYLQMNSLR
AEDTAVYYCARGRSLLFDYWGQGTLVTVSR
*(Human IL-15R α sushi domain)* 
ITCPPPMSVEHADIWVKSYSLYSRERYICNSGFKRKAGTSSLTECVLNKAT
NVAHWTTPSLKCIR
*(Human IL-21)*

The nucleic acid sequence of 7t15 construct (including signal peptide sequence) is as follows (SEQ ID NO: 65):
*(Signal peptide)* 
ATGAAGTGGGTGACCTTCATCAGCCTGCTGTTCCTGTTCTCCAGCGCCT
ACTCC
*(Human IL7)* 
GATTGCGACATCGAGGGCAAGGACGGCAAGCAGTACGAGAGCGTGCT
GATGGTGTCCATCGACCAGCTGCTGGACAGCATGAAGGAGATCGGCTCCAAC
TGCCTCAACAACGAGTTCAACTTCTTCAAGCGGCACATCTGCGACGCCAACA
AGGAGGGCATGTTCCTGTTCAGGGCCGCCAGGAAACTGCGGCAGTTCCTGAA
GATGAACTCCACCGGCGACTTCGACCTGCACCTGCTGAAGGTGTCCGAGGGC
ACCACCATCCTGCTGAACTGCACCGGACAGGTGAAGGGCCGGAAACCTGCTG
CTCTGGGAGAGGCCCAACCCACCAAGAGCCTGGAGGAGAACAAGTCCCTGA
AGGAGCAGAAGAAGCTGAACGACCTGTGCTTCCTGAAGAGGCTGCTGCAGG
AGATCAAGACCTGCTGGAACAAGATCCTGATGGGCACCAAGGAGCAT
*(Human Tissue Factor 219)* 
AGCGGCACAACCAACACAGTCGCTGCCTATAACCTCACTTGGAAGAG
CACCAACTTCAAAACCATCCTCGAATGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of 7t15 fusion protein (including the leader sequence) is as follows (SEQ ID NO: 66):
*(Signal peptide)*
   MKWVTFISLLFLFSSAYS
*(Human IL7)* 
DCDIEGKDGKQYESVLMVSIDQLLDSMKEIGSNCLNNEFNFFKRHICDAN
KEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEH
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of 7s construct (including signal peptide sequence) is as follows (SEQ ID NO: 67):
*(Signal peptide)* 
ATGGGAGTGAAAGTTCTTTTTGCCCTTATTTGTATTGCTGTGGCCGAGG
CC
*(Human IL7)* 
GATTGTGATATTGAAGGTAAAGATGGCAAACAATATGAGAGTGTTCTA
ATGGTCAGCATCGATCAATTATTGGACAGCATGAAAGAAATTGGTAGCAATT
GCCTGAATAATGAATTTAACTTTTTAAAAGACATATCTGTGATGCTAATAAG
GAAGGTATGTTTTATTCCGTGCTGCTCGCAAGTTGAGGCAATTTCTTAAAAT
GAATAGCACTGGTGATTTTGATCTCCACTTATTAAAAGTTTCAGAAGGCACAA
CAATACTGTTGAACTGCACTGGCCAGGTTAAAGGAAGAAAACCAGCTGCCCT
GGGTGAAGCCCAACCAACAAAGAGTTTGGAAGAAAATAAATCTTTAAAGGA
ACAGAAAAAACTGAATGACTTGTGTTTCCTAAAGAGACTATTACAAGAGATA
AAAACTTGTTGGAATAAAATTTTGATGGGCACTAAAGAACAC
*(Human IL-15R α sushi domain)*

ATCACGTGCCCTCCCCCCATGTCCGTGGAACACGCAGACATCTGGGTC
AAGAGCTACAGCTTGTACTCCAGGGAGCGGTACATTTGTAACTCTGGTTTCAA
GCGTAAAGCCGGCACGTCCAGCCTGACGGAGTGCGTGTTGAACAAGGCCACG
AATGTCGCCCACTGGACAACCCCCAGTCTCAAATGCATTAGA

The amino acid sequence of 7s fusion protein (including the leader sequence) is as follows (SEQ ID NO: 68):
*(Signal peptide)*
   MGVKVLFALICIAVAEA
*(Human IL7)* 
DCDIEGKDGKQYESVLMVSIDQLLDSMKEIGSNCLNNEFNFFKRHICDAN
KEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEH
*(Human IL-15R α sushi domain)*

ITCPPPMSVEHADIWVKSYSLYSRERYICNSGFKRKAGTSSLTECVLNKAT
NVAHWTTPSLKCIR

The nucleic acid sequence of the two TGFβ Receptor II/TF/IL-15 construct (including signal peptide sequence) is as follows (SEQ ID NO: 69):
*(Signal peptide)* 
ATGAAGTGGGTGACCTTCATCAGCCTGCTGTTCCTGTTCTCCAGCGCCT
ACTCC
*(Two Human TGFβ Receptor II fragments)* 
ATCCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
 
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human Tissue Factor 219)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG

GAGTGCGAAGAGCTGGAGGAGAAGAACATCAAGGAGTTTCTGCAATCCTTTG
TGCACATTGTCCAGATGTTCATCAATACCTCC

The amino acid sequence of TGFβ Receptor II/TF/IL-15 fusion protein (including the leader sequence) is as follows (SEQ ID NO: 70):
*(Signal peptide)*
   MKWVTFISLLFLFSSAYS
*(Human TGFβ Receptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of the TGFβ Receptor II/IL-15 RαSu construct (including signal peptide sequence) is as follows (SEQ ID NO: 71) :
*(Signal peptide)* 
ATGAAGTGGGTGACCTTCATCAGCCTGCTGTTCCTGTTCTCCAGCGCCT
ACTCC
*(Two human TGFβ Receptor II fragments)* 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human IL-15R α sushi domain)*

ATTACATGCCCCCCTCCCATGAGCGTGGAGCACGCCGACATCTGGGTG
AAGAGCTATAGCCTCTACAGCCGGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG

The amino acid sequence of the two TGFβ Receptor II/IL-15RαSu construct (including signal peptide sequence) is as follows (SEQ ID NO: 72):
*(Signal peptide)*
   MKWVTFISLLFLFSSAYS
*(Two human TGFβ Receptor II extra-cellular domains)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human IL-15R α sushi domain)*

ITCPPPMSVEHADIWVKSYSLYSRERYICNSGFKRKAGTSSLTECVLNKAT
NVAHWTTPSLKCIR

The nucleic acid sequence of the 7t15 construct (including signal peptide sequence) is as follows (SEQ ID NO: 73):
*(Signal peptide)* 
ATGAAGTGGGTGACCTTCATCAGCCTGCTGTTCCTGTTCTCCAGCGCCT
ACTCC
*(Human IL7)* 
GATTGCGACATCGAGGGCAAGGACGGCAAGCAGTACGAGAGCGTGCT
GATGGTGTCCATCGACCAGCTGCTGGACAGCATGAAGGAGATCGGCTCCAAC
TGCCTCAACAACGAGTTCAACTTCTTCAAGCGGCACATCTGCGACGCCAACA
AGGAGGGCATGTTCCTGTTCAGGGCCGCCAGGAAACTGCGGCAGTTCCTGAA
GATGAACTCCACCGGCGACTTCGACCTGCACCTGCTGAAGGTGTCCGAGGGC
ACCACCATCCTGCTGAACTGCACCGGACAGGTGAAGGGCCGGAAACCTGCTG
CTCTGGGAGAGGCCCAACCCACCAAGAGCCTGGAGGAGAACAAGTCCCTGA
AGGAGCAGAAGAAGCTGAACGACCTGTGCTTCCTGAAGAGGCTGCTGCAGG
AGATCAAGACCTGCTGGAACAAGATCCTGATGGGCACCAAGGAGCAT
*(Human Tissue Factor 219)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
 
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of 7t15 fusion protein (including the leader sequence) is as follows (SEQ ID NO: 74):
*(Signal peptide)*
   MKWVTFISLLFLFSSAYS
*(Human IL7)* 
DCDIEGKDGKQYESVLMVSIDQLLDSMKEIGSNCLNNEFNFFKRHICDAN
KEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEH
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
 
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid and protein sequences of the 21s137L are shown below. The nucleic acid sequence of the 21s137L construct (including signal peptide sequence) is as follows (SEQ ID NO: 75):
*(Signal peptide)* 
ATGAAGTGGGTGACCTTCATCAGCCTGCTGTTCCTGTTCTCCAGCGCCT
ACTCC
*(Human IL-21)* 
CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC
*(Human IL-15R α sushi domain)* 
ATTACATGCCCCCCTCCCATGAGCGTGGAGCACGCCGACATCTGGGTG
AAGAGCTATAGCCTCTACAGCCGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*((G4S)3 linker)*
   GGCGGTGGAGGATCCGGAGGAGGTGGCTCCGGCGGCGGAGGATCT
*(Human CD137L)*

CGCGAGGGTCCCGAGCTTTCGCCCGACGATCCCGCCGGCCTCTTGGAC
CTGCGGCAGGGCATGTTTGCGCAGCTGGTGGCCCAAAATGTTCTGCTGATCG
ATGGGCCCCTGAGCTGGTACAGTGACCCAGGCCTGGCAGGCGTGTCCCTGAC
GGGGGGCCTGAGCTACAAAGAGGACACGAAGGAGCTGGTGGTGGCCAAGGC
TGGAGTCTACTATGTCTTCTTTCAACTAGAGCTGCGGCGCGTGGTGGCCGGCG
AGGGCTCAGGCTCCGTTTCACTTGCGCTGCACCTGCAGCCACTGCGCTCTGCT
GCTGGGGCCGCCGCCCTGGCTTTGACCGTGGACCTGCCACCCGCCTCCTCCGA
GGCTCGGAACTCGGCCTTCGGTTTCCAGGGCCGCTTGCTGCACCTGAGTGCCG
GCCAGCGCCTGGGCGTCCATCTTCACACTGAGGCCAGGGCACGCCATGCCTG
GCAGCTTACCCAGGGCGCCACAGTCTTGGGACTCTTCCGGGTGACCCCCGAA
ATCCCAGCCGGACTCCCTTCACCGAGGTCGGAA

The amino acid sequence of 21s137L fusion protein (including the leader sequence) is as follows (SEQ ID NO: 76):
*(Signal peptide)*
   MKWVTFISLLFLFSSAYS
*(Human IL-21)*
*(Human IL-15R α sushi domain)* 
ITCPPPMSVEHADIWVKSYSLYSRERYICNSGFKRKAGTSSLTECVLNKAT
NVAHWTTPSLKCIR
*((G4S)3 linker)*
   GGGGSGGGGSGGGGS
*(Human CD137L)*

REGPELSPDDPAGLLDLRQGMFAQLVAQNVLLIDGPLSWYSDPGLAGVS
LTGGLSYKEDTKELVVAKAGVYYVFFQLELRRVVAGEGSGSVSLALHLQPLRSA
AGAAALALTVDLPPASSEARNSAFGFQGRLLHLSAGQRLGVHLHTEARARHAW
QLTQGATVLGLFRVTPEIPAGLPSPRSE

The nucleic acid sequence of 7t15 construct (including signal peptide sequence) is as follows (SEQ ID NO: 77):
*(Signal peptide)* 
ATGAAGTGGGTGACCTTCATCAGCCTGCTGTTCCTGTTCTCCAGCGCCT
ACTCC
*(Human IL7)* 
GATTGCGACATCGAGGGCAAGGACGGCAAGCAGTACGAGAGCGTGCT
GATGGTGTCCATCGACCAGCTGCTGGACAGCATGAAGGAGATCGGCTCCAAC
TGCCTCAACAACGAGTTCAACTTCTTCAAGCGGCACATCTGCGACGCCAACA
AGGAGGGCATGTTCCTGTTCAGGGCCGCCAGGAAACTGCGGCAGTTCCTGAA
GATGAACTCCACCGGCGACTTCGACCTGCACCTGCTGAAGGTGTCCGAGGGC
ACCACCATCCTGCTGAACTGCACCGGACAGGTGAAGGGCCGGAAACCTGCTG
CTCTGGGAGAGGCCCAACCCACCAAGAGCCTGGAGGAGAACAAGTCCCTGA
AGGAGCAGAAGAAGCTGAACGACCTGTGCTTCCTGAAGAGGCTGCTGCAGG
AGATCAAGACCTGCTGGAACAAGATCCTGATGGGCACCAAGGAGCAT
*(Human Tissue Factor 219)* 
AGCGGCACAACCAACACAGTCGCTGCCTATAACCTCACTTGGAAGAG
CACCAACTTCAAAACCATCCTCGAATGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of 7t15 fusion protein (including the leader sequence) is as follows (SEQ ID NO: 78):
*(Signal peptide)*
   MKWVTFISLLFLFSSAYS
*(Human IL7)* 
DCDIEGKDGKQYESVLMVSIDQLLDSMKEIGSNCLNNEFNFFKRHICDAN
KEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEH
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid and protein sequences of the 21s137L (short version) are shown below. The nucleic acid sequence of 21s137L (short version) construct (including signal peptide sequence) is as follows (SEQ ID NO: 79):
*(Signal peptide)* 
ATGAAGTGGGTGACCTTCATCAGCCTGCTGTTCCTGTTCTCCAGCGCCT
ACTCC
*(Human IL-21)* 
CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC
*(Human IL-15R α sushi domain)* 
ATTACATGCCCCCCTCCCATGAGCGTGGAGCACGCCGACATCTGGGTG
AAGAGCTATAGCCTCTACAGCCGGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*((G4S)3 linker)*
   GGCGGTGGAGGATCCGGAGGAGGTGGCTCCGGCGGCGGAGGATCT
*(Human CD137 Ligand short version)*

GATCCCGCCGGCCTCTTGGACCTGCGGCAGGGCATGTTTGCGCAGCTG
GTGGCCCAAAATGTTCTGCTGATCGATGGGCCCCTGAGCTGGTACAGTGACC
CAGGCCTGGCAGGCGTGTCCCTGACGGGGGGCCTGAGCTACAAAGAGGACA
CGAAGGAGCTGGTGGTGGCCAAGGCTGGAGTCTACTATGTCTTCTTTCAACTA
GAGCTGCGGCGCGTGGTGGCCGGCGAGGGCTCAGGCTCCGTTTCACTTGCGC
TGCACCTGCAGCCACTGCGCTCTGCTGCTGGGGCCGCCGCCCTGGCTTTGACC
GTGGACCTGCCACCCGCCTCCTCCGAGGCTCGGAACTCGGCCTTCGGTTTCCA
GGGCCGCTTGCTGCACCTGAGTGCCGGCCAGCGCCTGGGCGTCCATCTTCAC
ACTGAGGCCAGGGCACGCCATGCCTGGCAGCTTACCCAGGGCGCCACAGTCT
TGGGACTCTTCCGGGTGACCCCCGAAATC

The amino acid sequence of the 21s137L (short version) construct (including signal peptide sequence) is as follows (SEQ ID NO: 80):
*(Signal peptide)*
   MKWVTFISLLFLFSSAYS
*(Human IL-21)*
*(Human IL-15R α sushi domain)* 
ITCPPPMSVEHADIWVKSYSLYSRERYICNSGFKRKAGTSSLTECVLNKAT
NVAHWTTPSLKCIR
*((G4S)3 linker)*
   GGGGSGGGGSGGGGS
*(Human CD137 Ligand short version)*

DPAGLLDLRQGMFAQLVAQNVLLIDGPLSWYSDPGLAGVSLTGGLSYKE
DTKELVVAKAGVYYVFFQLELRRVVAGEGSGSVSLALHLQPLRSAAGAAALAL
TVDLPPASSEARNSAFGFQGRLLHLSAGQRLGVHLHTEARARHAWQLTQGATV
LGLFRVTPEI

The nucleic acid sequence of the 7t15 construct (including signal peptide sequence) is as follows (SEQ ID NO: 81):
*(Signal peptide)* 
ATGAAGTGGGTGACCTTCATCAGCCTGCTGTTCCTGTTCTCCAGCGCCT
ACTCC
*(Human IL7)* 
GATTGCGACATCGAGGGCAAGGACGGCAAGCAGTACGAGAGCGTGCT
GATGGTGTCCATCGACCAGCTGCTGGACAGCATGAAGGAGATCGGCTCCAAC
TGCCTCAACAACGAGTTCAACTTCTTCAAGCGGCACATCTGCGACGCCAACA
AGGAGGGCATGTTCCTGTTCAGGGCCGCCAGGAAACTGCGGCAGTTCCTGAA
GATGAACTCCACCGGCGACTTCGACCTGCACCTGCTGAAGGTGTCCGAGGGC
 
ACCACCATCCTGCTGAACTGCACCGGACAGGTGAAGGGCCGGAAACCTGCTG
CTCTGGGAGAGGCCCAACCCACCAAGAGCCTGGAGGAGAACAAGTCCCTGA
AGGAGCAGAAGAAGCTGAACGACCTGTGCTTCCTGAAGAGGCTGCTGCAGG
AGATCAAGACCTGCTGGAACAAGATCCTGATGGGCACCAAGGAGCAT
*(Human Tissue Factor 219)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of 7t15 fusion protein (including the leader sequence) is as follows (SEQ ID NO: 82):
*(Signal peptide)*
   MKWVTFISLLFLFSSAYS
*(Human IL7)* 
DCDIEGKDGKQYESVLMVSIDQLLDSMKEIGSNCLNNEFNFFKRHICDAN
KEGMFLFRAARKLRQFLKMNSTGDFDLHLLKVSEGTTILLNCTGQVKGRKPAAL
GEAQPTKSLEENKSLKEQKKLNDLCFLKRLLQEIKTCWNKILMGTKEH
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of the TGFRs construct (including signal peptide sequence) is as follows (SEQ ID NO: 83):
*(Signal peptide)* 
ATGAAGTGGGTGACCTTCATCAGCCTGCTGTTCCTGTTCTCCAGCGCCT
ACTCC
*(Human TGFβ Receptor II fragments)* 
ATCCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
 
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human IL-15R α sushi domain)*

ATTACATGCCCCCCTCCCATGAGCGTGGAGCACGCCGACATCTGGGTG
AAGAGCTATAGCCTCTACAGCCGGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG

The amino acid sequence of TGFRs fusion protein (including the leader sequence) is as follows (SEQ ID NO: 84):
*(Signal peptide)* MKWVTFISLLFLFSSAYS
*(Human TGFβ Receptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human IL-15R α sushi domain)*

ITCPPPMSVEHADIWVKSYSLYSRERYICNSGFKRKAGTSSLTECVLNKAT
NVAHWTTPSLKCIR

The nucleic acid sequence of the TGFRt15 construct (including signal peptide sequence) is as follows (SEQ ID NO: 85):
*(Signal peptide)* 
ATGAAGTGGGTGACCTTCATCAGCCTGCTGTTCCTGTTCTCCAGCGCCT
ACTCC
*(Human TGFβ Receptor II fragments)* 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human Tissue Factor 219)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
 
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of TGFRt15 fusion protein (including the leader sequence) is as follows (SEQ ID NO: 86):
*(Signal peptide)*
   MKWVTFISLLFLFSSAYS
*(Human TGFβ Receptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
 
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid and protein sequences of the 21s137L are shown below. The nucleic acid sequence of the 21s137L construct (including signal peptide sequence) is as follows (SEQ ID NO: 87):
*(Signal peptide)* 
ATGAAGTGGGTGACCTTCATCAGCCTGCTGTTCCTGTTCTCCAGCGCCT
ACTCC
*(Human IL-21)* 
CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC
*(Human IL-15R α sushi domain)* 
ATTACATGCCCCCCTCCCATGAGCGTGGAGCACGCCGACATCTGGGTG
AAGAGCTATAGCCTCTACAGCCGGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*((G4S)3 linker)*
   GGCGGTGGAGGATCCGGAGGAGGTGGCTCCGGCGGCGGAGGATCT
*(Human CD137L)*

CGCGAGGGTCCCGAGCTTTCGCCCGACGATCCCGCCGGCCTCTTGGAC
CTGCGGCAGGGCATGTTTGCGCAGCTGGTGGCCCAAAATGTTCTGCTGATCG
ATGGGCCCCTGAGCTGGTACAGTGACCCAGGCCTGGCAGGCGTGTCCCTGAC
GGGGGGCCTGAGCTACAAAGAGGACACGAAGGAGCTGGTGGTGGCCAAGGC
TGGAGTCTACTATGTCTTCTTTCAACTAGAGCTGCGGCGCGTGGTGGCCGGCG
AGGGCTCAGGCTCCGTTTCACTTGCGCTGCACCTGCAGCCACTGCGCTCTGCT
GCTGGGGCCGCCGCCCTGGCTTTGACCGTGGACCTGCCACCCGCCTCCTCCGA
GGCTCGGAACTCGGCCTTCGGTTTCCAGGGCCGCTTGCTGCACCTGAGTGCCG
GCCAGCGCCTGGGCGTCCATCTTCACACTGAGGCCAGGGCACGCCATGCCTG
GCAGCTTACCCAGGGCGCCACAGTCTTGGGACTCTTCCGGGTGACCCCCGAA
ATCCCAGCCGGACTCCCTTCACCGAGGTCGGAA

The amino acid sequence of 21s137L fusion protein (including the leader sequence) is as follows (SEQ ID NO: 88):
*(Signal peptide)*
   MKWVTFISLLFLFSSAYS
*(Human IL-21)*
*(Human IL-15R α sushi domain)* 
ITCPPPMSVEHADIWVKSYSLYSRERYICNSGFKRKAGTSSLTECVLNKAT
NVAHWTTPSLKCIR
*((G4S)3 linker)*
   GGGGSGGGGSGGGGS
*(Human CD137L)*

REGPELSPDDPAGLLDLRQGMFAQLVAQNVLLIDGPLSWYSDPGLAGVS
LTGGLSYKEDTKELVVAKAGVYYVFFQLELRRVVAGEGSGSVSLALHLQPLRSA

AGAAALALTVDLPPASSEARNSAFGFQGRLLHLSAGQRLGVHLHTEARARHAW
QLTQGATVLGLFRVTPEIPAGLPSPRSE

The nucleic acid sequence of the TGFRt15 construct (including signal peptide sequence) is as follows (SEQ ID NO: 89):
*(Signal peptide)* 
ATGAAGTGGGTGACCTTCATCAGCCTGCTGTTCCTGTTCTCCAGCGCCT
ACTCC
*(Human TGFβ Receptor II fragments)* 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human Tissue Factor 219)* 
AGCGGCACAACCAACACAGTCGCTGCCTATAACCTCACTTGGAAGAG
CACCAACTTCAAAACCATCCTCGAATGGGAACCCAAACCCGTTAACCAAGTT
TACACCGTGCAGATCAGCACCAAGTCCGGCGACTGGAAGTCCAAATGTTTCT
 
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of TGFRt15 fusion protein (including the leader sequence) is as follows (SEQ ID NO: 90):
*(Signal peptide)*
   MKWVTFISLLFLFSSAYS
*(Human TGFβ Receptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of the TGFRs21 construct (including signal peptide sequence) is as follows (SEQ ID NO: 91):
*(Signal peptide)* 
(signal peptide)
ATGAAGTGGGTGACCTTCATCAGCCTGCTGTTCCTGTTCTCCAGCGCCT
ACTCC
*(Human TGFβ Receptor II fragments)* 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
 
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human IL-15R α sushi domain)* 
ATTACATGCCCCCCTCCATGAGCGTGGAGCACGCCGACATCTGGGTG
AAGAGCTATAGCCTCTACAGCCGGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*(Human IL-21)*

CAGGGCCAGGACAGGCACATGATCCGGATGAGGCAGCTCATCGACAT
CGTCGACCAGCTGAAGAACTACGTGAACGACCTGGTGCCCGAGTTTCTGCCT
GCCCCCGAGGACGTGGAGACCAACTGCGAGTGGTCCGCCTTCTCCTGCTTTC
AGAAGGCCCAGCTGAAGTCCGCCAACACCGGCAACAACGAGCGGATCATCA
ACGTGAGCATCAAGAAGCTGAAGCGGAAGCCTCCCTCCACAAACGCCGGCA
GGAGGCAGAAGCACAGGCTGACCTGCCCCAGCTGTGACTCCTACGAGAAGA
AGCCCCCCAAGGAGTTCCTGGAGAGGTTCAAGTCCCTGCTGCAGAAGATGAT
CCATCAGCACCTGTCCTCCAGGACCCACGGCTCCGAGGACTCC

The amino acid sequence of TGFRs21 fusion protein (including the leader sequence) is as follows (SEQ ID NO: 92):
*(Signal peptide)*
   MKWVTFISLLFLFSSAYS
*(Human TGFβ Receptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human IL-15R α sushi domain)* 
ITCPPPMSVEHADIWVKSYSLYSRERYICNSGFKRKAGTSSLTECVLNKAT
NVAHWTTPSLKCIR
*(Human IL-21)*

The nucleic acid sequence of the TGFRt15 construct (including signal peptide sequence) is as follows (SEQ ID NO: 93):
*(Signal peptide)* 
ATGAAGTGGGTGACCTTCATCAGCCTGCTGTTCCTGTTCTCCAGCGCCT
ACTCC
*(Human TGFβ Receptor II fragments)* 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
 
GACCTTTTTCATGTGCTCCTGCAGCAGCGACGAATGCAACGACAATATCATCT
TTAGCGAGGAATACAATACCAGCAACCCCGAC
*(Human Tissue Factor 219)* 
AGCGGCACAACCAACACAGTCGCTGCCTATAACCTCACTTGGAAGAG
CACCAACTTCAAAACCATCCTCGAATGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of TGFRt15 fusion protein (including the leader sequence) is as follows (SEQ ID NO: 94):
*(Signal peptide)*
   MKWVTFISLLFLFSSAYS
*(Human TGFβ Receptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of the TGFRs16 construct (including signal peptide sequence) is as follows (SEQ ID NO: 95):
*(Signal peptide)* 
ATGAAGTGGGTGACCTTCATCAGCCTGCTGTTCCTGTTCTCCAGCGCCT
ACTCC
*(Human TGFβReceptor II fragments)* 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
 
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human IL-15R α sushi domain)* 
ATTACATGCCCCCCTCCCATGAGCGTGGAGCACGCCGACATCTGGGTG
AAGAGCTATAGCCTCTACAGCCGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*(Anti-human CD16scFv)*

TCCGAGCTGACCCAGGACCCTGCTGTGTCCGTGGCTCTGGGCCAGACC
GTGAGGATCACCTGCCAGGGCGACTCCCTGAGGTCCTACTACGCCTCCTGGT
ACCAGCAGAAGCCCGGCCAGGCTCCTGTGCTGGTGATCTACGGCAAGAACAA
CAGGCCCTCCGGCATCCCTGACAGGTTCTCCGGATCCTCCTCCGGCAACACCG
CCTCCCTGACCATCACAGGCGCTCAGGCCGAGGACGAGGCTGACTACTACTG
CAACTCCAGGGACTCCTCCGGCAACCATGTGGTGTTCGGCGGCGGCACCAAG
CTGACCGTGGGCCATGGCGGCGGCGGCTCCGGAGGCGGCGGCAGCGGCGGA
GGAGGATCCGAGGTGCAGCTGGTGGAGTCCGGAGGAGGAGTGGTGAGGCCT
GGAGGCTCCCTGAGGCTGAGCTGTGCTGCCTCCGGCTTCACCTTCGACGACTA
CGGCATGTCCTGGGTGAGGCAGGCTCCTGGAAAGGGCCTGGAGTGGGTGTCC
GGCATCAACTGGAACGGCGGATCCACCGGCTACGCCGATTCCGTGAAGGGCA
GGTTCACCATCAGCAGGGACAACGCCAAGAACTCCCTGTACCTGCAGATGAA
CTCCCTGAGGGCCGAGGACACCGCCGTGTACTACTGCGCCAGGGGCAGGTCC
CTGCTGTTCGACTACTGGGGACAGGGCACCCTGGTGACCGTGTCCAGG

The amino acid sequence of TGFRs16 fusion protein (including the leader sequence) is as follows (SEQ ID NO: 96):
*(Signal peptide)*
   MKWVTFISLLFLFSSAYS
*(Human TGFβReceptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human IL-15R α sushi domain)* 
ITCPPPMSVEHADIWVKSYSLYSRERYICNSGFKRKAGTSSLTECVLNKAT
NVAHWTTPSLKCIR
*(Anti-human CD16scFv)*

SELTQDPAVSVALGQTVRITCQGDSLRSYYASWYQQKPGQAPVLVIYGK
NNRPSGIPDRFSGSSSGNTASLTITGAQAEDEADYYCNSRDSSGNHVVFGGGTKL
TVGHGGGGSGGGGSGGGGSEVQLVESGGGVVRPGGSLRLSCAASGFTFDDYGM
SWVRQAPGKGLEWVSGINWNGGSTGYADSVKGRFTISRDNAKNSLYLQMNSLR
AEDTAVYYCARGRSLLFDYWGQGTLVTVSR

The nucleic acid sequence of the TGFRt15 construct (including signal peptide sequence) is as follows (SEQ ID NO: 97):
*(Signal peptide)* 
ATGAAGTGGGTGACCTTCATCAGCCTGCTGTTCCTGTTCTCCAGCGCCT
ACTCC
*(Human TGFβReceptor II fragments)* 
ATCCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
 
CTCCATCTGCGAGAAGCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human Tissue Factor 219)* 
AGCGGCACAACCAACACAGTCGCTGCCTATAACCTCACTTGGAAGAG
CACCAACTTCAAAACCATCCTCGAATGGAACCCAAACCCGTTAACCAAGTT
TACACCGTGCAGATCAGCACCAAGTCCGGCGACTGGAAGTCCAAATGTTTCT
ATACCACCGACACCGAGTGCGATCTCACCGATGAGATCGTGAAAGATGTGAA
ACAGACCTACCTCGCCCGGGTGTTTAGCTACCCCGCCGGCAATGTGGAGAGC
ACTGGTTCCGCTGGCGAGCCTTTATACGAGAACAGCCCCGAATTTACCCCTTA
CCTCGAGACCAATTTAGGACAGCCCACCATCCAAAGCTTTGAGCAAGTTGGC
ACAAAGGTGAATGTGACAGTGGAGGACGAGCGGACTTTAGTGCGGCGGAAC
AACACCTTTCTCAGCCTCCGGGATGTGTTCGGCAAAGATTTAATCTACACACT
GTATTACTGGAAGTCCTCTTCCTCCGGCAAGAAGACAGCTAAAACCAACACA
AACGAGTTTTTAATCGACGTGGATAAAGGCGAAAACTACTGTTTCAGCGTGC
AAGCTGTGATCCCCTCCCGGACCGTGAATAGGAAAAGCACCGATAGCCCCGT
TGAGTGCATGGGCCAAGAAAAGGGCGAGTTCCGGGAG
*(Human IL-15)*

AACTGGGTGAACGTCATCAGCGATTTAAAGAAGATCGAAGATTTAATT
CAGTCCATGCATATCGACGCCACTTTATACACAGAATCCGACGTGCACCCCTC
TTGTAAGGTGACCGCCATGAAATGTTTTTACTGGAGCTGCAAGTTATCTCTT
TAGAGAGCGGAGACGCTAGCATCCACGACACCGTGGAGAATTTAATCATTTT
AGCCAATAACTCTTTATCCAGCAACGGCAACGTGACAGAGTCCGGCTGCAAG
GAGTGCGAAGAGCTGGAGGAGAAGAACATCAAGGAGTTTCTGCAATCCTTTG
TGCACATTGTCCAGATGTTCATCAATACCTCC

The amino acid sequence of TGFRt15 fusion protein (including the leader sequence) is as follows (SEQ ID NO: 98):
*(Signal peptide)*
   MKWVTFISLLFLFSSAYS
*(Human TGFβReceptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human Tissue Factor 219)* 
SGTTNTVAAYNLTWKSTNFKTILEWEPKPVNQVYTVQISTKSGDWKSKC
FYTTDTECDLTDEIVKDVKQTYLARVFSYPAGNVESTGSAGEPLYENSPEFTPYL
ETNLGQPTIQSFEQVGTKVNVTVEDERTLVRRNNTFLSLRDVFGKDLIYTLYYW
KSSSSGKKTAKTNTNEFLIDVDKGENYCFSVQAVIPSRTVNRKSTDSPVECMGQE
KGEFRE
*(Human IL-15)*

NWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQ
MFINTS

The nucleic acid sequence of the TGFRs137L construct (including signal peptide sequence) is as follows (SEQ ID NO: 99):
*(Signal peptide)* 
ATGAAGTGGGTGACCTTCATCAGCCTGCTGTTCCTGTTCTCCAGCGCCT
ACTCC
*(Human TGFβReceptor II fragments)* 
ATCCCCCCCATGTGCAAAAGAGCGTGAACAACGATATGATCGTGACC
GACAACAACGGCGCCGTGAAGTTTCCCCAGCTCTGCAAGTTCTGCGATGTCA
GGTTCAGCACCTGCGATAATCAGAAGTCCTGCATGTCCAACTGCAGCATCAC
CTCCATCTGCGAGAAGCCCCAAGAAGTGTGCGTGGCCGTGTGGCGGAAAAAT
GACGAGAACATCACCCTGGAGACCGTGTGTCACGACCCCAAGCTCCCTTATC
ACGACTTCATTCTGGAGGACGCTGCCTCCCCCAAATGCATCATGAAGGAGAA
GAAGAAGCCCGGAGAGACCTTCTTTATGTGTTCCTGTAGCAGCGACGAGTGT
AACGACAACATCATCTTCAGCGAAGAGTACAACACCAGCAACCCTGATGGAG
GTGGCGGATCCGGAGGTGGAGGTTCTGGTGGAGGTGGGAGTATTCCTCCCCA
CGTGCAGAAGAGCGTGAATAATGACATGATCGTGACCGATAACAATGGCGCC
GTGAAATTTCCCCAGCTGTGCAAATTCTGCGATGTGAGGTTTTCCACCTGCGA
CAACCAGAAGTCCTGTATGAGCAACTGCTCCATCACCTCCATCTGTGAGAAG
CCTCAGGAGGTGTGCGTGGCTGTCTGGCGGAAGAATGACGAGAATATCACCC
TGGAAACCGTCTGCCACGATCCCAAGCTGCCCTACCACGATTTCATCCTGGA
AGACGCCGCCAGCCCTAAGTGCATCATGAAAGAGAAAAAGAAGCCTGGCGA
GACCTTTTTCATGTGCTCCTGCAGCAGCGACGAATGCAACGACAATATCATCT
TTAGCGAGGAATACAATACCAGCAACCCCGAC
*(Human IL-15R α sushi domain)* 
ATTACATGCCCCCCTCCATGAGCGTGGAGCACGCCGACATCTGGGTG
AAGAGCTATAGCCTCTACAGCCGGGAGAGGTATATCTGTAACAGCGGCTTCA
AGAGGAAGGCCGGCACCAGCAGCCTCACCGAGTGCGTGCTGAATAAGGCTA
CCAACGTGGCTCACTGGACAACACCCTCTTTAAAGTGCATCCGG
*((G4S)3 linker)* GGCGGTGGAGGATCCGGAGGAGGTGGCTCCGGCGGCGGAGGATCT
*(Human CD137L)*

CGCGAGGGTCCCGAGCTTTCGCCCGACGATCCCGCCGGCCTCTTGGAC
CTGCGGCAGGGCATGTTTGCGCAGCTGGTGGCCCAAAATGTTCTGCTGATCG
ATGGGCCCCTGAGCTGGTACAGTGACCCAGGCCTGGCAGGCGTGTCCCTGAC
GGGGGGCCTGAGCTACAAAGAGGACACGAAGGAGCTGGTGGTGGCCAAGGC
TGGAGTCTACTATGTCTTCTTTCAACTAGAGCTGCGGCGCGTGGTGGCCGGCG
AGGGCTCAGGCTCCGTTTCACTTGCGCTGCACCTGCAGCCACTGCGCTCTGCT
GCTGGGGCCGCCGCCCTGGCTTTGACCGTGGACCTGCCACCCGCCTCCTCCGA
GGCTCGGAACTCGGCCTTCGGTTTCCAGGGCCGCTTGCTGCACCTGAGTGCCG
GCCAGCGCCTGGGCGTCCATCTTCACACTGAGGCCAGGGCACGCCATGCCTG
GCAGCTTACCCAGGGCGCCACAGTCTTGGGACTCTTCCGGGTGACCCCCGAA
ATCCCAGCCGGACTCCCTTCACCGAGGTCGGAA

The amino acid sequence of TGFRs137L fusion protein (including the leader sequence) is as follows (SEQ ID NO: 100):
*(Signal peptide)*
   MKWVTFISLLFLFSSAYS
*(Human TGFβReceptor II)* 
IPPHVQKSVNNDMIVTDNNGAVKFPQLCKFCDVRFSTCDNQKSCMSNCSI
TSICEKPQEVCVAVWRKNDENITLETVCHDPKLPYHDFILEDAASPKCIMKEKKK
PGETFFMCSCSSDECNDNIIFSEEYNTSNPDGGGGSGGGGSGGGGSIPPHVQKSVN
NDMIVTDNNGAVKFPQLCKFCDVRFSTCDNQKSCMSNCSITSICEKPQEVCVAV
WRKNDENITLETVCHDPKLPYHDFILEDAASPKCIMKEKKKPGETFFMCSCSSDE
CNDNIIFSEEYNTSNPD
*(Human IL-15R α sushi domain)* 
ITCPPPMSVEHADIWVKSYSLYSRERYICNSGFKRKAGTSSLTECVLNKAT
NVAHWTTPSLKCIR
*((G4S)3 linker)* GGGGSGGGGSGGGGS
*(Human CD137L)*

REGPELSPDDPAGLLDLRQGMFAQLVAQNVLLIDGPLSWYSDPGLAGVS
LTGGLSYKEDTKELVVAKAGVYYVFFQLELRRVVAGEGSGSVSLALHLQPLRSA
AGAAALALTVDLPPASSEARNSAFGFQGRLLHLSAGQRLGVHLHTEARARHAW
QLTQGATVLGLFRVTPEIPAGLPSPRSE

### Compositions/Kits

Also provided herein are compositions that include any of the affinity chromatography resins described herein. Also provided herein are chromatography columns that include any of the affinity chromatography resins described herein. Also provided herein are kits that include any of the affinity chromatography resins or chromatography columns described herein. Some embodiments of any of the kits described herein further include instructions for performing any of the methods described herein.

### EXAMPLES

### Example 1: Comparison of CNBr-activated Sepharose vs NHS Sepharose

Solid support materials for coupling proteins may contain different activation groups or use different chemical reactions. Thus, two different solid support materials for anti-tissue factor antibody coupling were evaluated to see which one is a better based resin for high yield recovery of human tissue factor (TF)-containing proteins (e.g., any of the single-chain or multi-chain chimeric proteins described herein).

Two anti-tissue factor affinity columns were produced using NHS-Activated Sepharose 4 Fast Flow resin and CNBr Activated Sepharose 4 Fast Flow resin from GE Healthcare. The anti-tissue factor used in these experiments include a heavy chain variable domain comprising SEQ ID NO: 7 and a light chain variable domain comprising SEQ ID NO: 8.

For the same amount of each resin, the same amount of anti-tissue factor antibody was conjugated under the same conditions per the manufacturer's instructions. The two anti-tissue factor affinity chromatography resins were then packed into two columns and evaluated using 18t15-12s harvest for yield recovery and performance. When the same amount of 18t15-12s was loaded onto each anti-tissue factor affinity chromatography column, the anti-tissue factor CNBr- Sepharose column yielded 50% higher (6 mg vs 4 mg) 18t15-12s in the elution peak compared to anti-tissue factor-NHS Sepharose column (NB#07, PG#08-09) (see, Table 1).

**Table 1. Comparison of CNBr-activated Sepharose vs NHS Sepharose**

| Parameters | CNBr-activated Sepharose 4 Fast Flow | NHS-activated Sepharose 4 Fast Flow |
|---|---|---|
| Resin volume or weight | 6.83 mL | 6.0 mL |
| Column volume, mL | 6.83 | 6.0 |
| Anti-TF Ab amount conjugated, mg | 30 | 30 |
| Volume of 18t15-12s harvest loaded, mL | 480 | 480 |
| Total amount of 18t15-12s in elution peak, mg | 6.04 | 4.24 |

### Example 2: Elution Conditions

An elution profile of 18t15-12s from a 1.6 cm x 3.4 cm (6.83 mL CV) anti-tissue factor CNBr-Sepharose affinity column that was equilibrated with 5CVs phosphate buffered saline, loaded with 18t15-12s washed with 7CVs phosphate buffered saline, and eluted with 6CVs of 0.1 M acetic acid, pH 2.9 is shown in FIG. 2. The resulting chromatograph in FIG. 2 shows that elution using 0.1 M acetic acid resulted in a sharp protein peak with minimal tailing. In view of these data, 0.1 M acetic acid was selected as a useful elution buffer for the anti-tissue factor affinity chromatography resin.

### Example 3: Coupling Density and Binding Capacity of Target Protein

To evaluate the binding capacity of anti-tissue factor affinity columns, resins containing different coupling densities of the anti-tissue factor were prepared using CNBr-Sepharose, and then 18t15-12s was loaded to generate yield recovery data. FIG.3 represents historical data from many different resin preparation lots showing an increasing binding capacity with increasing quantities of anti-tissue factor conjugated to the CNBr-activated Sepharose resin. This increased binding capacity begins to level off at around 9 mg anti-tissue factor/mL CNBr-activated Sepharose resin.

### Example 4: Coupling Time and Mixing Method

Different mixing methods and conjugation times were evaluated for anti-tissue factor coupling conditions. No difference was observed in coupling for 2-4 hours versus overnight conjugation time. The data is represented in Table 2 below. Also, no difference was observed for rocker versus impeller mixing method.

**Table 2. Historical resin preparation data**

| Column ID | Mixing Method | CV Column (mL) | Ligand Density (mg antibody/mL resin) | Total mg of 18t15-12s recovered per column | Binding capacity (mg product/mL resin) | Amount of 18t15-12s recovered per mg of immobilized anti-tissue factor antibody |
|---|---|---|---|---|---|---|
| 1 | Rocker | 6.83 | 2.19 | 6.86 | 1 | 0.457 |
| 2 | Rocker | 9 | 4.44 | 17.6 | 1.955 | 0.44 |
| 3 | Rocker | 15.57 | 8.06 | 62.71 | 4.027 | 0.500 |
| 15-37cond2 | Impellor | 2.67 | 8.23 | 12.47 | 4.67 | 0.567 |
| 15-37cond3 | Rocker | 2.67 | 9.582 | 12.24 | 4.58 | 0.478 |
| 15-37cond1 | Impellor | 2.67 | 8.86 | 11.815 | 4.4 | 0.497 |
| 14-25 | Impellor | 40 | 8.52 | ND | 4 | 0.469 |

### Example 5: Wash Studies

A set of experiments were performed to identify optimal wash conditions for the anti-tissue factor chromatography resins described herein. In these experiments, two different first wash buffers were compared: phosphate buffered saline and 20 mM Tris, 1 M NaCl, pH 8.0. Using 20 mM Tris, 1 M NaCl, pH 8.0 as the first wash buffer led to loss of material without an increase in product quality. After this initial experiment, 0.1 M sodium citrate, pH 5.0 was evaluated as a second wash buffer (after use of a first wash buffer of phosphate buffered saline).

The use of a first wash buffer of phosphate buffered saline, following by a second wash buffer of 0.1 M sodium citrate, pH 5.0 was adopted as it led to an increase in elution pool pH. This increased elution pool pH was found to be protective for the product quality as it relates to aggregation. FIG. 4 shows the optimization of wash conditions for 18t15-12s purification by anti-tissue factor CNBr-Sepharose affinity column. Each experiment was performed using a 2.6 cm x 7.4 cm (39.27 mL CV) column equilibrated with 5 CVs of phosphate buffered saline, loaded, washed with 5CVs of phosphate buffered saline and further washed with either 5 CVs of phosphate buffered saline or 5 CVs of 0.1 M citrate, pH 5.0, and eluted with 6 CVs of 0.1M acetic acid, pH 2.9.

**Table 3. Effect of wash 2 (0.1M sodium citrate, pH 5.0) on 18t15-12s product quality**

| Experiment | Wash condition | 18t15-12s aggregates (%) | 18t15-12s main peak (%) |
|---|---|---|---|
| 15-01 | No pH 5.0 wash | 19.81 | 80.19 |
| 15-03 | Wash with 0.1M sodium citrate, pH 5.0 | 8.4 | 91.6 |

### Example 6: Stripping Condition, Storage Solution, and Stability Data

0.1M Glycine pH 2.5 was selected as the strip buffer for the anti-tissue factor-CNBr Sepharose column. The conjugated column was stored in phosphate buffered saline, 0.05% sodium azide. In order to utilize a storage solution that could be easily transferred to manufacturing, 20% ethanol was adopted as the new storage solution. No column performance loss is observed when storing in 20% ethanol for up to a year. Additionally, anti-tissue factor CNBr-Sepharose columns have been used for up to a year without significant loss of binding capacity. FIGs. 5 and 6 show the elution profiles of the first run, with a newly generated anti-tissue factor-CNBr Sepharose column and the 51^{st} run with the same column. FIG. 5 shows the elution profile of the first run of a newly generated 2.6 cm x 9.5 cm (50.41 mL CV) anti-tissue factor-CNBr Sepharose column purified by equilibrating with 5CVs phosphate buffered saline, loaded, washed with 5 CVs phosphate buffered saline, and eluted with 6 CVs of 0.1M acetic acid, pH 2.9. FIG. 6 shows the elution profile of the 51^{st} purification run (the same column used in Fig. 5), where the chromatography run was performed using the steps of equilibration using 5 CVs phosphate buffered saline, loading, washing with 5 CVs of phosphate buffered saline, and eluted with 6 CVs of 0.1 M acetic acid, pH 2.9.

### Example 7: Development of Sanitation Method

In order to avoid contamination, sanitization solutions are typically used in column chromatography before column use, post use, and storage. Initially 50 mM NaOH 1 M NaCl with a 15-minute contact time was tested. This resulted in a 60% loss of the column binding capacity, and thus 50 mM NaOH, 1 M NaCl was no longer used as a sanitation solution. A commercial solution of 120 mM phosphoric acid, 167 mM acetic acid, 2.2% benzyl alcohol, pH 5.0 was also tested as a possible sanitization buffer. Although 120 mM phosphoric acid, 167 mM acetic acid, 2.2% benzyl alcohol, pH 5.0 resulted in good column stability, it was abandoned as a sanitization strategy due to its interaction with plastic containers. Then, 50 mM citrate, 2% benzyl alcohol, pH 5.0 with a 15-minute contact time was evaluated as a sanitation solution. The results indicated that there was no loss of column binding capacity. Therefore, 50 mM citrate, 2% benzyl alcohol, pH 5.0 was selected as a sanitation solution for the anti-tissue factor affinity chromatography columns.

Studies were also performed to determine if product carryover of loaded product from one run to the next was occurring. This was accomplished by equilibrating a 2.6 cm x 9.5 cm (50.41 mL CV) anti-tissue factor-CNBr Sepharose column with 5CVs phosphate buffered saline, loading 18t15-12s onto the column, washing with 5 CVs of PBS, washing with 5 CVs of 0.1 M citrate, pH 5.0, eluting with 0.1 M acetic acid, pH 2.9, stripping with 4 CVs of 0.1 M glycine, pH 2.5, neutralizing with 4 CVs of phosphate buffered saline, and storing with 7 CVs of 20% ethanol. The same process was then repeated, but instead of loading 18t15-12s the column was loaded with phosphate buffered saline, and the elution pool was analyzed by ELISA for residual 18t15-12s A minimal amount- 1.162 µg/mL 18t15-12s was detected in the elution of the next run.

An eluate from a process demonstration run, using the same purification process listed above was tested by ELISA for residual anti-tissue factor antibody to evaluate the stability of the column, and to verify that a minimum amount of antibody leaching was occurring from the column into the elution pool during processing. A negligible quantity, 17 ppm, was detected in the elution.

### Example 8: Examples of TF Fusion Proteins and TF Fusion Protein Complexes

A variety of different single-chain and multi-chain chimeric polypeptides including a soluble tissue factor domain have been successfully purified using anti-tissue factor CNBr Sepharose affinity chromatography resins provided herein. Multi-chain chimeric polypeptides that have been successfully purified using anti-tissue factor CNBr Sepharose affinity chromatography resins provided herein include 18t15-12s, 7t15-21s, TGFRt15-TGFRs, 7t15-21s137L, 21t15-TGFRs, TGFR115-21s137L, TGFRt15-TGFRs137L, TGFRt15-TGFRs21, TGFRt15-TGFRs16, 7t15-TGFRs, 21t15-7s, 18t15-12s16, 7t15-16s21, TGFRt15-16s21, and 7t15-7s. Single-chain chimeric polypeptides that have been successfully purified using anti-tissue factor CNBr Sepharose affinity chromatography resins described herein are 2t2, 3t28, and 15t15.

A preliminary stability study was performed on 18t15-12s a fusion protein product purified through the anti-tissue factor-CNBr Sepharose column and then subsequent unit operations were performed (as outlined in FIG. 1). The resulting manufactured 18t15-12s was stable for up to 5 weeks (Table 4).

**Table 4. Stability data for 18t15-12s incubated at 2-8 °C, 25 °C, and 37 °C, and then analyzed by HPLC-SEC 1 week timepoints up to 5 weeks.**

| | **18t15-12s 2-8 °C Stability Data** | | | **18t15-12s 25 °C Stability Data** | | | **18t15-12s 37 °C Stability Data** | | |
|---|---|---|---|---|---|---|---|---|---|
| Time points | **HMWS (%)** | **Main (%)** | **LMWS (%)** | **HMWS (%)** | **Main (%)** | **LMWS (%)** | **HMWS (%)** | **Main (%)** | **LMWS (%)** |

| | | | | | | | | | |
|---|---|---|---|---|---|---|---|---|---|
| **0 week** | 8.91 | 91.09 | 0 | 8.91 | 91.09 | 0 | 8.91 | 91.09 | 0 |
| **1 week** | 9.46 | 90.54 | 0 | 10.45 | 89.55 | 0 | 10.83 | 89.17 | 0 |
| **2 weeks** | 7.89 | 92.11 | 0 | 9.44 | 90.47 | 0.9 | 9.84 | 87.07 | 3.1 |
| **3 weeks** | 10.2 | 89.65 | 0.15 | 11.19 | 88.7 | 0.11 | 12.27 | 87.69 | 0.09 |
| **4 weeks** | 9.07 | 90.7 | 0.24 | 10.62 | 89.37 | 0.02 | 11.7 | 88.18 | 0.12 |
| **5 weeks** | 5.1 | 94.84 | 0.06 | 7.65 | 92.32 | 0.03 | 8.23 | 90.67 | 1.1 |

### Example 9: Purification of TGFRt15-TGFRs and 2t2

The conditions to purify TGFRt15-TGFRs and 2t2 using an affinity chromatograph resin as described herein (anti-tissue factor CNBr Sepharose affinity chromatography resin). In general, the anti-tissue factor CNBr Sepharose affinity chromatography resin was prepared as previously described. The anti-tissue factor CNBr Sepharose affinity chromatography resin was then packed into a column and clarified cell harvest containing either TGFRt15-TGFRs or 2t2 was loaded onto the column which had been equilibrated with 4 column volume (CV) of PBS. After sample loading, the column was washed with 5 CV of PBS and 5 CV of 0.1 M sodium citrate, pH 5.0. The target protein (TGFRt15-TGFRs or 2t2) was then eluted with 3-4 CV of 0.1 M acetic acid, pH 2.9. The eluted protein peak of TGFRt15-TGFRs or 2t2 was then adjusted to neutral pH (7-8) with 1 M Tris if the protein material is used for research purposes, adjusted to pH 3.6 with 1 M citric acid, followed by incubation at this low pH for 1 hour at room temperature if the protein material is used for clinical study. The low pH incubation serves to inactivate viruses that may be potentially present in cell culture harvest. After low pH incubation, 1 M Tris was then added to adjust pH to 6.0 for TGFRt15-TGFRs or to 5.0 for 2t2, followed by depth filtration to remove or reduce product or process-related protein contaminants. After protein elution, the column was stripped and stored as described in Example 7.

Chromatograms showing elution of TGFRt15-TGFRs and 2t2 are shown in FIG. 9 and FIG. 10, respectively.

## Claims

1. Use of an affinity chromatography resin for purifying a single-chain or a multi-chain chimeric polypeptide which comprises a soluble tissue factor, the affinity chromatography resin comprising an anti-tissue factor antibody or antigen-binding fragment thereof comprising a heavy chain variable domain comprising CDRs of SEQ ID NOs: 1, 2 or 9, and 3, and a light chain variable domain comprising CDRs of SEQ ID NOs: 4, 5, and 6, attached to a base resin.

2. The use of claim 1, wherein the heavy chain variable domain comprises a sequence that is at least 90% identical to SEQ ID NO: 7, and the light chain variable domain comprises a sequence that is at least 90% identical to SEQ ID NO: 8.

3. The use of claim 2, wherein the heavy chain variable domain comprises SEQ ID NO: 7, and the light chain variable domain comprises SEQ ID NO: 8.

4. The use of any one of claims 1-3, wherein the base resin is sepharose.

5. The use of any one of claims 1-3, wherein the base resin is any support material.

6. The use of claim 5, wherein the support material is agarose or a capto resin.

7. The use of any one of claims 1-6, wherein the anti-tissue factor antibody or antigen-binding fragment thereof is non-covalently attached to the base resin.

8. The use of any one of claims 1-6, wherein the anti-tissue factor antibody or antigen-binding fragment thereof is covalently attached to the base resin.

9. The use of claim 8, wherein the anti-tissue factor antibody or antigen-binding fragment thereof is covalently attached to the base resin through the formation of a disulfide bond between a cysteine in the anti-tissue factor antibody or antigen-binding fragment thereof and a chemical group on the base resin.

10. The use of claim 8, wherein the anti-tissue factor antibody or antigen-binding fragment thereof is covalently attached to the base resin through the formation of a covalent bond between a free amine of the anti-tissue factor antibody or antigen-binding domain and a chemical group on the base resin.

11. The use of claim 10, wherein the base resin is CNBr-activated or N-hydroxysuccinimide (NHS)-activated solid support material.

12. The use of claim 10, wherein the covalent bond is represented by Formula I below: 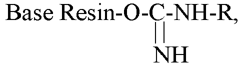 wherein R represents the anti-tissue factor antibody or antigen-binding fragment thereof.

13. Use of a kit comprising the affinity chromatography resin of any one of claims 1-12 for purifying a single-chain or a multi-chain chimeric polypeptide which comprises a soluble tissue factor.

14. The use of any one of claims 1-13, wherein: the single-chain chimeric polypeptide comprises:
(i) a first target-binding domain;
(ii) a soluble tissue factor domain; and
(iii) a second target-binding domain; and
the multi-chain chimeric polypeptide comprises:
(a) a first chimeric polypeptide comprising:
(i) a first target-binding domain;
(ii) a soluble tissue factor domain; and
(iii) a first domain of a pair of affinity domains;
(b) a second chimeric polypeptide comprising:
(i) a second domain of a pair of affinity domains; and
(ii) a second target-binding domain,
wherein the first chimeric polypeptide and the second chimeric polypeptide associate through the binding of the first domain and the second domain of the pair of affinity domains.

## Patentansprüche

1. Verwendung eines Affinitätschromatographieharzes zur Reinigung eines einkettigen oder mehrkettigen chimären Polypeptids, das einen löslichen Gewebefaktor umfasst, wobei das Affinitätschromatographieharz einen Anti-Gewebefaktor-Antikörper oder ein antigenbindendes Fragment davon umfassend eine variable Schwerkettendomäne umfassend CDRs der SEQ ID NOS: 1, 2 oder 9, und 3 und eine variable Leichtkettendomäne umfassend CDRs der SEQ ID NOS: 4, 5 und 6 an ein Grundharz gebunden umfasst.

2. Verwendung nach Anspruch 1, wobei die variable Schwerkettendomäne eine Sequenz umfasst, die wenigstens 90 % identisch mit SEQ ID NO: 7 ist, und die variable Leichtkettendomäne eine Sequenz umfasst, die wenigstens 90 % identisch mit SEQ ID NO: 8 ist.

3. Verwendung nach Anspruch 2, wobei die variable Schwerkettendomäne SEQ ID NO: 7 umfasst und die variable Leichtkettendomäne SEQ ID NO: 8 umfasst.

4. Verwendung nach einem der Ansprüche 1-3, wobei das Grundharz Sepharose ist.

5. Verwendung nach einem der Ansprüche 1-3, wobei das Grundharz ein beliebiges Trägermaterial ist.

6. Verwendung nach Anspruch 5, wobei das Trägermaterial Agarose oder ein Capto-Harz ist.

7. Verwendung nach einem der Ansprüche 1-6, wobei der Anti-Gewebefaktor-Antikörper oder das antigenbindende Fragment davon nichtkovalent an das Grundharz gebunden ist.

8. Verwendung nach einem der Ansprüche 1-6, wobei der Anti-Gewebefaktor-Antikörper oder das antigenbindende Fragment davon kovalent an das Grundharz gebunden ist.

9. Verwendung nach Anspruch 8, wobei der Anti-Gewebefaktor-Antikörper oder das antigenbindende Fragment davon durch die Bildung einer Disulfidbindung zwischen einem Cystein in dem Anti-Gewebefaktor-Antikörper oder dem antigenbindenden Fragment davon und einer chemischen Gruppe an dem Grundharz kovalent an das Grundharz gebunden ist.

10. Verwendung nach Anspruch 8, wobei der Anti-Gewebefaktor-Antikörper oder das antigenbindende Fragment davon durch die Bildung einer kovalenten Bindung zwischen einem freien Amin des Anti-Gewebefaktor-Antikörpers oder der antigenbindenden Domäne und einer chemischen Gruppe an dem Grundharz kovalent an das Grundharz gebunden ist.

11. Verwendung nach Anspruch 10, wobei das Grundharz CNBr-aktiviertes oder N-Hydroxysuccinimid(NHS)-aktiviertes festes Trägermaterial ist.

12. Verwendung nach Anspruch 10, wobei die kovalente Bindung durch die nachstehende Formel I dargestellt wird: 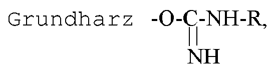 wobei R den Anti-Gewebefaktor-Antikörper oder das antigenbindende Fragment davon darstellt.

13. Verwendung eines Kits, das das Affinitätschromatographieharz nach einem der Ansprüche 1-12 umfasst, zur Reinigung eines einkettigen oder eines mehrkettigen chimären Polypeptids, das einen löslichen Gewebefaktor umfasst.

14. Verwendung nach einem der Ansprüche 1-13, wobei: das einkettige chimäre Polypeptid umfasst:
(i) eine erste Zielbindungsdomäne;
(ii) eine lösliche Gewebefaktordomäne; und
(iii) eine zweite Zielbindungsdomäne; und
das mehrkettige chimäre Polypeptid umfasst:
(a) ein erstes chimäres Polypeptid umfassend:
(i) eine erste Zielbindungsdomäne;
(ii) eine lösliche Gewebefaktordomäne; und
(iii) eine erste Domäne eines Paars von Affinitätsdomänen;
(b) ein zweites chimäres Polypeptid umfassend:
(i) eine zweite Domäne eines Paars von Affinitätsdomänen; und
(ii) eine zweite Zielbindungsdomäne,
wobei das erste chimäre Polypeptid und das zweite chimäre Polypeptid durch die Bindung der ersten Domäne und der zweiten Domäne des Paars von Affinitätsdomänen assoziieren.

## Revendications

1. Utilisation d'une résine de chromatographie d'affinité pour purifier un polypeptide chimérique monocaténaire ou multicaténaire qui comprend un facteur tissulaire soluble, la résine de chromatographie d'affinité comprenant un anticorps anti-facteur tissulaire ou un fragment de liaison d'antigène de celui-ci comprenant un domaine variable de chaîne lourde comprenant des CDR de SEQ ID NO: 1, 2 ou 9, et 3, et un domaine variable de chaîne légère comprenant des CDR de SEQ ID NO: 4, 5 et 6, fixé à une résine de base.

2. Utilisation selon la revendication 1, dans laquelle le domaine variable de chaîne lourde comprend une séquence qui est au moins 90 % identique à SEQ ID NO: 7, et le domaine variable de chaîne légère comprend une séquence qui est au moins 90 % identique à SEQ ID NO: 8.

3. Utilisation selon la revendication 2, dans laquelle le domaine variable de chaîne lourde comprend SEQ ID NO: 7, et le domaine variable de chaîne légère comprend SEQ ID NO: 8.

4. Utilisation selon l'une quelconque des revendications 1 à 3, dans laquelle la résine de base est du sépharose.

5. Utilisation selon l'une quelconque des revendications 1 à 3, dans laquelle la résine de base est un matériau de support quelconque.

6. Utilisation selon la revendication 5, dans laquelle le matériau de support est de l'agarose ou une résine capto.

7. Utilisation selon l'une quelconque des revendications 1 à 6, dans laquelle l'anticorps anti-facteur tissulaire ou son fragment de liaison à l'antigène est fixé de manière non covalente à la résine de base.

8. Utilisation selon l'une quelconque des revendications 1 à 6, dans laquelle l'anticorps anti-facteur tissulaire ou son fragment de liaison à l'antigène est fixé de manière covalente à la résine de base.

9. Utilisation selon la revendication 8, dans laquelle l'anticorps anti-facteur tissulaire ou son fragment de liaison à l'antigène est fixé de manière covalente à la résine de base par la formation d'une liaison disulfure entre une cystéine dans l'anticorps anti-facteur tissulaire ou son fragment de liaison à l'antigène et un groupe chimique sur la résine de base.

10. Utilisation selon la revendication 8, dans laquelle l'anticorps anti-facteur tissulaire ou son fragment de liaison à l'antigène est fixé de manière covalente à la résine de base par la formation d'une liaison covalente entre une amine libre de l'anticorps anti-facteur tissulaire ou du domaine de liaison d'antigène et un groupe chimique sur la résine de base.

11. Utilisation selon la revendication 10, dans laquelle la résine de base est un matériau de support solide activé par CNBr ou activé par N-hydroxysuccinimide (NHS).

12. Utilisation selon la revendication 10, dans laquelle la liaison covalente est représentée par la formule I ci-dessous : 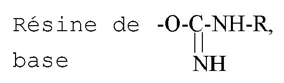 dans laquelle R représente l'anticorps anti-facteur tissulaire ou un fragment de liaison d'antigène de celui-ci.

13. Utilisation d'un kit comprenant la résine de chromatographie d'affinité selon l'une quelconque des revendications 1-12 pour purifier un polypeptide chimérique monocaténaire ou multicaténaire qui comprend un facteur tissulaire soluble.

14. Utilisation selon l'une quelconque des revendications 1 à 13, dans laquelle : le polypeptide chimérique monocaténaire comprend :
(i) un premier domaine de liaison de cible ;
(ii) un domaine de facteur tissulaire soluble ; et
(iii) un deuxième domaine de liaison de cible ; et
le polypeptide chimérique multicaténaire comprend :
(a) un premier polypeptide chimérique comprenant :
(i) un premier domaine de liaison de cible ;
(ii) un domaine de facteur tissulaire soluble ; et
(iii) un premier domaine d'une paire de domaines d'affinité ;
(b) un second polypeptide chimérique comprenant :
(i) un deuxième domaine d'une paire de domaines d'affinité ; et
(ii) un deuxième domaine de liaison de cible,
dans laquelle le premier polypeptide chimérique et le second polypeptide chimérique s'associent par l'intermédiaire de la liaison du premier domaine et du second domaine de la paire de domaines d'affinité.
